(19) Europäisches Patentamt
European Patent Office
Office européen des brevets

(11) EP 1 177 439 B1

(12) EUROPEAN PATENT SPECIFICATION

(45) Date of publication and mention of the grant of the patent: **08.09.2004 Bulletin 2004/37**

(21) Application number: **00931983.1**

(22) Date of filing: **28.04.2000**

(51) Int Cl.[7]: **G01N 33/50**, G01N 33/68, C12Q 1/68, C07K 14/47

(86) International application number: **PCT/US2000/011697**

(87) International publication number: **WO 2000/067023 (09.11.2000 Gazette 2000/45)**

# (54) SCREENING FOR IMMUNOSTIMULATORY DNA FUNCTIONAL MODIFIERS

SCREENING NACH MODULATOREN DER FUNKTION VON IMMUNSTIMULATORISCHER DNA

CRIBLAGE DE MODIFICATEURS FONCTIONNELS DE L'ADN IMMUNOSTIMULANT

(84) Designated Contracting States:
**AT BE CH CY DE DK ES FI FR GB GR IE IT LI LU MC NL PT SE**

(30) Priority: **29.04.1999 US 131830 P**
**03.03.2000 US 186845 P**

(43) Date of publication of application:
**06.02.2002 Bulletin 2002/06**

(73) Proprietors:
- **Coley Pharmaceutical GmbH**
  **40764 Langenfeld (DE)**
- **UNIVERSITY OF IOWA RESEARCH FOUNDATION**
  **Iowa City, IA 52242 (US)**

(72) Inventors:
- **NOLL, Berhard, O.**
  **D-40233 Duesseldorf (DE)**
- **SCHETTER, Christian**
  **D-40723 Hilden (DE)**
- **KRIEG, Arthur, M.**
  **Iowa City, IA 52246 (US)**

(74) Representative: **Jump, Timothy John Simon et al**
**Venner Shipley LLP**
**20 Little Britain**
**London EC1A 7DH (GB)**

(56) References cited:
**WO-A-96/02555** **WO-A-98/40100**

- **KRIEG, A.M.: "The CpG Motif: Implications for Clinical Immunology" BIODRUGS, vol. 10, no. 5, November 1998 (1998-11), pages 341-346, XP000949909 cited in the application**
- **HÄCKER, H. ET AL.: "CpG-DNA-specific activation of antigen-presenting cells requires stress kinase activity and is preceded by non-specific endocytosis and endosomal maturation" THE EMBO JOURNAL, vol. 17, no. 21, 2 November 1998 (1998-11-02), pages 6230-6240, XP000946495**
- **KRIEG A M ET AL: "CPG MOTIFS IN BACTERIAL DNA TRIGGER DIRECT B-CELL ACTIVATION" NATURE,GB,MACMILLAN JOURNALS LTD. LONDON, vol. 374, 6 April 1995 (1995-04-06), pages 546-549, XP000197060 ISSN: 0028-0836 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 177 439 B1

## Description

### Field Of The Invention

The present invention relates to regulation of the immune response. The invention also relates to immune stimulation in response to immunostimulatory DNA. In particular, the invention relates to complexes involving immunostimulatory DNA binding proteins, wherein the immunostimulatory DNA binding protein is an RNA binding protein.

### Background Of The Invention

In contrast to vertebrate DNA, bacterial DNA triggers a set of immunostimulatory activities (reviewed in Krieg AM (1998) *BioDrugs* 10:341-346 and Lipford GB et al (1998) *Trends Microbiol* 6:496-500). It was previously shown that most of these effects can be attributed to the presence of unmethylated CpG dinucleotides in particular sequence motifs. Krieg AM et al. (1995) *Nature* 374:546-549. In vertebrate DNA CpG dinucleotides are statistically under represented and usually methylated at position 5 of the cytosine. Bird AP (1987) *Trends Genet* 3:342-347. Oligodeoxyribonucleotides (ODNs) with particular CpG motifs mimic the immunostimulatory effect of bacterial DNA. Krieg AM et al. (1995) *Nature* 374:546-549. Modulating the immune system with specific immunostimulatory ODNs appears to offer exciting potential for the treatment and prevention of cancer, infectious diseases and allergies (reviewed in Krieg AM (1998) *BioDrugs* 10:341-346).

B lymphocytes respond quickly to the presence of immunostimulatory ODNs and start proliferating and secreting interleukin (IL)-6 and immunoglobulin (Ig). Klinman DM et al. (1996) *Proc Natl Acad Sci USA* 93:2879-2883; Yi A-K et al. (1996) *J Immunol* 156:558-564; Yi AK et al. (1996) *J Immunol* 157:5394-5402. The cytokines induced by immunostimulatory DNA are most notable for the predominant secretion of the Thl-like cytokines IL-12, IL-18 and interferon (IFN)-γ. Klinman DM et al. (1996) *Proc Narl Acad Sci USA* 93:2879-2883; Halpern MD et al. (1996) *Cell Immunol* 167:72-78; Yamamoto S et al. (1992) *J Immunol 148:4072-4076;* Roman M et al. (1997) *Nat Med* 3 :849. The IL-12 is predominantly derived from monocytic cells such as macrophages and dendritic cells, which also produce other pro-inflammatory cytokines such as Type 1 interferons, IL-6, and tumor necrosis factor (TNF)-α. In addition, these cells are induced to upregulate their surface expression of class II major histocompatibility complex (MHC) molecules and of costimulatory molecules. Thus, immunostimulatory DNA creates a Th1-like cytokine environment and enhances the function of antigen-presenting cells. Together with immunostimulatory DNA, the IL-12 activates natural killer (NK) cells to secrete IFN-γ and to have enhanced lytic activity. Ballas ZK et al. (1996) *J Immunol* 157:1840-1845; Cowdery JS et al. (1996) *J Immunol* 156:4570-4575. The IFN-γ secretion promotes the B cell response to immunostimulatory DNA, including both IL-6 secretion as well as IgM secretion. Of note, although immunostimulatory DNA can be an extremely powerful mitogen that drives more than 95% of B cells into the cell cycle, at lower concentrations, the DNA costimulates strongly with signals mediated through the B cell antigen receptor (BCR). Krieg AM et al. (1995) *Nature* 374:546-549. Thus, B cells that have bound specific antigen will be preferentially activated by immunostimulatory DNA. Like monocytic cells, B cells also upregulate their expression of costimulatory molecules and class II MHC upon activation with immunostimulatory DNA. Krieg AM et al. (1995) *Nature* 374:546-549; Davis HL et al. (1998) *J Immunol* 160:870-876.

The molecular mechanism underlying the response to CpG motifs is hitherto not well understood. One possible mechanism of action for immunostimulatory DNA could involve the binding to a cell surface receptor that then transduces a stimulatory signal. Indeed, evidence in support of this model has been published. Liang H et al. (1996) *J Clin Invest* 98:1119-1129. However, our own experiments using a different experimental model system have led to the opposite conclusion. Krieg AM et al. (1995) *Nature* 374:546-549. To determine whether cellular uptake of immunostimulatory ODN is required for cell activation, Liang et al. cultured B cells in tissue culture wells in which immunostimulatory ODN had been bound to sepharose beads. Liang H et al. (1996) *J Clin Invest* 98:1119-1129. 48 hour tritiated thymidine incorporation assays were then performed to determine whether the cells were still induced to proliferate, as they were in wells in which the ODN were bound. Since similar cell activation was still observed, those investigators concluded that cellular uptake of the DNA was not required, since they assumed that the DNA on the beads could not be taken up during the incubation. Others have performed studies using a similar methodology, but found that ODN on sepharose beads were still taken up, casting doubt on the earlier conclusion. Manzel L and Macfarlane DE (1999) *Antisense Nucl Acid Drug Develop* 9:459-464. Moreover, ODN bound to particles or covalently linked to a solid Teflon support were nonstimulatory. Krieg AM et al. (1995) *Nature* 374:546-549; Manzel L and Macfarlane DE (1999) *Antisense Nucl Acid Drug Develop* 9:459-464. Furthermore, in extensive studies using both human and murine B cells and monocytes to detect possible cell surface membrane proteins with binding specificity for immunostimulatory ODN, we performed flow cytometry measuring the surface binding, uptake, and egress of FITC ODN. These studies showed no difference between the ODN with or without an immunostimulatory motif, suggesting that specificity does not lie at the level of cell binding or uptake (Krieg et al., unpublished data). immunostimulatory ODN conjugated to either FITC or biotin retained full mitogenic properties, indicating no apparent steric hindrance. Thus, we have concluded from our studies that there is no CpG-specific cell membrane receptor and that cellular uptake of immunostimulatory ODN is required in order for cell activation. Our findings are in agreement with those of other investigators (D. Hume, personal communication).

The prior art does not recognize proteins specific for single-stranded (ss) CpG motifs, but it has long been recognized that there are DNA binding proteins on the cell surface (Lerner RA et al. (1971) *Proc Natl Acad Sci USA* 68:1212-1216; Aggarwal SK et al. (1975) *Proc Natl Acad Sci USA* 72:928-932; Beltinger C et al. (1995) *J Clin Invest* 95:1814-23; Yao G-Q et al. (1996) *Biochem Pharmacol* 51:431-436; Emlen W et al. (1992) *J Immunol* 148:3042-3048; Hefeneider SH et al. (1992) *Clin Immunol Immunopath* 63:245-251) and that DNA is internalized by cells spontaneously into an endosomal compartment where the DNA is acidified and degraded (Tonkinson JL and Stein CA (1994) *Nucleic Acids Res* 22:4268-4275; Krieg AM, Uptake and localization of phosphodiester and chimeric oligodeoxynucleotides in normal and leukemic primary cells. In: *Delivery Strategies for Antisense Oligonucleotide Therapeutics.* Editor, S. Akhtar. CRC Press, Inc., 1995, pp 177; Bennett RM et al. (1985) *J Clin Invest* 76:2182-2190). Nearly all cell types appear to be capable of internalizing DNA. Among cells of the immune system, uptake is inducible and generally appears to be highest among monocyte-derived cells, cancer cells, and B cells. Krieg AM, et al. (1991) *Antisense Res Dev* 1:161-171; Zhao Q et al. (1996) *Blood* 88:1788-1795. Since ODN are taken up by cells into endosomes and subsequently some fraction of the intracellular ODN enters the nucleus, the stimulatory effects may be mediated through an interaction with intracellular proteins or other molecules in the endosomes or in the nucleus. Nonstimulatory ODN, even at a five-fold molar excess, generally does not compete out B cell stimulation by immunostimulatory ODN, unless the nonstimulatory ODN is complementary to the immunostimulatory ODN. This indicates that the stimulatory pathway requires single-stranded intracellular CpG motifs. Double stranded DNA can induce the pathway, but this seems to be due to the fact that as it is internalized, much of the DNA is denatured into its single strands.

Since our studies indicate that single-stranded immunostimulatory ODN are immune stimulatory, but double-stranded are not (unless denatured), the effects are not likely to be directly mediated by transcription factors or other known proteins which bind to double-stranded DNA containing unmethylated CpGs. Iguchi-Ariga SM and Schaffner W (1989) *Genes Dev* 3:612-619; Gaston K and Fried M (1995) *Nucleic Acids Res* 23:901-909; Prendergast GC et al. (1991) *Cell* 65:395-407; Klempnauer K-H (1992) *Oncogene* 8:111-115; Cox GS et al. (1998) *Biochem Biophys Acta* 1396:67-68; reviewed in Tate PH and Bird AP (1993) *Curr Opin Genet Dev* 3:226-231. Likewise, proteins known to bind to methylated immunostimulatory DNA (Lewis JD et al. (1992) *Cell* 69:905-914) seem unlikely to be involved in the pathways triggered by ss immunostimulatory DNA. Of the several intracellular sequence-specific ssDNA binding proteins that have been cloned and analyzed using standard techniques, some have been shown to regulate transcription (e.g., Yee HA et al. (1991) *Nucleic Acids Res* 19:949-953). However, none have been shown to have any specificity for binding unmethylated CpG motifs. Brunel F et al. (1991) *Nucleic Acids Res* 19:5237-5245; Yee HA et al. (1991) *Nucleic Acids Res* 19:949-953; Hollingsworth MA et al. (1994) *Nucleic Acids Res* 22:1138-1146; Graumann P and Marahiel MA (1994) *FEBS Letts* 338:157-160; Wold MS (1997) *Ann Rev Biochem* 66:61-92; Ammerpohl O et al. (1997) *Gene* 200:75-84; Wang Z-Y et al. (1993) *J Biol Chem* 268:10681-10685.

Regardless of the route of DNA entry into cells, B cells and monocytic cells that have internalized immunostimulatory DNA rapidly shows signs of activation including generation of reactive oxygen species, degradation of I-κB and translocation of NF-κB to the nucleus, activation of the mitogen-activated protein kinase (MAPK) signaling pathways, and induction of transcription of multiple protooncogene and cytokine mRNAs. Krieg AM et al. (1995) *Nature* 374:546-549; Klinman DM et al. (1996) *Proc Natl Acad Sci USA* 93:2879-2883; Yi AK et al. (1996) *J Immunol* 157:4918-4925; Yi A-K et al. (1996) *J Immunol* 157:5394-5402; Yi A-K et al. (1998) *J Immunol* 160:4755; Yi A-K and Krieg AM (1998) *J Immunol* 161:4493-4497; Stacey KJ et al. (1996) *J Immunol* 157:2116-2122; Sparwasser T et al. (1997) *Eur J Immunol* 27:1671-1679. The earliest activation events such as the reactive oxygen burst, NF-κB activation, and MAPK activation (which occur within a few minutes of exposure to immunostimulatory DNA) all appear to be mediated through a specific chloroquine-sensitive pathway as these are completely blocked by low concentrations of chloroquine and related compounds, but B cell or monocyte activation by other activators such as endotoxin or CD40 are not inhibited. Yi A-K et al. (1998) *J Immunol* 160:4755-4761; Macfarlane DE and Manzel L (1998) *J Immunol* 160:1122-1131; Hacker H et al. (1998) *EMBO J* 17:6230-6240.

Recent discoveries have also revealed that certain other ODNs, independent of the previously described motifs, also have immunostimulatory effects. These immunostimulatory ODNs contain at least one T-rich or poly T motif. Immunostimulatory T-rich nucleic acids and poly T ODN were disclosed in co-pending U.S. provisional patent application 60/156,113, filed 25 September 1999, the entire contents of which is hereby incorporated by reference.

Since cell uptake is apparently required for immune activation, and since both CpG-and non-immunostimulatory ODNs are internalized and enclosed in endosomes, the sequence-specific discriminating step that leads to cell activation by immunostimulatory DNA must take place inside the cell after endocytosis. There exists a need to identify proteins within the cell which specifically bind to ss immunostimulatory ODNs, particularly ss CpG ODNs, and mediate or transduce their immunostimulatory effects.

**Summary of the Invention**

The invention provides screening methods for identifying compounds which bind to immunostimulatory DNA binding proteins and act as inhibitors mimics, agonists, antagonists, and cellular targets of immunostimulatory DNA. The immunostimulatory DNA binding proteins are defined as being RNA binding proteins hereinafter. The invention further provides methods for identifying cellular targets of immunostimulatory DNA binding proteins. The invention also provides for optimizing an immunostimulatory ODN for immune stimulation. The invention further provides for an isolated immunostimulatory ODN-immunostimulatory DNA binding protein complex and a kit using such a complex to screen for cellular targets of an immunostimulatory ODN.

According to one aspect of the invention, the invention provides a screening method for identifying compounds which bind to immunostimulatory DNA binding protein and belong to a group of immunostimulatory DNA functional modifiers. The method involves contacting an immunostimulatory DNA binding protein with a sample containing at least one candidate immunostimulatory DNA binding protein ligand, determining if the at least one candidate immunostimulatory DNA binding protein ligand binds to the immunostimulatory DNA binding protein, and determining if the at least one candidate immunostimulatory DNA binding protein ligand that binds the immunostimulatory DNA binding protein belongs to a group of immunostimulatory DNA functional modifiers consisting of: immunostimulatory DNA binding inhibitors, immunostimulatory DNA agonists, and immunostimulatory DNA mimics.

In certain embodiments of this first aspect of the invention, the immunostimulatory DNA binding protein binds specifically to CpG ODN, i.e., it is a CpG ODN binding protein. In certain other embodiments of this first aspect of the invention, the immunostimulatory DNA binding protein binds specifically to T-rich ODN, i.e., it is a T-rich ODN binding protein. In certain other embodiments of this first aspect of the invention, the immunostimulatory DNA binding protein binds specifically to poly T ODN, i.e., it is a poly T ODN binding protein.

According to a second aspect of the invention, the invention provides a screening method for identifying an immunostimulatory DNA binding competitor. This screening method involves contacting an immunostimulatory DNA binding protein with a sample containing at least one candidate immunostimulatory DNA competitor compound and an immunostimulatory ODN, and determining a first amount of reference immunostimulatory ODN bound to the immunostimulatory DNA binding protein in the presence of the sample relative to a second amount of reference immunostimulatory ODN bound to the immunostimulatory binding protein in the absence of an immunostimulatory DNA competitor compound, wherein the sample includes at least one immunostimulatory DNA competitor compound when the first amount is less than the second amount. In certain embodiments the method further comprises contacting the immunostimulatory DNA competitor compound with an immune cell in the presence of an immunostimulatory ODN to determine if the immunostimulatory DNA competitor compound is an immunostimulatory DNA inhibitor.

According to a third aspect of the invention, the invention provides for optimizing an immunostimulatory ODN for immune stimulation. This aspect involves providing an immunostimulatory DNA binding protein, a reference immunostimulatory DNA that binds to the immunostimulatory DNA binding protein, and at least one candidate optimized immunostimulatory DNA that differs from the reference immunostimulatory DNA by at least one nucleotide or at least one intemucleotide linkage, contacting the immunostimulatory DNA binding protein with a sample containing at least one candidate optimized immunostimulatory DNA and the reference immunostimulatory ODN, determining which, if any, candidate optimized immunostimulatory DNA binds the immunostimulatory DNA binding protein to a greater extent than the reference immunostimulatory DNA, redesignating a candidate optimized immunostimulatory DNA which binds the immunostimulatory DNA binding protein to a greater extent than the reference immunostimulatory DNA as the reference immunostimulatory DNA, and repeating the steps of providing, contacting, determining, and redesignating until no candidate optimized immunostimulatory DNA binds the immunostimulatory DNA binding protein better than the reference immunostimulatory DNA against which it is compared.

In certain embodiments of this third aspect of the invention, the initial reference immunostimulatory DNA is a CpG ODN. In certain other embodiments of this aspect of the invention, the initial reference immunostimulatory DNA is a T-rich ODN. In certain other embodiments of this aspect of the invention, the initial reference immunostimulatory DNA is a poly T ODN.

According to a fourth aspect of the invention, the invention provides a method for identifying cellular targets of an immunostimulatory ODN. The method involves screening a panel of candidate target molecules with a complex formed between an immunostimulatory ODN and an immunostimulatory DNA binding protein, selecting the candidate target molecules that bind to the complex, and determining the sequence of the bound candidate target molecules to identify the cellular targets of the immunostimulatory ODN. In certain embodiments the candidate target molecule is labeled with a marker selected from the group consisting of: digoxigenin, biotin, an isotope, a fluorophore, and an enzyme.

The invention in another aspect provides an isolated immunostimulatory ODN-immunostimulatory DNA binding protein complex. In certain embodiments of this aspect of the invention, the immunostimulatory DNA binding protein is an RNA binding protein. In certain preferred embodiments of this aspect of the invention, the immunostimulatory DNA binding protein is an RNA binding protein having at least one motif selected from the group consisting of: RBD, RGG domain, KH motif, and ARM (discussed below). In certain preferred embodiments of this aspect of the invention, the immunostimulatory DNA binding protein is selected from the group consisting of: hnRNPs, nucleolin, and lupus La protein. In a most preferred embodiment of this aspect of the invention, the immunostimulatory DNA binding protein is selected from the group consisting of: hnRNP D, AUF1, hnRNP A1, nucleolin, and lupus La protein.

In yet another aspect of the invention, the invention provides a kit for identifying cellular targets of an immunostimulatory ODN. This kit includes an immunostimulatory ODN in a container, an immunostimulatory DNA binding protein in a container, and instructions for preparing an immunostimulatory ODN-immunostimulatory DNA binding protein complex and for using the complex to screen a panel of candidate target molecules to identify cellular targets of an immunostimulatory ODN.

In certain embodiments the immunostimulatory DNA binding protein is selected from the group consisting of: nucleolin, lupus La protein, hnRNP D, AUF1, hnRNP A1, hnRNP U, and isoforms, fragments, variants, and equivalents thereof. The invention contemplates complexes of binding proteins, particularly those containing at least one of nucleolin, lupus La protein, hnRNP D, AUF1, hnRNP A1, hnRNP U, and isoforms, fragments, variants, and equivalents thereof. The immunostimulatory DNA binding protein in certain embodiments is attached to a substrate selected from the group consisting of: a plastic multiwell plate, a bead, a resin, a filter, a glass slide, a plastic slide, a BIAcore chip, and a silicon chip. In certain embodiments the immunostimulatory DNA binding protein is labeled with a marker selected from the group consisting of: digoxigenin, biotin, an isotope, a fluorophore, and an enzyme.

In certain embodiments the immunostimulatory ODN is a CpG ODN. In certain embodiments the CpG ODN is selected from the group consisting of: SEQ ID NO:1 (#2059), SEQ ID NO:2 (#2080), SEQ ID NO:3 (#1619), SEQ ID NO:11 (#5007), and SEQ ID NO:12 (#5004). In certain other embodiments the immunostimulatory ODN is a phosphorothioate ODN. In certain embodiments the phosphorothiate ODN is selected from the group consisting of: SEQ ID NO:11 (#5007), SEQ ID NO:16 (#5018), SEQ ID NO:17 (#5027), and SEQ ID NO:18 (#5030). In yet other embodiments the immunostimulatory ODN is a T-rich. ODN. In yet other embodiments the immunostimulatory ODN is a poly T ODN. In a particular embodiment the poly T ODN is SEQ ID NO:9 (#5017). In certain embodiments the immunostimulatory ODN is labeled with a marker selected from the group consisting of: digoxigenin, biotin, an isotope, a fluorophore, and an enzyme.

In certain embodiments the sample and the immunostimulatory ODN are contacted with the immunostimulatory DNA binding protein simultaneously, and in other embodiments the sample and the immunostimulatory ODN are contacted with the immunostimulatory DNA binding protein sequentially.

In certain embodiments the amount of bound immunostimulatory ODN is detected using a method selected from the group consisting of: EMSA, ELISA, surface plasmon resonance (BIA), fluorimetry, bioluminescence, refractometry, autoradiography, autoradiometry, polymerase chain reaction (PCR), and scintillation detection.

In certain embodiments the screening method includes an assay of the immune cell for the induction of cytokines, the production of antibodies, the isotype of secreted antibody, the expression of a cell surface molecule, the activation of cellular kinases or proliferation, or the change in redox potential.

In certain embodiments the at least one candidate compound is an isolated molecule selected from the group consisting of: polynucleotides, peptide nucleic acids, polypeptides, carbohydrates, lipids, hormones, small organic molecules, small inorganic molecules, variants of a reference immunostimulatory ODN incorporating at least one substitution of a phosphorothioate-type bond for a phosphodiester bond, variants of a reference immunostimulatory ODN incorporating at least one substitution of a phosphodiester bond for a phosphorothioate-type bond, variants of a reference immunostimulatory ODN incorporating at least one substitution of one phosphorothioate-type bond for a different phosphorothioate-type bond, and variants of a reference immunostimulatory ODN incorporating at least one substitution of one nucleotide with a different nucleotide. In certain embodiments the candidate compound is part of a combinatorial library of compounds.

In certain embodiments the immunostimulatory DNA binding protein ligand is contacted with an immune cell to determine whether the immunostimulatory DNA competitor compound is an immunostimulatory DNA functional mimic. In certain embodiments the step of contacting the immunostimulatory DNA binding protein ligand with an immune cell is performed in the presence of an immunostimulatory ODN to determine if the immunostimulatory DNA binding protein ligand is an immunostimulatory DNA inhibitor.

In certain embodiments the immunostimulatory ODN is tethered to the immunostimulatory DNA binding protein or is otherwise associated with the immunostimulatory DNA binding protein.

These and other objects of the invention will be described in further detail in connection with the detailed description of the invention.

**Brief Description of the Drawings**

**FIG. 1** depicts the results of competitive electrophoretic mobility shift assay (EMSA) of cytosolic extract using labeled immunostimulatory ODN #2059 (lane 1) and unlabeled ODNs #2059 (lanes 2-4) or #2060 (lanes 5-7) as competitor. The observed complexes C0 - C5 are indicated.

**FIG. 2** depicts crosslink assays from cytosolic extracts ofNK cells, Ramos cells, and HeLa cells with various digoxigenin-labeled ODN. MW standard markers are shown on the right.

**FIG. 3** depicts EMSA (panel A) and crosslink assay (panel B) with labeled CpG ODN #5004 of fractions eluted from heparin affinity column. MW standard markers are shown on the right in panel B. Binding proteins BP1 - BP5 are as indicated.

**FIG. 4** depicts EMSA (panel A) and crosslink assay (panel B) with labeled CpG ODN #5004 of fractions eluted from UNO-Q anion exchange column. MW standard markers are shown on the right in panel B.

**FIG. 5** depicts EMSA (upper panel) and crosslink assay (lower panel) with labeled CpG ODN #5004 of A1 anion exchange fractions further purified on CpG ODN-coated sepharose column. MW standard markers are shown on the right in the crosslink panel. The presence of BP2 (lupus La protein) in the 500 mM KCl eluate is indicated in the EMSA panel.

**FIG. 6** depicts EMSA (upper panel) and crosslink assay (lower panel) with labeled CpG ODN #5004 of anion exchange fraction A3 further purified on CpG ODN-coated sepharose column. MW standard marker is shown on the left in the crosslink panel.

**FIG. 7** depicts silver stain assays of protein fractions acquired during purification in Example 2. MW standard markers are shown on the right.

**FIG. 8** depicts EMSA (panel A) and crosslink assay (panel B) of the cation exchange run performed on the H3 heparin column fractions. Binding proteins BP1 and BP3 are as indicated. MW standard markers are shown on the right.

**FIG. 9** depicts EMSA (panel A) and crosslink assay (panel B) with labeled CpG ODN #5004 of cation exchange fraction C 1 further purified on CpG ODN-coated sepharose column. Binding proteins BP3 and BP4 are as indicated in the crosslink panel. MW standard markers are shown on the right in the crosslink panel.

**FIG. 10** depicts silver stain assays of protein fractions acquired during purification in Example 2. MW standard markers are shown on the right.

**FIG. 11** depicts cold competition EMSA with isolated BP1, BP2, BP3, and BP4.

**FIG. 12** depicts cold competition EMSA with isolated BP5.

**FIG. 13** depicts crosslink assay with isolated BP6.

**FIG. 14** depicts western blot assays of cytosol of different human cell lines (left panel) and of fractions H1 - H4 of the heparin column and the purified BP1 (right panel). Lanes in the left panel are: N, NK cells; J, Jurkat cells; H, HeLa cells; abd R, Ramos cells. Monoclonal anti-nucleolin antibody (Santa Cruz Biotechnology sc-8031) was used for detection. MW standard markers are shown on the right of each panel.

**FIG. 15** depicts a kit (11) for identifying cellular targets of an immunostimulatory ODN, comprising an immunostimulatory DNA binding protein (17), an immunostimulatory ODN in a container (19), instructions (21), and a box-like packaging (15).

**Brief Description of the Sequences**

SEQ ID NO:1 is the nucleotide sequence of the CpG phosphodiester ODN #2059.

SEQ ID NO:2 is the nucleotide sequence of the CpG phosphodiester ODN #2080.

SEQ ID NO:3 is the nucleotide sequence of the CpG phosphodiester ODN #1619.

SEQ ID NO:4 is the nucleotide sequence of phosphodiester ODN #1765, in which the CpG dinucleotide is methylated.

SEQ ID NO:5 is the nucleotide sequence of the non-CpG phosphodiester ODN #2049.

SEQ ID NO:6 is the nucleotide sequence of the CpG phosphodiester ODN #5011 in which all the CpG dinucleotides are methylated.

SEQ ID NO:7 is the nucleotide sequence of the non-CpG phosphodiester ODN #5015.

SEQ ID NO:8 is the nucleotide sequence of the non-CpG phosphodiester ODN # 5009 which is a poly C ODN and 5'-labeled with digoxigenin.

SEQ ID NO:9 is the nucleotide sequence of the non-CpG phosphodiester ODN #5017 which is a poly T ODN and 5'-labeled with digoxigenin.

SEQ ID NO:10 is the the nucleotide sequence of the CpG phosphodiester ODN #2059 modified by addition of an amino group on the 5' end.

SEQ ID NO:11 is the nucleotide sequence of the phosphorothioate CpG ODN #5007, corresponding to SEQ ID NO:1 with a digoxigenin label at the 5' T.

SEQ ID NO:12 is the nucleotide sequence of the phosphodiester CpG ODN #5004, corresponding to SEQ ID NO:1 with a digoxigenin label at the 5' T.

SEQ ID NO:13 is the nucleotide sequence of the methylated phosphodiester CpG ODN #5012, corresponding to SEQ ID NO:6 with a digoxigenin label at the 5' T.

SEQ ID NO:14 is the nucleotide sequence of the phosphodiester non-CpG ODN #5016, corresponding to SEQ ID NO:7 with a digoxigenin label at the 5' T.

SEQ ID NO:15 is the nucleotide sequence of the CpG phosphodiester ODN #5008 with a digoxigenin label at the 5' T.

SEQ ID NO:16 is the nucleotide sequence of the phosphorothioate CpG ODN #5029, corresponding to SEQ ID NO:10.

SEQ ID NO:17 is the nucleotide sequence of the phosphorothioate poly T ODN #5018, corresponding to SEQ ID NO:9.

SEQ ID NO:18 is the nucleotide sequence of the phosphorothioate poly C ODN #5030, corresponding to SEQ ID NO:8.

SEQ ID NO:19 is the nucleotide sequence of the phosphorothioate non-CpG ODN #5027, corresponding to SEQ ID NO:14.

SEQ ID NO:20 is the nucleotide sequence of poly T ODN #5021.

SEQ ID NO:21 is the nucleotide sequence of poly C ODN #5031.

SEQ ID NO:22 is the nucleotide sequence of CpG ODN #2060.

## Detailed Description of the Invention

The invention is based on the discovery that certain naturally occurring intracellular proteins bind specifically to immunostimulatory immunostimulatory ODNs. The complexes formed between these immunostimulatory DNA binding proteins and immunostimulatory DNA are the first described direct connection between immunostimulatory DNA taken up by cells and any intracellular protein. Methods are disclosed for using these binding proteins to characterize and study the compounds they bind. The methods include screening and optimizing immunostimulatory ODNs that are bound by immunostimulatory DNA binding proteins. Disclosed methods are also useful for screening candidate compounds that can either interfere with immunostimulatory DNA binding and immunostimulation by immunostimulatory DNA, or that can bind in place of immunostimulatory DNA and mimic the immunostimulation by immunostimulatory DNA. Also disclosed are complexes formed between immunostimulatory ODN and immunostimulatory DNA binding protein, methods for their use, and kits, all useful for identifying intracellular target molecules of immunostimulatory DNA.

A. Immunostimulatory DNA Binding Proteins. An "immunostimulatory DNA binding protein" as used herein is a protein found in nature that binds immunostimulatory DNA specifically and are defined as being RNA binding proteins. Immunostimulatory DNA binding proteins can but need not necessarily bind to immunostimulatory DNA in a sequence-specific manner.

"Immunostimulatory DNA" as used herein is a synthetic or naturally occurring polynucleotide or oligonucleotide characterized by its ability to activate certain immune cells, including B cells, dendritic cells, monocytes, natural killer (NK) cells, and T cells. Immunostimulatory DNA specifically includes but is not restricted to CpG oligodeoxynucleotide, i.e., CpG ODN, characterized by the inclusion of at least one 5'-cytosine-guanine-3'(5'-CG-3') dinucleotide in which the cytosine is not methylated at the 5 position. The 5'-CG-3' dinucleotide in CpG ODN can but need not be part of a palindrome, i.e., a self-complementary sequence following Watson-Crick base pairing convention. CpG DNA can be isolated, e.g., derived from invertebrates, preferably as DNA derived from bacteria and other human pathogens. Alternatively, CpG DNA can be a synthetic immunostimulatory ODN that incorporates a 5'-CG-3' dinucleotide and is at least six bases long, and preferably eight or more bases long. CpG ODNs can include at least one phosphorothioate-type linkage.

"Immunostimulatory DNA" as used herein also encompasses certain synthetic or naturally occurring polynucleotides or oligonucleotides which, despite their lacking the unmethylated CpG motif described above, are nonetheless characterized by their ability to activate certain immune cells, including B cells, natural killer (NK) cells, and dendritic cells. Included in this category of immunostimulatory DNA are T-rich nucleic acids, poly T ODN, poly G ODN, and methylated CpG ODN. A T-rich nucleic acid is a nucleic acid which includes at least one poly T sequence and/or which has a nucleotide composition of greater than 25 percent T nucleotide residues. Poly T ODN include at least four consecutive thymine (T) nucleotides. Poly G ODN include at least four consecutive guanine (G) nucleotides. As used herein, CpG ODN and T-rich, poly T, and poly G ODN are not necessarily mutually exclusive. For example, a given ODN containing at least one CpG dinucleotide and having at least 25 percent T nucleotide content can be considered both a CpG ODN and a T-rich ODN.

An "immunostimulatory DNA binding protein complex" as used herein is a complex of at least two polypeptides which binds immunostimulatory DNA specifically and is formed by at least one immunostimulatory DNA binding protein and at least one of the following: another molecule of the same immunostimulatory DNA binding protein, a different immunostimulatory DNA binding protein, or another ssDNA binding protein which is not an immunostimulatory DNA binding protein. In certain preferred embodiments, an immunostimulatory DNA binding protein complex includes at least one immunostimulatory DNA binding protein and nucleolin.

In its broadest sense, an immunostimulatory DNA binding protein is an RNA binding protein. Many known RNA binding proteins are also ssDNA binding proteins. Preferred RNA binding proteins are characterized by having at least one structural motif selected from an RNA-binding domain (RBD), an arginine-glycine-glycine (RGG) domain, a K-homology (KH) motif, and an arginine-rich motif (ARM). The RBD is also known in the literature as the ribonucleoprotein (RNP) motif, RNA recognition motif (RRM), RNP consensus sequence (RNP-CS), and consensus sequence RNA-binding domain (CS-RBD). These RBD and other motifs have been previously described in detail; for a review see Burd CG and Dreyfuss G (1994) *Science* 265:615-6621. As is evident from a list of RNA binding proteins provided in **Table 1,** certain motifs are present in multiple copies and/or in combination with one another in various RNA binding proteins. The ssDNA binding protein recombination protein A (RPA) is specifically excluded as an RNA binding protein for the purposes of this invention.

Table 1.

| RNA Binding Proteins | | | | |
|---|---|---|---|---|
| **Name** | **Motifs** | **Function** | **Preference** | **Reference** |
| AUF1 | 2x RBD 3x RGG | early response gene mRNA regulation | | Bhattacharya et al. (1999) *Nucleic Acids Res* 27:1464-1472 |
| betaARB | hnRNP family | destabilization of mRNA | A/C + U rich RNA | Blaxell et al. (2000) *J Biol Chem* 275: 4290-4297 |
| CBF-A | | transcription | RNA ssDNA CArG-box | Kamada et al. (1992) *Gene* 119: 229-236 |
| CUG-BP | 3x RBD | splicing mRNA translation myotonic dystrophy | | |
| EWS | RGG | Ewing sarcoma transiocation | | |
| FMR1 | 2x KH 1x RGG | | | Siomi et al. (1993) *Cell* 74:291-288 |
| hnRNP A0 | 2x RBD 1x RGG | splicing | | |
| hnRNP A 1 | 2x RBD 1x RGG | splicing mRNA transport telomere binding | ssRNA | |
| hnRNP A2/B1 | 2x RBD 1x RGG | splicing mRNA trafficking | | |
| hnRNP C1/C2 | 1x RBD | transcript packaging splicing mRNA biogenesis nuclear retention mRNA stability | | |
| hnRNP D | 2x RBD 3x RGG | transcription recombination mRNA turnover | RNA; UUAG | |

Table 1. (continued)

| RNA Binding Proteins | | | | |
|---|---|---|---|---|
| **Name** | **Motifs** | **Function** | **Preference** | **Reference** |
| hnRNP E1 | 3x KH | translational silencing | | |
| hnRNP E2 | 3x KH | mRNA stability | | |
| hnRNP F | 3x RBD GY-rich region | splicing | RNA, otigo(dG), poly (rG) nuclear cap-binding complex RNA pol II | Yoshida et al. (1999) *Genes Cells* 4: 707-719 Matunis et al. ( 1994) *Nucleic Acids Res* 22: 1059-1067 |
| hnRNP G | 1x RBD 1x RGG | | | |
| hnRNP H/H' | 3x RBD | splicing polyadenylation | poly(rG) | Matunis et al. (1994) *Nucleic Acids Res* 22:1059-1067 |
| hnRNP I | 4x RBD | splicing polyadenylation | | |
| hnRNP K | 3x KH 1x RGG | transcription translational silencing mRNA export | | |
| hnRNP L | 4x RBD | mRNA stability mRNA transport | | |
| hnRNP M | 3x RBD | splicing heat-shock response | | |
| hnRNP P2 (TLS; FUS) | 1x RBD 1x RGG | transcriptional activation domain in FUS-ERG oncoprotein; proliferative response of lymphocytes | RNA DNA | Hicks et al. (2000) *Nat Genet* 24: 175-179 |
| hnRNP R | 3x RBD 1x RGG | | | |
| hnRNP U | 1x RGG | mRNA biogenesis, nuclear retention | mRNA ssDNA | Kiledjian et al. (1992) *EMBO J* 11: 2655-2664 |
| hRP-A | | recombination | ssDNA | Baumann et al. (1997) *EMBO J* 16: 5198-5206 |
| HuR | 3x RDB | mRNA stability mRNA transport | | |
| lupus La protein; SSB | 1 x RBD | transcription | RNA 3' ends histone mRNA RNA pol III calmodulin | Castro et al. (1996) *Cell Calcium* 20: 493-500 Goodier et al. (1998) *J Biol Chem* 273: 26110-26116 |

Table 1. (continued)

| RNA Binding Proteins | | | | |
|---|---|---|---|---|
| **Name** | **Motifs** | **Function** | **Preference** | **Reference** |
| Nova-2 | KH | regulation neuron specific splicing | ss 5'-UCAPy-3' | Lewis et al. (2000) *Cell* 100:323-332 |
| nucleolin | 4x RBD 1x RGG | transcription | RNA dsDNA histone H1 | |
| PABP | 4x RBD | | poly A | Deo et al. (1999) *Cell* 98:835-845 |
| PC4 | | transcription positive cofactor 4, replication | ssDNA | Pan et al. (1996) *J Biol Chem* 271: 22111-22116 |
| RAD51 | | recombination | | Baumann et al. (1997) *EMBO J* 16: 5198-5206 |
| RAD52 | | recombination | ssDNA | Sugiyama et al. (1998) *Proc Natl Acad Sci USA* 95: 6049-6054 |
| RAG I | | | | Spanopoulou et al. (1995) *Immunity* 3: 715-726 |
| RAG2 | | | | Spanopoulou et al. (1995) *Immunity* 3: 715-726 |
| RBP56/hTAFII 68 | 1x RBD 1x RGG | homology to hnRNP P2 | RNA ssDNA, TFIID RNA pol II | Morohoshi et al. (1998) *Gene* 221: 191-198 Bertolotti et al. (1996) *EMBO J* 15:5022-5031 |
| rho | | transcription termination factor | | |
| SAF-B | 1x RBD | scaffold attachment factor, transcription, heat-shock response | | |
| ssDBF | hnRNP A/B family | represses estrogen-induced transcription | ssDNA in ERE | Smidt et al. (1995) *Nucleic Acids Res* 23:2389-2395 |
| trp | | RNA-binding attenuation protein | | |

Among the immunostimulatory DNA binding proteins identified in the invention are some that belong to a class of eukaryotic nuclear proteins called heterogeneous nuclear ribonucleoproteins (hnRNPs), a functionally diverse set of highly conserved RNA- and ssDNA binding proteins. At least some of these are known to be found in the cytoplasm as well as in the nucleus. Structurally, hnRNPs are characterized by one or more RBDs, KH motifs, and/or RGGs. Dreyfuss G et al. (1993) *Annu Rev Biochem* 62:289-321; Burd CG and Dreyfuss G (1994) *Science* 265:615-621; Dempsey LA et al. (1999) *J Biol Chem* 274:1066-1071. Many of the hnRNPs each exist in multiple isoforms which represent transcriptional splice variants. Previously these proteins were known to be involved in various aspects of post-transcriptional regulation of gene expression, pre-mRNA processing, and intracellular RNA transport and localization. At least some hnRNPs are targets for autoimmune response, especially in lupus erythematosus. Burd CG and Dreyfuss G (1994) *Science* 265:615-621. Particular hnRNPs identified in the invention as immunostimulatory DNA binding proteins include hnRNP D, AUF1, and hnRNP A1.

One of the hnRNP proteins identified as an immunostimulatory DNA binding protein is hnRNP D, also known as hnRNP D0 (GenBank accession no. 870749, 2815614, 870745). Each of three known isoforms of hnRNP D, derived from mRNAs that reflect alternative splicing of two coding exons, possesses two RBDs and three RGG motifs. Kajita Y et al. (1995) *J Biol Chem* 270:22167-22175. The largest isoform incorporates both exons and encodes a 355 amino acid polypeptide with a predicted molecular mass of 38.4 kDa; a second isoform includes only the larger of the two exons and encodes a 336 amino acid polypeptide with a predicted molecular mass of 36.2 kDa; and a third isoform includes only the smaller of the two exons and encodes a 306 amino acid polypeptide with a predicted molecular mass of 32.8 kDa. A fourth isoform, derived from the splice variant lacking both the larger and the smaller exons, corresponds to the 287 amino acid isoform of the RNA binding protein AUF1. Bhattacharya S et al. (1999) *Nucleic Acids Res* 27: 1464-1472. Like the other three isoforms of hnRNP D, AUF1 possesses two RBDs and three RGG motifs. The hnRNP D proteins shuttle between the nucleus and the cytoplasm and can be isolated from either compartment. Each of the two RBDs can bind nucleic acid, either alone or simultaneously to either a single nucleic acid or to distinct nucleic acids. Isolated hnRNP D has been reported to exhibit exceptionally sequence-specific binding to non-CpG nucleic acids (TTAGGG), and $(UUAGGG)_4$; binding is abolished by the substitution of each of the first four bases of repeat units. Kajita Y et al. (1995) *J Biol Chem* 270:22167-22175. This specificity arises from sequence within the RBDs that is relatively unique to hnRNP D. Kajita Y et al. (1995) *J Biol Chem* 270:22167-22175. While the *in vivo* functions of hnRNP D remain largely unknown, hnRNP D has been reported to bind *in vitro* to 3' splice sites, and the 306 amino acid isoform, in a heterodimeric complex with nucleolin called LR1, participates in binding to non-CpG-specific sequence duplex DNA. Dempsey LA et al. (1998) *J Biol Chem* 273:29224-29229. Furthermore, the LR1 complex is a B-cell specific transcriptional activator and may also function in immunoglobulin heavy chain class switch recombination. Dempsey LA et al. (1998) *J Biol Chem* 273:29224-29229; Williams M and Maizels N (1991) *Genes Dev* 5:2353-61.

A second immunostimulatory DNA binding protein identified in the invention is hnRNP A1 (SwissProt accession no. P09651). Like hnRNP D, hnRNP A1 posesses two RBDs and an RGG motif, and it shuttles between the nucleus and the cytoplasm. Pinol-Roma S and Dreyfuss G (1992) *Nature* 355:730-732. In the nucleus, hnRNP A1 has been reported to bind to 3' and 5' intronic splice sites at polypyrimidine stretches bordered by AG. Burd CG and Dreyfuss G (1994) *EMBO J* 13:1197-1204. In the cytoplasm, hnRNP A1 has been reported to bind to AU-rich elements (reiterated AUUUA sequences; AREs) characteristic of 3' untranslated regions of many cytokines and proto-oncogenes. Hamilton BJ et al. (1997) *J Biol Chem* 45:28732-28741. Cytoplasmic and nuclear hnRNP A1 thus exhibit different specific RNA binding profiles and appear to have different roles with respect to post-transcriptional regulation of gene expression. Cytoplasmic hnRNP A1, phosphorylated on serines and threonines, may act to stabilize transcripts characterized by AREs. Nuclear hnRNP A1, dephosphorylated on serines and threonines, appears to modulate pre-mRNA splicing through its antagonistic effect on splicing factor 2 (SF2/ASF). Caceres JF et al. (1994) *Science* 265:1706-1710; Hamilton BJ et al. (1997) *J Biol Chem* 45:28732-28741.

A third protein identified as an immunostimulatory DNA binding protein is lupus La protein, also known as Sjögren's syndrome type B antigen or SS-B (GenBank accession no. 125985). Lupus La protein is a 48 kDa 408 amino acid transcription termination factor which contains one RBD and binds to the 3' termini of nascent RNA polymerase III transcripts. Chan EKL et al. (1989) *Nucleic Acids Res* 17:2233-2244; Gottlieb E and Steitz JA (1989) *EMBO J* 8: 851-861. Lupus La protein is present in a myriad of nuclear and cytoplasmic ribonucleoprotein complexes in vivo where it may function as an RNA-folding protein or RNA chaperone. Rosenblum JS et al. (1998) *J Cell Biol* 143:887-99. It also is a calmodulin-binding protein (Castro A et al. (1996) *Cell Calcium* 20:493-500), and it interacts with the small subunit of ribosomes (Peek R et al. (1996) *Eur J Biochem* 236:649-55) and transcription termination factor (Maraia RJ et al. (1994) *Mol Cell Biol* 14:2147-58). Sera from about 10 percent of patients with systemic lupus erythematosus contain anti-SS-B/La antibodies that are directed against the RBD and react with normal La protein as if it were foreign. Rischmueller M et al. (1995) *Clin Exp Immunol* 101:39-44.

A fourth protein now identified in immunostimulatory DNA binding protein complexes is nucleolin. Nucleolin is a highly conserved, multifunctional, multidomain phosphoprotein present in particular abundance in the nucleolus and associated with cell proliferation (reviewed in Srivastava M and Pollard HB (1999) *FASEB J* 13:1911-1922). Because of its highly acidic N-terminal domain, nucleolin has an apparent MW of 105 kDa on SDS-PAGE despite its predicted MW of 77 kDa based on its cDNA sequence. In addition to its roles in ribosomal DNA transcription, preribosomal packaging, and organization of nucleolar chromatin, nucleolin also functions as a cell surface receptor and shuttle protein between plasma membrane, cytoplasm, and nucleus. Kibbey MC et al. (1997) *J Neurosci Res* 42: 314-322; Nigg FA et al. (1997) *Nature* 386:779-787; Lee CH et al. (1998) *J Biol Chem* 273:7650-7656; Bates PJ et al. (1999) *J Biol Chem* 274:26369-26377. Like other RNA binding proteins, including hnRNPs, nucleolin contains motifs characteristic of proteins capable of interacting with RNA and ssDNA: four RBDs and one RGG. Nucleolin has previously been shown to recognize specific sequences of RNA (UCCCGA) and T-rich, but not A-rich ssDNA. Dickinson LA et al. (1995) *Mol Cell Biol* 15:456-465; Ghisolfi-Nieto L et al. (1996) *J Mol Biol* 260:34-54.

Together with hnRNP D, nucleolin forms the B-cell specific transcription factor LR1. Increased amounts of intact nucleolin correlate with increased cell proliferation. Evidence suggests that the phosphorylation state of nucleolin influences degradation of nucleolin and therefore cell proliferation.

Both alone and in association with hnRNP D, nucleolin has been shown to bind to G-quartets, structures in which four guanines associate in a planar ring and each guanine interacts with two other guanines through G-G Hoogsteen bonding. Runs of G-quartets can stabilize four single strands of G-rich DNA into four-stranded G4 DNA, which is also bound by nucleolin. Dempsey LA et al. (1999) *J Biol Chem* 274:1066-1071; Hanakahi LA et al. (1999) *J Biol Chem* 274:15908-15912. G-rich DNA is particularly characteristic of ribosomal DNA, immunoglobulin heavy chain switch regions, and telomeres. Despite the reported association between nucleolin and cell proliferation, binding of G-rich DNA to nucleolin has been associated with inhibition of cell proliferation. Bates PJ et al. (1999) *J Biol Chem* 274: 26369-26377. Nucleolin was also recently shown to bind to G-rich phosphodiester oligonucleotides and to inhibit proliferation of a variety of human tumor cell lines. Bates PJ et al. (1999) *J Biol Chem* 274:26369-26377.

The methods employed in the specific isolation and identification of these immunostimulatory DNA binding proteins are described in the Examples below. Briefly, both the isolation and identification began with the preparation of cytosolic or nuclear extracts from cells likely to express protein of interest. In this instance, the human B cell line Ramos was selected because B cells are susceptible to CpG stimulation and, unlike many B cell lines, the Ramos cell line does not carry genes of the Epstein-Barr virus (EBV). Proteins isolated from this cell line therefore should be free of EBV-encoded protein. Because initial experiments revealed no major differences between extracts prepared with and without detergent (0.1 % NP-40), cytosolic extracts prepared without detergent were used for isolation and identification procedures described below.

The isolation proceeded from the extraction to a heparin affinity chromatography step. Owing to its strong negative charge, heparin is known to mimic DNA in binding to some proteins and to associate with a wide variety of DNA binding proteins. Bound proteins were eluted from the heparin affinity column by increasing the ionic strength of the elution buffer. Proteins within individual elution fractions were further separated on the basis of their charge by ion exchange chromatography using either an anionic UNO-Q column or a cationic UNO-S column. After elution from the ion exchange column, again with increasing ionic strength, buffer of the resulting protein fractions was changed to DNA binding buffer without Triton X-100 and applied on a 1ml sepharose column that was previously conjugated with immunostimulatory oligodeoxynucleotide. Proteins bound to the ODN-conjugated column in proportion to their degree of specificity and avidity for the oligodeoxynucleotide. After the column was washed with unspecific competitor poly(dI-dC)·poly(dI-dC) and 0.15 M KCl to reduce the extent of unspecific binding, bound proteins were then eluted from the ODN-conjugated column by exposure to high salt. Elution fractions were desalted and concentrated by ultrafiltration, and individual proteins were isolated by SDS-PAGE.

At least six immunostimulatory DNA binding proteins, BP1 - BP6, were isolated following this scheme.

Sequence-specific binding was assessed by standard electromobility shift assay (EMSA). Fried M and Crothers DM (1981) *Nucl Acids Res* 9:6505-6525. This assay involved mixing individual aliquots of cell extract each with an isotope- or digoxigenin-labeled specific oligonucleotide and then separating bound from unbound oligonucleotide by non-denaturing PAGE. Unbound oligonucleotides migrated through the gel faster than bound oligonucleotides, so that lanes containing protein-oligonucleotide complexes were readily identified by the apparent mobility shift of the oligonucleotide upon autoradiography or suitable label detection. Other methods of labeling and detecting oligonucleotides can be used, e.g., photo-emissive chemiluminescence or fluorescence techniques. Using this approach, oligonucleotides which bound to a protein or proteins in a given sample were distinguished from oligonucleotides which did not bind. Conversely, a protein or proteins which bound to a specific oligonucleotide were distinguished from a protein or proteins which did not bind.

The EMSA technique was also used to determine thermodynamic physical constants of DNA binding proteins, including relative affinity and association and dissociation rate constants. See, e.g., *Current Protocols in Molecular Biology,* John Wiley & Sons, New York, 1999, Unit 12.2. By adding unlabeled oligonucleotides in various concentrations to a sample of the complex of binding protein and labeled oligonucleotide, the unlabeled oligonucleotides competed for binding to the protein. With increasing affinity and concentration of the unlabeled oligonucleotides, the read-out of the protein-labeled oligonucleotide complex weakened because the ratio of labeled to unlabeled complex shifted to the unlabeled complex.

A limitation of EMSA is its lack of information as to the size of the proteins that participate in complex formation. Bands visualized by EMSA under non-denaturing conditions correspond to and are referred to herein as complexes. Such complexes are presumed to include at least one protein and at least one nucleotide. Molecular size determination for individual proteins was made by crosslinking complexes formed in solution between oligonucleotides and proteins and analysing them with denaturing SDS-PAGE. Crosslinking was readily accomplished by ultraviolet irradiation. The apparent molecular weight of any given crosslinked complex was determined by comparison to the mobility of similarly denatured defined molecular weight standards run on the same gel. Because the oligonucleotide was crosslinked to the protein, the apparent molecular weight of the complex reflected the contribution from the oligonucleotide. Correction for this contribution is complicated by the fact that every phosphodiester bond of the nucleic acid carries one negative charge, making it migrate faster than predicted on the basis of readily calculable weight alone. It is also possible that more than one oligonucleotide is linked to the protein, so even a single protein identified on EMSA may give rise to more than an single band in a crosslink assay. Furthermore, every protein associated to the oligonucleotide in a multi-protein complex also becomes crosslinked to the principal binding protein, giving an apparent molecular weight that represents the sum of the weights of all the proteins participating in the complex.

Identification of individual proteins so obtained was accomplished by mass spectrometry (MS). Matrix-assisted laser desorption/ionization (MALDI)-MS and electrospray ionization (ESI)-MS are now standard methods used for identifying peptides available in femtomole quantities. Mann M and Talbo G (1996) *Curr Opin Biotechnol* 7:11-19; Mann M and Wilm M (1995) *Trends Biochem Sci* 20:219-224; Mann M et al. (1989) *Anal Chem* 61:1702-1708. Individual bands were cut out of the polyacrylamide gel, cleaved with trypsin and then eluted to yield peptide fragments that are subjected to MALDI-MS or ESI-MS analysis. The combination of mass/charge data from the MS, cleavage site specificity of the trypsin digest, and peptide sequence data permitted identification of individual proteins and peptides. MALDI-MS analysis gives a mass fingerprint of the cleaved and analyzed proteins. The fingerprint is useful only for scanning against a database of calculated peptide masses corresponding to fully sequenced proteins. The ESI-MS analysis is more difficult, but it permits identification based on comparison to either complete or partial sequence data. Mass accuracies for either method can exceed 0.01 percent, i.e., 1 Da per 10 kDa.

B. Screening Methods Useful for Identifying Compounds Which Bind to Immunostimulatory DNA Binding Proteins. The invention provides for screening methods useful for identifying compounds which bind to immunostimulatory DNA binding proteins. In particular, the invention provides a screening method for identifying compounds useful for inhibiting the interaction between immunostimulatory DNA and an immunostimulatory DNA binding protein. The invention further particularly provides a screening method to identify agonist compounds useful in immunotherapy.

"Screening" as used herein refers to a process in which a sample containing at least one candidate compound is processed according to the methods of the invention. Preferably, the screening method is a high throughput screening method, in which many candidate compounds are processed in a short period of time. For instance, many thousands of candidate compounds can be screened in a single day for their ability to bind to an immunostimulatory DNA binding protein.

"Bind" as used herein refers to the physical association between molecules which is more prolonged and/or of greater strength or affinity than would be observed following random collisions of molecules that do not bind to one another. Two entities which are "bound" are physically associated with one another, either transiently or for longer periods, or cause a biochemical or conformational change that can be detected.

"Isolated" as used herein with respect to oligonucleotides, polynucleotides, and polypeptides, means a compound is separated from its native environment in sufficiently pure form so that it can be manipulated or used for any one of the purposes of the invention. Thus, isolated means sufficiently pure to be used (i) to raise and/or isolate antibodies, (ii) as a reagent in an assay, or (iii) for sequencing, etc.

In the first instance the invention provides a method of screening for compounds which bind to an immunostimulatory DNA binding protein. Any compound which binds to an immunostimulatory DNA binding protein is herein termed a "immunostimulatory DNA binding protein ligand" or simply "ligand compound." A ligand compound can bind to any site on an immunostimulatory DNA binding protein; thus a ligand compound can but need not necessarily compete with immunostimulatory DNA for binding. Candidate ligand compounds can be selected from among immunostimulatory DNAs, other polynucleotides, peptide nucleic acids (PNAs), polypeptides, carbohydrates, lipids, hormones, small organic molecules, and small inorganic molecules.

"Phosphorothioate-type linkage" as used herein is a phosphorothioate or phosphorodithioate linkage connecting adjacent nucleotide bases along a backbone occurring within a nucleic acid molecule. Prior studies have shown that ODN uptake by lymphocytes is markedly affected by the backbone chemistry. Zhao Q et al. (1993) *Antisense Res Dev 3:53*-66. The highest cell membrane binding and uptake was seen with chimeric phosphorothioate ODN in which the central linkages are phosphodiester, but the two 5' and five 3' linkages are phosphorothioate modified. Prior studies have also shown that phosphorothioate modified ODN are far more immunostimulatory than corresponding unmodified ODN. Phosphodiester ODN were synthesized on an Applied Biosystems Inc. Model 380A, 380B, or 394 DNA synthesizer using standard beta-cyanoethyl phosphoramidite chemistry. Beaucage SL and Caruthers MH (1981) *Tetrahedron Lett* 22:1859. Phosphorothioate linkages were introduced by oxidizing the phosphite linkage with elemental sulfur instead of the standard iodine oxidation. The four common nucleoside phosphoramidites were purchased from Applied Biosystems. All phosphodiester and phosphorothioate-containing ODN were deprotected by treatment with concentrated ammonia at 55°C for 12 hours. The ODN were purified by gel exclusion chromatography and lyophilized to dryness prior to use. Phosphorodithioate linkages were introduced by using deoxynucleoside S-(β-benzoylmercaptoethyl)pyrrolidino thiophosphoramidites. Wiesler WT et al., Synthesis and purification of phosphorodithioate DNA. In: *Methods in Molecular Biology: Protocols for Oligonucleotides and Analogs-Synthesis and Properties*, Agrawal S (ed.),

Humana Press, Totowa, NJ, pp. 191-206, 1993. Phosphorodithioate-containing ODN were deprotected by treatment with concentrated ammonia at 55°C for 12 hours followed by reverse phase HPLC purification.

"Other polynucleotides" as used herein refers to polynucleotide sequences lacking an unmethylated 5'-CG-3' dinucleotide. For example, previous studies have shown that even though the level of stimulation is reduced by methylation of the CpG motif, such methylated ODN retain a level of immune stimulatory activity that is clearly above background, suggesting that the recognition complex or some part of the complex can bind methylated motifs. Yi AK et al. (1998) *J Immunol* 160:5898-906. These other polynucleotides can be synthetic, semi-synthetic, or naturally occurring sequences of DNA or RNA.

"Peptide nucleic acids" (PNAs) as used herein are nucleic acid analog molecules prepared as oligonucleotides in which at least a portion of the sugar-phosphate backbone has been replaced with a neutral achiral polyamide (peptide) backbone. Nielsen PE et al. (1991) *Science* 254:1497-1500. PNAs can bind to complementary DNA or RNA, and they are highly resistant to proteases and nucleases. Demidov VV et al. (1994) *Biochem Biopharmacol* 48:1310-1313. PNA oligomers are known to exert an antisense effect when they are delivered to the interior of cells. Hanvey JC et al. (1992) *Science* 258:1481-1485; Bonham MA et al. (1995) *Nucleic Acids Res* 23:1197-1203.

"Polypeptides" as used herein are used consistently with their known meaning in the art and include isolated whole proteins and partial proteins, encoded by nucleic acids. Such polypeptides can be isolated from biological samples including tissue or cell homogenates, and can also be expressed recombinantly in a variety of prokaryotic and eukaryotic expression systems by constructing an expression vector appropriate to the expression system, introducing the expression vector into the expression system, and isolating the recombinantly expressed protein. Short polypeptides, including antigenic peptides (such as are presented by major histocompatibility complex (MHC) molecules on the surface of a cell for immune recognition) also can be synthesized chemically using well established methods of peptide synthesis. Polypeptides can also include glycoproteins, which are proteins modified by the addition of at least one carbohydrate moiety, and phosphoproteins, which are proteins modified by the addition of at least one phosphoric acid through a peptide or ester bond.

"Carbohydrates" as used herein are native or substituted polyhydroxyaldehydes, polyhydroxyketones, mono-, di-, and polysaccharides. Examples include but are not limited to glucose, cellulose, starch, glycogen, chitosan, and chitin.

"Lipids" as used herein include fats, oils, fatty acids, sterols, steroids, terpenes, lipoproteins, glycolipids, phospholipids, sulfolipids, and aminolipids.

"Hormones" as used herein include steroid hormones and peptide hormones.

"Small organic molecules" as used herein includes naturally occurring, synthetic, and semi-synthetic organic molecules. Preferably the small organic molecules have a molecular mass of less than about 1 kDa. These include drugs.

"Small inorganic molecules" as used herein includes naturally occurring, synthetic, and semi-synthetic inorganic molecules. Preferably the small inorganic molecules have a molecular mass of less than about 1 kDa.

The screening method involves contacting at least one candidate ligand compound with an immunostimulatory DNA binding protein under conditions which, in the absence of a ligand compound, exerts no effect on the apparent molecular mass of the immunostimulatory DNA binding protein or the ability to detect the immunostimulatory DNA binding protein according to the methods of the invention.

"Candidate" compounds encompass numerous chemical classes, although typically they are organic compounds. Candidate compounds comprise functional chemical groups necessary for structural interactions with polypeptides, and typically include at least an amine, carbonyl, hydroxyl or carboxyl group, preferably at least two of the functional chemical groups and more preferably at least three of the functional chemical groups. The candidate compounds can comprise cyclic carbon or heterocyclic structure and/or aromatic or polyaromatic structures substituted with one or more of the above-identified functional groups. Candidate compounds also can be biomolecules such as peptides, saccharides, fatty acids, sterols, isoprenoids, purines, pyrimidines, derivatives or structural analogs of the above, or combinations thereof and the like. Where the compound is a nucleic acid, the compound typically is a DNA or RNA molecule, although modified nucleic acids having non-natural bonds or subunits are also contemplated.

"Sample" as used herein is any type of material which contains or is thought to contain at least one compound of interest. A sample containing at least one candidate ligand compound is any type of material which includes the at least one candidate compound. The sample can be, for instance, a biological isolate containing a plurality of candidate ligand compounds. The sample can also be a single candidate ligand compound in isolated form. Alternatively, the sample can be a library or panel of natural and/or synthetic compounds. The library can, for instance, be a combinatorial library of chemical compounds generated using a synthetic strategy in which chemical members of the library are made according to a systematic methodology by assembling chemical subunits. Every member of the library is thus made up of one or more of the subunits. Depending on the ultimate nature of the library members desired, the chemical subunits used to generate the library can include naturally occurring or modified amino acids, naturally occurring or modified nucleotides, naturally occurring or modified saccharides or other organic or inorganic molecules. Recombinant libraries can also be generated using molecular biology tools in bacteria or bacteriophage particles.

In order to determine whether a sample contains an immunostimulatory DNA binding protein ligand compound, the amount of ligand compound bound to the immunostimulatory DNA binding protein must be determined. To accomplish this, either the ligand compound or the immunostimulatory DNA binding protein can be labeled. A substance is "labeled" when it is physically or chemically associated with a marker such that detection or measurement of the marker is equivalent to the detection of the presence or measurement of the amount of substance in a sample.

A "marker" as used herein is a molecular tag which can be readily detected by physical, chemical, biochemical, enzymatic, or other means, and includes but is not limited to: digoxigenin, an isotope; an enzyme; a fluorescent, luminescent, or chromophoric moiety; an antibody with any such marker; a hapten that can be detected using an antibody; and labeled binding partners such as (strept)avidin and biotin. Isotopic markers include but are not limited to $^{3}H$, $^{14}C$, $^{32}P$, $^{35}S$, $^{125}I$ and $^{131}I$. Typical fluorescent markers include fluorescein isothiocyanate, rhodamine, phycoerythrin, phycocyanin, allophycocyanin, and fluorescamine. Typical chemiluminescent compounds include luminol, isoluminol, aromatic acridinium esters, imidazoles, and the oxalate esters. Typical bioluminescent compounds include luciferin, and luciferase. Typical enzymes include alkaline phosphatase, β-galactosidase, glucose-6-phosphate dehydrogenase, maleate dehydrogenase, glucose oxidase, and horseradish peroxidase.

The screening assay involves measuring a binding interaction. The amount of binding that occurs between two binding partners can be assessed in a variety of ways. For instance, the ligand compound can be labeled and then contacted with the immunostimulatory DNA binding protein under conditions under which they would normally interact, such as conditions that mimic the *in vivo* environment. A separation step can then be performed to separate all of the unbound material. Finally, the amount of labeled ligand compound which is bound can be determined. The method for determining the amount of bound labeled ligand compound will depend on the type of marker used. The ability of a ligand compound to bind with an immunostimulatory DNA binding protein can be determined by comparing the amount of labeled ligand compound which is bound to the immunostimulatory DNA binding protein in the presence or absence of the candidate ligand compound.

This assay can involve the separation of unbound labeled ligand compound from the sample. The separation step can be accomplished in any way known in the art, for instance, the immunostimulatory DNA binding protein can be immobilized on a substrate, i.e., a solid-phase support, prior to the binding reaction with the labeled ligand compound, and then unbound labeled ligand compound can be removed after the binding reaction by washing the solid-phase support.

An alternative approach employs labeled immunostimulatory DNA binding protein rather than a labeled ligand compound. For instance, the immunostimulatory DNA binding protein can be labeled and then contacted with the candidate ligand compound under conditions under which they would normally interact, such as conditions that mimic the in vivo environment. A separation step can then be performed to separate all of the unbound material. Finally, the amount of labeled immunostimulatory DNA binding protein which is bound to candidate ligand compound can be determined in a manner similar to the method described above. The method for determining the amount of bound labeled immunostimulatory DNA binding protein will depend on the type of marker used. The ability of an immunostimulatory DNA binding protein to bind with a ligand compound can be determined by comparing the amount of labeled immunostimulatory DNA binding protein which is bound to the candidate ligand compound in the presence or absence of the immunostimulatory DNA binding protein.

This assay can involve the separation of unbound labeled immunostimulatory DNA binding protein from the sample. The separation step can be accomplished in any way known in the art, in a manner similar to the method described above.

Many types of solid-phase supports are well known in the art. These include, for instance, but are not limited to, a plastic multiwell microtiter plate, a microarray plate, a bead, resin, a nitrocellulose filter, a slide, a silicon chip microarray, or a biomolecular interaction analysis (BIA) chip.

"Beads" as used herein include but are not limited to, for example, cellulose beads, controlled-pore glass beads, silica gels, polystyrene beads, optionally cross-linked with divinylbenzene and/or polyethylene glycol and optionally functionalized with amino, hydroxy, carboxyl, or halo groups, co-poly beads, polyacrylamide beads, latex beads, dimethylacrylamide beads, glass particles coated with hydrophobic polymers. The solid support can be coated with a variety of materials to produce a charged or neutral surface or can be coated with compounds such as a binding partner or linker molecules. In addition to beads, solid supports also include any type of insoluble matrix, such as an acrylamide derivative, agarose, cellulose, nylon, silica, magnetized particles, a cell, or a bacteriophage particle.

The immunostimulatory DNA binding protein or ligand compound can be attached to the solid phase support by any way known in the art, including but not limited to chemical cross-linking, nonspecific adhesion to a plastic surface, interaction with an antibody attached to the solid phase, and interaction with one of a pair of binding partners, such as biotin which is capable of binding with avidin or streptavidin or amino-groups that can be liked to activated sepharose.

The separation step can also be performed by capturing complexes formed in solution phase onto a solid-phase support, and then, if indicated, washing away unbound reactants. For instance, the materials can be separated by size using an ultrafiltration device or filter having pores which allow passage of the unbound material but which capture the bound complex. The same effect can be accomplished using a gel filtration matrix.

Detection of the bound ligand compound or of ligand compound-immunostimulatory binding protein complex can be accomplished using any of a number of methods well known in the art. These methods include but are not limited to EMSA, enzyme-linked immunosorbent assay (ELISA), ELIspot assay, surface plasmon resonance (BIAcore), fluorimetry, bioluminescence, refractometry, autoradiography, autoradiometry, PCR, and scintillation detection. The technique of surface plasmon resonance is discussed in, e.g., Szabo A et al. (1995) *Curr Opin Struct Biol* 5:699-705.

As mentioned above, the invention specifically provides a screening method for identifying a compound that inhibits the interaction between immunostimulatory DNA and an immunostimulatory DNA binding protein. An "immunostimulatory DNA binding inhibitor" is a compound which specifically inhibits the interaction between immunostimulatory DNA and an immunostimulatory DNA binding protein. An immunostimulatory DNA binding inhibitor will in some instances compete with immunostimulatory DNA for binding to an immunostimulatory DNA binding protein. In other instances an immunostimulatory DNA binding inhibitor will bind to a site on the immunostimulatory DNA binding protein that is distinct from the site for binding immunostimulatory DNA. An immunostimulatory DNA binding inhibitor will also be an immunostimulatory DNA functional inhibitor when the binding inhibitor is not also an immunostimulatory DNA functional mimic (see below).

The screening method for identifying a compound that inhibits the interaction between immunostimulatory DNA and an immunostimulatory DNA binding protein involves contacting at least one candidate inhibitor compound selected from a group of candidate immunostimulatory DNA binding inhibitors with an immunostimulatory DNA binding protein under conditions which, in the absence of an inhibitor, permit a reference immunostimulatory DNA to bind to the immunostimulatory DNA binding protein. The candidate inhibitor compound is contacted with the immunostimulatory DNA binding protein before, after, or simultaneously with contact between a labeled reference immunostimulatory DNA and the immunostimulatory DNA binding protein. The residual binding of the labeled reference immunostimulatory DNA to the immunostimulatory DNA binding protein is then detected. Detection of a decrease in binding of the reference immunostimulatory DNA indicates that the candidate inhibitor compound interferes with the binding of the reference immunostimulatory DNA to the immunostimulatory DNA binding protein. Examples of reference immunostimulatory ODNs include immunostimulatory ODNs SEQ ID NO:1, SEQ ID NO:2, SEQ ID NO:3, SEQ ID NO:10, SEQ ID NO:11, SEQ ID NO:12 and SEQ ID NO:15. A candidate immunostimulatory DNA binding inhibitor can itself be an immunostimulatory DNA or RNA, a nucleic acid without a CpG dinucleotide, or a compound other than a nucleic acid. Candidate immunostimulatory DNA binding inhibitor compounds can include but are not limited to peptide nucleic acids (PNAs), antibodies, polypeptides, carbohydrates, lipids, hormones, and small molecules. Candidate immunostimulatory DNA binding inhibitor compounds can further include variants of a reference immunostimulatory ODNincorporating any one or combination of the following: at least one substitution of a phosphorothioate-type bond for a phosphodiester bond, at least one substitution of a phosphodiester bond for a phosphorothioate-type bond, at least one substitution of one phosphorothioate-type bond for a different phosphorothioate-type bond, or at least one substitution of one nucleotide with a different nucleotide. Candidate inhibitor compounds can be generated as members of a combinatorial library of compounds.

This assay can involve the separation of both unbound unlabeled candidate inhibitor compounds and unbound labeled reference immunostimulatory DNA from the sample. The separation step can be accomplished in any way known in the art, in a manner similar to the separation method described above. Likewise, the detection of the remaining bound labeled reference immunostimulatory DNA can be accomplished in any way known in the art, in a manner similar to the detection method described above.

As mentioned above, the invention also specifically provides a screening method for identifying agonist compounds useful in immunotherapy. An "immunostimulatory DNA agonist compound" as used herein is a compound which binds to an immunostimulatory DNA binding protein and causes an increase in at least one aspect of an immune response that is ordinarily induced by immunostimulatory DNA. An immunostimulatory DNA agonist compound will in some instances compete with immunostimulatory DNA for binding to an immunostimulatory DNA binding protein. In other instances an immunostimulatory DNA agonist compound will bind to a site on the immunostimulatory DNA binding protein that is distinct from the site for binding immunostimulatory DNA.

The screening method for identifying agonist compounds useful in immunotherapy involves contacting at least one candidate agonist compound selected from a group of candidate immunostimulatory DNA agonist compounds with an immunostimulatory DNA binding protein under conditions which, in the absence of an agonist, permit a reference immunostimulatory DNA to bind to the immunostimulatory DNA binding protein. The candidate agonist compound is contacted with the immunostimulatory DNA binding protein before, after, or simultaneously with contact between a labeled reference immunostimulatory DNA and the immunostimulatory DNA binding protein. The residual binding of the labeled reference immunostimulatory DNA to the immunostimulatory DNA binding protein is then detected. Detection of a decrease in binding of the reference immunostimulatory DNA indicates that the candidate agonist compound interferes with the binding of the reference immunostimulatory DNA to the immunostimulatory DNA binding protein. Detection of an increase in binding of the reference immunostimulatory DNA indicates that the candidate agonist compound promotes or stabilizes the binding of the reference immunostimulatory DNA to the immunostimulatory DNA binding protein. Examples of reference immunostimulatory ODNs include immunostimulatory ODNs SEQ ID NO:1, SEQ ID NO:2, SEQ ID NO:3, SEQ ID NO:10, SEQ ID NO:11, SEQ ID NO:12 and SEQ ID NO:15. A candidate immunostimulatory DNA agonist compound can itself be an immunostimulatory DNA or RNA, a nucleic acid without a CpG dinucleotide, or a compound other than a nucleic acid. Candidate immunostimulatory DNA agonist compounds can include but are not limited to peptide nucleic acids (PNAs), antibodies, polypeptides, carbohydrates, lipids, hormones, and small molecules. Candidate immunostimulatory DNA agonist compounds can further include variants of a reference immunostimulatory ODN incorporating any one or combination of the substitutions described above for candidate immunostimulatory DNA binding inhibitor compounds. Candidate agonist compounds can be generated as members of a combinatorial library of compounds.

This assay can involve the separation of both unbound unlabeled candidate agonist compounds and unbound labeled reference immunostimulatory DNA from the sample. The separation step can be accomplished in any way known in the art, in a manner similar to the separation method described above. Likewise, the detection of the remaining bound labeled reference immunostimulatory DNA can be accomplished in any way known in the art, in a manner similar to the detection method described above.

The screening method for immunostimulatory DNA agonist compounds further requires contacting a candidate agonist compound with an immune cell and determining if the candidate agonist compound is an immunostimulatory DNA functional mimic.

An "immunostimulatory DNA functional mimic" as used herein is a compound which binds to an immunostimulatory DNA binding protein and mimics at least one function of immunostimulatory ODNs.

"Immune cell" as used herein refers to a bone marrow derived cell which can circulate or take up residence in a tissue and which participates in the recognition, presentation, or response to molecules or parts of molecules that are foreign or signal potential danger to the host. Examples of immune cells include B lymphocytes, T lymphocytes, monocytes, macrophages, natural killer (NK) cells, dendritic cells, mast cells, basophils, polymorphonuclear granulocytes, and the progenitors of all these cells. Methods of isolating and identifying immune cells are well known to those skilled in the art. See, e.g., *Current Protocols in Molecular Biology,* John Wiley & Sons, New York, 1999. Many of these immune cells secrete products in response to their activation. Such secreted products include immunoglobulins (antibodies) which bind specifically to cognate molecular antigens; cytokines, which act as signaling molecules to promote or inhibit proliferation or function of populations of immune cells; and chemokines, which act as chemoattractants important in the recruitment of immune cells to a site of immune stimulation. Examples of human immunoglobulins include molecules belonging to classes or isotypes denoted as IgG, IgM, IgE, IgA, and IgD. Human and rodent IgG immunoglobulins are further categorized into isotype subclasses denoted IgGI, IgG2, IgG3, IgG4, IgG2a, and IgG2b. Cytokines include interleukins, interferons, certain growth factors, and colony stimulating factors. Included among these are, e.g., interleukin (IL)-2, IL-4, IL-6, IL-10, IL-12, interferon (IFN)-$\gamma$, tumor necrosis factor (TNF)-$\alpha$, transforming growth factor (TGF)-$\beta$, and granulocyte colony stimulating factor (G-CSF). Chemokines include compounds in four subfamilies based on their structure: CXC, CC, C, and $CX_3C$. Examples of chemokines include MIP-1$\alpha$, MIP-1$\beta$, RANTES, MCP-1, MCP-2, IL-8, and GRO$\alpha$, among others. Assays for immunoglobulins, cytokines, and chemokines are well known to those skilled in the art. See, e.g., *Current Protocols in Molecular Biology,* John Wiley & Sons, New York, 1999. A number of commercial kits, particularly ELISAs, are available for most of these secreted products.

The screening method for immunostimulatory DNA functional mimics involves contacting at least one candidate immunostimulatory DNA agonist compound, selected from a group of candidate immunostimulatory DNA agonist compounds, with at least one immune cell under conditions which, in the presence of a reference immunostimulatory DNA, result in a measurable change in the state of immune cell activation or secreted products. Expected changes can include increased production and secretion of IL-6, IL-12, IFN-$\gamma$, TNF-$\alpha$, G-CSF, and IgG2a; increased B cell proliferation and NK cell activity; increased expression of cell surface markers of activation; and diminished production and secretion of IgE and IL-4. Such measurements can be accomplished using appropriately selected specific probes in ELISA, Western blotting, Northern blotting, quantitative reverse transcriptase-polymerase chain reaction (RT-PCR), or flow cytometry, kinase assays, or assays for production of reactive oxygen species or reduction of intracellular glutathione content.

The invention also provides for a screening method for immunostimulatory DNA functional inhibitors. An "immunostimulatory DNA functional inhibitor" as used herein is a compound which binds to an immunostimulatory DNA binding protein and prevents at least one aspect of the immune response that is ordinarily induced by immunostimulatory ODNs. The screening method for immunostimulatory DNA functional inhibitors involves contacting at least one candidate immunostimulatory DNA competitor compound, selected from a group of candidate immunostimulatory DNA competitor compounds, with at least one immune cell under conditions which, in the presence of a reference immunostimulatory DNA, result in a measurable change in the state of immune cell activation or secreted products. Expected changes can include decreased production and secretion of IL-6, IL-12, IFN-γ, TNF-α, G-CSF, and IgG2a; decreased B cell proliferation and NK cell activity; decreased expression of cell surface markers of cell activation; and increased or undiminished production and secretion of IgE and IL-4. Such measurements can be accomplished using methods described above for immunostimulatory DNA agonist compounds.

Another aspect of the invention provides a method of screening for compounds which directly compete with immunostimulatory DNA for binding to immunostimulatory DNA binding proteins. An "immunostimulatory DNA binding competitor" as used herein is a compound which can interact with an immunostimulatory DNA binding protein and prevent the binding of another immunostimulatory ODN to the immunostimulatory DNA binding protein. In some cases, a candidate immunostimulatory DNA binding competitor can be an inhibitor of immunostimulatory DNA function. In other instances the candidate immunostimulatory DNA competitor compound can be an immunostimulatory DNA functional mimic. In other instances, the candidate compound can have mixed immunostimulatory DNA mimic and inhibitor properties. The immunostimulatory DNA binding competitor may bind to the same site on the binding protein as immunostimulatory ODN, thus, physically blocking the interaction between immunostimulatory ODN and the binding protein. Alternately the competitor may bind to a remote site and still prevent the interaction between the immunostimulatory ODN and the binding protein, i.e., by causing a conformational change.

The screening method for immunostimulatory DNA binding competitors involves contacting at least one candidate immunostimulatory DNA binding competitor compound selected from a group of candidate immunostimulatory DNA binding competitors with an immunostimulatory DNA binding protein under conditions which, in the absence of a competitor, permit a reference immunostimulatory DNA to bind or remain bound to the immunostimulatory DNA binding protein. The candidate binding competitor compound is contacted with the immunostimulatory DNA binding protein before, after, or simultaneously with contact between labeled reference immunostimulatory DNA and the immunostimulatory DNA binding protein. The residual binding of the labeled reference immunostimulatory DNA to the immunostimulatory DNA binding protein is then detected. Detection of a decrease in binding of the reference immunostimulatory DNA indicates that the candidate competitor compound interferes with the binding of the reference immunostimulatory DNA to the immunostimulatory DNA binding protein. Examples of reference immunostimulatory ODNs include immunostimulatory ODNs SEQ ID NO:1, SEQ ID NO:2, SEQ ID NO:3, SEQ ID NO:10, SEQ ID NO:11, SEQ ID NO:12 and SEQ ID NO:15. A candidate immunostimulatory DNA binding competitor can itself be an immunostimulatory DNA or RNA, a nucleic acid without a CpG dinucleotide, or a compound other than a nucleic acid. Possible immunostimulatory DNA binding competitor compounds can include but are not limited to nucleic acids as described above, peptide nucleic acids (PNAs), antibodies, polypeptides, carbohydrates, lipids, hormones, and small molecules. Possible immunostimulatory DNA binding competitor compounds can further include variants of a reference immunostimulatory ODN incorporating any one or combination of the substitutions described above for candidate immunostimulatory DNA binding inhibitor compounds. Candidate immunostimulatory DNA binding competitors can be generated as members of a combinatorial library of compounds.

This assay can involve the separation of both unbound unlabeled candidate competitor compounds and unbound labeled reference immunostimulatory DNA from the sample. The separation step can be accomplished in any way known in the art, in a manner similar to the separation method described above. Likewise, the detection of the remaining bound labeled reference immunostimulatory DNA can be accomplished in any way known in the art, in a manner similar to the detection method described above.

In addition to being useful for identifying inhibitors and mimics, the screening assays of the invention are also useful for identifying candidate immunostimulatory ODN competitors to allow the investigation of structure/activity relationships between immunostimulatory ODNs and immunostimulatory DNA binding proteins.

C. Method for Optimizing Immunostimulatory ODN for Immune Stimulation. Yet another aspect of the invention provides a method for optimizing a selected immunostimulatory ODN for immune stimulation. "Optimizing" as used herein refers to an iterative process of introducing changes to an existing system or compound and evaluating the functional significance of each change, followed by selecting the resulting system or compound associated with a functional outcome that is most improved; these steps are repeated until a desired endpoint is achieved or it appears further changes will not improve the functional outcome. The same objective can be achieved in a parallel manner by generating a library of closely related compounds and screening the library for the compound or compounds possessing the most favorable embodiment of the characteristic being optimized. In this particular instance, optimizing a selected immunostimulatory ODN for immune stimulation involves testing a panel of structurally related immunostimulatory ODNs for their ability to bind to immunostimulatory DNA binding protein. The screening method involves contacting at least one candidate optimized immunostimulatory DNA selected from a group of candidate optimized immunostimulatory DNAs with an immunostimulatory DNA binding protein under conditions which, in the absence of a competitor, permit a reference immunostimulatory DNA to bind or remain bound to the immunostimulatory DNA binding protein. The candidate optimized immunostimulatory DNA is contacted with the immunostimulatory DNA binding protein before, after, or simultaneously with contact between the labeled reference immunostimulatory DNA and the immunostimulatory DNA binding protein. The residual binding of the labeled reference immunostimulatory DNA to the immunostimulatory DNA binding protein is then detected. Detection of a decrease in binding of the reference immunostimulatory DNA indicates that the candidate optimized immunostimulatory DNA interferes with the binding of the reference immunostimulatory DNA to the immunostimulatory DNA binding protein. Examples of reference immunostimulatory ODNs include immunostimulatory ODNs SEQ ID NO:1, SEQ ID NO:2, SEQ ID NO:3, SEQ ID NO:10, SEQ ID NO:11, SEQ ID NO:12 and SEQ ID NO:15. A candidate optimized immunostimulatory DNA can be an immunostimulatory DNA, a CpG ODN, or a peptide nucleic acid with a CpG dinucleotide. Candidate optimized immunostimulatory DNAs can include variants of a reference immunostimulatory ODN incorporating any one or combination of the substitutions described above for candidate immunostimulatory DNA binding inhibitor compounds. Candidate optimized immunostimulatory DNAs can be generated as members of a combinatorial library of compounds, for example using SELEX technology. Tuerk C and Gold L (1990) *Science* 249:505-510; Gold L et al. (1995) *Annu Rev Biochem* 64:763-797.

This assay can involve the separation of both unbound unlabeled candidate optimized immunostimulatory DNAs and unbound labeled reference immunostimulatory DNA from the sample. The separation step can be accomplished in any way known in the art, in a manner similar to the separation method described above. Likewise, the detection of the remaining bound labeled reference immunostimulatory DNA can be accomplished in any way known in the art, in a manner similar to the detection method described above.

The screening assay can also be performed as a competition between labeled candidate optimized immunostimulatory DNAs and unlabeled reference immunostimulatory DNA for the immunostimulatory DNA binding protein. In this format, binding of the labeled optimized immunostimulatory DNA to the immunostimulatory DNA binding protein is then detected. Detection of bound optimized immunostimulatory DNA indicates that the candidate optimized immunostimulatory DNA interferes with the binding of the reference immunostimulatory DNA to the immunostimulatory DNA binding protein.

The screening assay can also be performed by contacting labeled immunostimulatory DNA binding protein to immobilized immunostimulatory DNA. In this format a panel of candidate optimized immunostimulatory DNAs can be presented in an array fashion on a silicon chip or in a plastic multiwell microtiter or microarray plate. Alternatively, each candidate optimized immunostimulatory DNA can be separately coupled to a bead, a resin, a nitrocellulose filter, a slide, or a biomolecular interaction analysis (BIA) chip. After contacting the immunostimulatory DNA binding protein with the immobilized candidate immunostimulatory DNAs and, if indicated, washing away unbound immunostimulatory DNA binding protein, detection of complexes formed between the immobilized immunostimulatory DNA and the immunostimulatory DNA binding protein provides the basis for selecting particular immunostimulatory DNAs as optimized.

D. Screening Methods Useful for Identifying Cellular Targets of Immunostimulatory DNA Binding Proteins. According to another aspect of the invention, the screening assays of the invention also allow for the identification of cellular target molecules. The method involves screening a panel of candidate target molecules with a complex formed between an immunostimulatory ODN and an immunostimulatory DNA binding protein, selecting the candidate target molecules that bind to the complex, and determining the identity of the bound candidate target molecules.

A "complex formed between an immunostimulatory ODN and an immunostimulatory DNA binding protein" as used herein is a bimolecular complex formed by the binding of an immunostimulatory DNA or an immunostimulatory DNA competitor to an immunostimulatory DNA binding protein. Such a bimolecular complex may be part of a larger complex involving at least one other nucleic acid molecule or other non-nucleic acid molecule, e.g., another polypeptide molecule. The immunostimulatory ODN can be tethered to the immunostimulatory DNA binding protein in the complex formed between an immunostimulatory ODN and an immunostimulatory DNA binding protein, for instance by crosslinking by ultraviolet light.

A "cellular target molecule" is a molecule bound by a complex formed between an immunostimulatory ODN and an immunostimulatory DNA binding protein. Examples of possible cellular target molecules include nucleic acids, other proteins, protein-nucleic acid complexes, carbohydrates, hormones, and lipids. Panels of candidate cellular target molecules can include, for example, a cDNA library, a nucleic acid microchip, a polypeptide library, and a cell lysate. A marker as previously described can be used to label the immunostimulatory ODN forming part of the complex formed between an immunostimulatory ODN and an immunostimulatory DNA binding protein. Alternatively, a marker can be used to label an immunostimulatory DNA binding protein forming part of the complex formed between an immunostimulatory ODN and an immunostimulatory DNA binding protein. As another alternative, a marker can be used to label the candidate target molecule to be bound by the complex formed between an immunostimulatory ODN and an immunostimulatory DNA binding protein. In this method the immunostimulatory DNA binding protein is selected from the group consisting of nucleolin, hnRNP D, AUF1, hnRNP A1, lupus La protein, and isoforms, fragments, variants, and equivalents thereof. Examples of reference immunostimulatory ODNs include immunostimulatory ODNs SEQ ID NO: 1, SEQ ID NO:2, SEQ ID NO:3, SEQ ID NO:10, SEQ ID NO:11, SEQ ID NO:12 and SEQ ID NO:15.

The screening step of the method involves contacting a complex formed between an immunostimulatory ODN and an immunostimulatory DNA binding protein with at least one candidate target molecule. Preferably the reaction is performed under conditions that, in the absence of a target molecule, permit the complex to remain substantially intact. In other words, the preferred conditions should not cause the complex to dissociate or self-aggregate. The selection step of the method involves detecting the presence of candidate target molecule bound to the complex. This step involves the separation of unbound components, i.e., candidate target molecules and unbound complexes, from the sample. The separation step can be accomplished in any way known in the art, in a manner similar to the separation method described above. Likewise, the detection of the remaining bound labeled reference immunostimulatory DNA can be accomplished in any way known in the art, in a manner similar to the detection method described above. For example, following the step of contacting the complex formed between an immunostimulatory ODN and an immunostimulatory DNA binding protein with labeled candidate target molecule, the sample can be passed over a filter that can trap compounds at least the size of the complex but will not trap unbound labeled target molecule. Detection of the marker on the filter, above any background binding which occurs without complex present, indicates the presence of candidate target molecule bound to the complex formed between an immunostimulatory ODN and an immunostimulatory DNA binding protein.

Depending on the nature of the candidate target molecule, the step of identifying the bound target molecule then involves determining its sequence or structure. In some instances the identity will be apparent from the manner in which the panel of candidate target molecules was prepared, e.g., by looking up the identity of the compound prepared as part of a spatially arrayed combinatorial library. In other instances it will be necessary to perform nucleotide or peptide sequencing following standard techniques well known in the art. In yet other instances, where the candidate target molecules are not isolated molecules but rather are present in a sample representing cell lysate, it may be necessary to dissociate the target molecule from the complex formed between an immunostimulatory ODN and an immunostimulatory DNA binding protein and then analyze the target molecule using, e.g., MALDI-MS or ESI-MS, Northern or Western blotting, or polymerase chain reaction.

The invention embraces variants of the immunostimulatory DNA binding proteins described above. As used herein, a "variant" of an immunostimulatory DNA binding protein is a polypeptide which contains one or more modification(s) to the primary amino acid sequence of an immunostimulatory DNA binding protein. Modifications which create an immunostimulatory DNA binding protein variant are typically made to the nucleic acid which encodes the immunostimulatory DNA binding protein, and can include deletions, point mutations, truncations, amino acid substitutions and addition of amino acids or non-amino acid moieties to: 1) retain functional activity of an immunostimulatory DNA binding protein; 2) enhance a property of an immunostimulatory DNA binding protein, such as protein stability in an expression system or the stability of protein-protein binding; 3) provide a novel activity or property to an immunostimulatory DNA binding protein, such as addition of an antigenic epitope or addition of a detectable moiety; or 4) to provide equivalent or better binding to an immunostimulatory DNA or other molecule. Alternatively, modifications can be made directly to the polypeptide, such as by cleavage, addition of a linker molecule, addition of a detectable moiety, such as biotin, addition of a fatty acid, a histidine tag, and the like. Modifications also embrace fusion proteins comprising all or part of the immunostimulatory DNA binding protein amino acid sequence. One of skill in the art will be familiar with methods for predicting the effect on protein conformation of a change in protein sequence, and can thus "design" a variant immunostimulatory DNA binding protein according to known methods. One example of such a method is described by Dahiyat and Mayo in *Science* 278:82 (1997), whereby proteins can be designed *de novo.* The method can be applied to a known protein to vary a only a portion of the polypeptide sequence. By applying the computational methods of Dahiyat and Mayo, specific variants of a polypeptide can be proposed and tested to determine whether the variant retains a desired conformation.

Variants can include immunostimulatory DNA binding proteins which are modified specifically to alter a feature of the polypeptide unrelated to its physiological activity. For example, cysteine residues can be substituted or deleted to prevent unwanted disulfide linkages. Similarly, certain amino acids can be changed to enhance expression of an immunostimulatory DNA binding protein by eliminating proteolysis by proteases in an expression system (e.g., dibasic amino acid residues in yeast expression systems in which KEX2 protease activity is present).

Mutations of a nucleic acid which encodes an immunostimulatory DNA binding protein preferably preserve the amino acid reading frame of the coding sequence, and preferably do not create regions in the nucleic acid which are likely to hybridize to form secondary structures, such a hairpins or loops, which can be deleterious to expression of the variant polypeptide.

Mutations can be made by selecting an amino acid substitution, or by random mutagenesis of a selected site in a nucleic acid which encodes the polypeptide. Variant polypeptides are then expressed and tested for one or more activities to determine which mutation provides a variant polypeptide with the desired properties. Further mutations can be made to variants (or to non-variant immunostimulatory DNA binding proteins) which are silent as to the amino acid sequence of the polypeptide, but which provide preferred codons for translation in a particular host. The preferred codons for translation of a nucleic acid in, e.g., *E. coli,* are well known to those of ordinary skill in the art. Still other mutations can be made to the noncoding sequences of an immunostimulatory DNA binding protein gene or cDNA clone to enhance expression of the polypeptide.

The skilled artisan will realize that conservative amino acid substitutions may be made in immunostimulatory DNA binding proteins to provide functionally equivalent variants of the foregoing polypeptides, i.e, the variants retain the functional capabilities of the immunostimulatory DNA binding proteins and can be used in the assays of the invention. As used herein, a "conservative amino acid substitution" refers to an amino acid substitution which does not alter the relative charge or size characteristics of the protein in which the amino acid substitution is made. Variants can be prepared according to methods for altering polypeptide sequence known to one of ordinary skill in the art such as are found in references which compile such methods, e.g., *Molecular Cloning: A Laboratory Manual*, J. Sambrook, et al., eds., Second Edition, Cold Spring Harbor Laboratory Press, Cold Spring Harbor, New York, 1989, or *Current Protocols in Molecular Biology,* F.M. Ausubel, et al., eds., John Wiley & Sons, Inc., New York. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

Thus the use in the screening assays and products of the invention of functionally equivalent variants of immunostimulatory DNA binding proteins, i.e., variants of immunostimulatory DNA binding proteins which retain the function of the natural immunostimulatory DNA binding protein, are contemplated. Conservative amino acid substitutions in the amino acid sequence of immunostimulatory DNA binding proteins to produce functionally equivalent variants of immunostimulatory DNA binding proteins typically are made by alteration of a nucleic acid encoding immunostimulatory DNA binding protein. Such substitutions can be made by a variety of methods known to one of ordinary skill in the art. For example, amino acid substitutions may be made by PCR-directed mutation, site-directed mutagenesis according to the method of Kunkel (Kunkel TA (1985) *Proc Nat Acad Sci USA* 82:488), or by chemical synthesis of a gene encoding an immunostimulatory DNA binding protein. The activity of functionally equivalent fragments of immunostimulatory DNA binding proteins can be tested by cloning the gene encoding the altered immunostimulatory DNA binding protein into a bacterial or mammalian expression vector, introducing the vector into an appropriate host cell, expressing the altered immunostimulatory DNA binding protein, and testing for a functional capability of the immunostimulatory DNA binding proteins in the assays disclosed herein.

E. Isolated Complex Formed Between an Immunostimulatory ODN and an Immunostimulatory DNA Binding Protein. The invention also provides for a compositions that are complexes formed between an immunostimulatory ODN and an immunostimulatory DNA binding protein. Such complexes are useful in screening for immunostimulatory DNA target molecules. The immunostimulatory ODN can be tethered to the immunostimulatory DNA binding protein in the complex formed between an immunostimulatory ODN and an immunostimulatory DNA binding protein, for instance by crosslinking by ultraviolet light. A marker can be used to label the immunostimulatory ODN forming part of the complex. Alternatively, a marker can be used to label an immunostimulatory DNA binding protein forming part of the complex. Examples of immunostimulatory ODNs and of immunostimulatory DNA binding proteins that can participate in the complex are as described above.

F. Kit for Identifying Cellular Targets of an Immunostimulatory Oligonucleotide. In another aspect of the invention, an immunostimulatory DNA binding protein can be placed in a vial and incorporated into a kit to be used in identification of cellular targets of immunostimulatory DNA. In certain embodiments, immunostimulatory DNA according to the invention can also be placed in a vial and included in the same kit. The kits can include instructions or other printed material for preparing a complex formed between an immunostimulatory ODN and an immunostimulatory DNA binding protein and for using the complex to screen a panel of nucleic acid molecules to identify cellular targets of an immunostimulatory ODN. Examples of immunostimulatory DNA binding proteins that can be included in a kit are nucleolin, lupus La protein, hnRNP D, AUF1, hnRNP A1, and isoforms, fragments, variants, and equivalents thereof. Examples of reference immunostimulatory ODNs include immunostimulatory ODNs SEQ ID NO:1, SEQ ID NO:2, SEQ ID NO:3, SEQ ID NO:10, SEQ ID NO:11, SEQ ID NO:12 and SEQ ID NO:15.

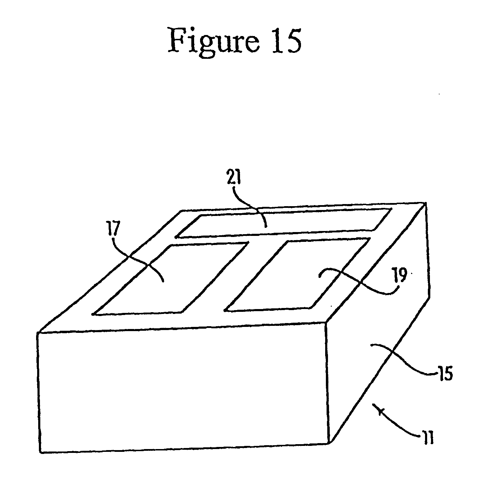

A kit embodying features of the present invention, generally designated by the numeral **11,** is illustrated in **FIG. 15**. Kit 11 is comprised of the following major elements: packaging **15,** a compartment containing an immunostimulatory DNA binding protein **17,** an immunostimulatory DNA **19** that binds the immunostimulatory DNA binding protein **17**, and instructions **21.** Packaging **15** is a box-like structure for holding immunostimulatory DNA binding protein **17,** an immunostimulatory DNA **19** and instructions **21.** Individuals skilled in the art can readily modify packaging **15** to suit individual needs.

Each of the limitations of the invention can encompass various embodiments of the invention. It is, therefore, anticipated that each of the limitations of the invention involving any one element or combinations of elements can be included in each aspect of the invention.

## EXAMPLE 1

### Proteins in Crude Extracts from B Lymphocytes Bind to Immunostimulatory ODN

*Cell Culture*. Suspension cultures of Ramos cells, a human Burkitt lymphoma B cell line (ATCC CRL-1923 or CRL-1596), were grown in RPMI 1640 medium with 10% fetal bovine serum, 1.5 mM L-glutamine, 100 U/ml penicillin, 100 µg/ml streptomycin at 37°C, 5% $CO_2$.

*Preparation of cell extracts.* Cells were harvested by low speed centrifugation, washed two times with cold phosphate buffered saline, and 5 x $10^9$ cells were resuspended in 15 ml of hypotonic buffer A [10 mM HEPES/KOH (pH7.9), 10 mM KCl, 1.5 mM $MgCl_2$, 0.5 mM dithiothreitol (DTT), 0.5 mM phenylmethylsulfonyl fluoride (PMSF), 30 μg/ml Leupeptin, 50 μg/ml Aprotinin, 5 mg/ml Antipain, 5 μg/ml Pepstatin]. After incubation on ice for 15 minutes the cytoplasmic membranes were disrupted in a Dounce homogenizer by applying 15 to 20 strokes. The suspension was centrifuged at 1000 x g for 5 minutes and the supernatant was removed as cytoplasmic fraction and stored at -70°C. Protein concentrations were measured using a Bradford protein assay (Bio-Rad) according to instructions supplied by the manufacturer.

*Oligodeoxyribonucleotides.* Oligodeoxyribonucleotides (ODNs) were obtained from Operon Technologies, Inc. (Alameda, CA). The various ODNs used for DNA-binding analysis were:

| | | | |
|---|---|---|---|
| #2059: | 5'-TCGTCGTTTTGTCGTTTTGTCGTT-3' | (CpG) | SEQ ID NO:1 |
| #2080: | 5'-TCGTCGTTCCCCCCCCCCCC-3' | (CpG) | SEQ ID NO:2 |
| #1619: | 5'-TCCATGTCGTTCCTGATGCT-3' | (CpG) | SEQ ID NO:3 |
| #1765: | 5'-TCCATGTZGTTCCTGATGCT-3' | (methylated CpG) | SEQ ID NO:4 |
| #2049: | 5'-TCCATGTGCTTCCTGATGCT-3' | (non-CpG) | SEQ ID NO:5 |
| #5011: | 5'-TZGTZGTTTTGTZGTTTTGTZGTT-3' | (methylated CpG) | SEQ ID NO:6 |
| #5015: | 5'-TGCTGCTTTTGTGCTTTTGTGCTT-3' | (non-CpG; GpC) | SEQ ID NO:7 |
| #5009: | DIG-5'-CCCCCCCCCCCCCCCCCCCC-3' | (poly C) | SEQ ID NO:8 |
| #5017: | DIG-5'-TTTTTTTTTTTTTTTTTTTT-3' | (poly T) | SEQ ID NO:9 |
| #5001: | $NH_2$-5'-TCGTCGTTTTGTCGTTTTGTCGTT-3' | (CpG) | SEQ ID NO:10 |
| #5007: | DIG-5'-TCGTCGTTTTGTCGTTTTGTCGTT-3' | (phosphorothioate CpG) | SEQ ID NO:11 |
| #5004: | DIG-5'-TCGTCGTTTTGTCGTTTTGTCGTT-3' | (CpG) | SEQ ID NO:12 |
| #5012: | DIG-5'-TZGTZGTTTTGTZGTTTTGTZGTT-3' | (methylated CpG) | SEQ ID NO:13 |
| #5016: | DIG-5'-TGCTGCTTTTGTGCTTTTGTGCTT-3' | (non-CpG; GpC) | SEQ ID NO:14 |
| #5008: | DIG-5'-TCGTCGTTCCCCCCCCCCCC-3' | (phosphorothioate CpG) | SEQ ID NO:15 |
| #5029: | $NH_2$-5'-TCGTCGTTTTGTCGTTTTGTCGTT-3' | (phosphorothioate CpG) | SEQ ID NO:16 |

| | | | |
|---|---|---|---|
| #5018: | DIG-5'-TTTTTTTTTTTTTTTTTTTT-3' | (phosphorothioate poly T) | SEQ ID NO:17 |
| #5030: | DIG-5'-CCCCCCCCCCCCCCCCCCCC-3' | (phosphorothioate poly C) | SEQ ID NO:18 |
| #5027: | 5'-TCGTCGTTTTGTCGTTTTGTCGTT-3'-DIG | (CpG) | SEQ ID NO:19 |
| #5021: | 5'-TTTTTTTTTTTTTTTTTTTT-3' | (poly T) | SEQ ID NO:20 |
| #5031: | 5'-CCCCCCCCCCCCCCCCCCCC-3' | (poly C) | SEQ ID NO:21 |
| #2060: | 5'-CTGGTCTTTCTGGTTTTTTTCTGG-3' | (non-CpG) | SEQ ID NO:22 |

Oligonucleotide backbones were phosphodiester unless otherwise noted. CpG motifs are underlined, Z designates 5-methylcytosine, and DIG represents digoxigenin.

*DNA binding assay.* For electrophoretic mobility shift assays (EMSAs), ODNs were end-labeled with T4-polynucleotide kinase (New England Biolabs) and [$\gamma$-$^{32}$P]ATP according to the supplier. Binding reactions were performed with 2 - 4 µg protein of whole cell extracts, or less with purified protein extracts, in DNA binding buffer [10 mM Tris-HCl (pH 7.5), 150 mM KCl, 1 mM $MgCl_2$, 0.5 mM EDTA, 1 mM DTT, 0.1 % Triton X-100, and 8% glycerol] supplemented with 100 - 500 ng of poly(dI,dC)-(dI,dC)) and 20,000 - 40,000 cpm [$\gamma$-$^{32}$P]ATP-labeled ODN or 10 ng of digoxigenin-labeled ODN in 10 µl total volume. If unlabeled competitor ODNs (1 - 500 ng) were used, the samples were incubated for 10 minutes at room temperature prior to addition of labeled ODN. Following incubation for 30 minutes at room temperature, loading buffer was added and the probes were electrophoresed on a 5% polyacrylamide gel at 10 V per cm in 22 mM Tris borate-0.5 mM EDTA buffer. Alternatively the samples were analyzed on a 1% agarose gel at 12 V per cm in 0.5 x TAE buffer. Gels were dried and exposed to film for 6 to 20 hours at -80°C with an intensifying screen.

In some EMSAs 10 ng of non-radioactive digoxigenin-labeled ODN were used for binding, rather than [$\gamma$-$^{32}$P] ATP-labeled ODN. In these assays the non-radioactive labeling gels were electroblotted on a Biodyne Plus membrane (Pierce), UV-crosslinked for one minute, and developed with a peroxidase-DIG-Luminescence Kit (Roche) used according to the supplier.

*UV crosslinking in solution and calculation of molecular weight (MW).* From a partially purified protein extract 1 - 5 µl (0.75 µg total protein) were mixed in DNA binding buffer supplemented with 100 - 300 ng poly(dI-dC)·poly(dI-dC)] and 20,000 to 40,000 cpm [$\gamma$-$^{32}$P]ATP-labeled ODN or with 10 ng of digoxigenin-labeled ODN, and, where indicated, with 100 to 1000 ng competitor ODN in a final volume of 10 µl. If competitor was added, the samples were incubated for 10 minutes at room temperature prior to addition of the labeled ODN. After incubation at room temperature for 30 minutes, the samples were transferred into the wells of a 96-well plate and DNA-protein complexes were crosslinked with UV light in a Stratalinker (Stratagene) for 3 minutes or the indicated time. The probes were then analyzed by SDS-polyacrylamide gel electrophoresis (SDS-PAGE, see below). Prestained protein marker (Bio-Rad, low range) was used as a MW standard. Gels run with radioactive label were dried on Whatman paper and autoradiographed. For gels run with non-radioactive label, proteins were transferred from the gel onto a Biodyne Plus membrane by electroblotting and then detected using a Chemiluminescence Detection Kit (Roche) according to the supplier's instructions.

After the exposure was developed, the MW marker lanes were marked on the film and the distance between each band of the marker and the running front was measured. Plotting the distance in mm (linear scale) against the molecular weight of the marker proteins (logarithmic scale) yielded a standard curve which was used to calculate the molecular weight of the crosslinked protein-ODN complexes. The calculated molecular weight of each crosslinked protein-ODN complex was then corrected by subtracting the MW of the appropriate ODN probe as calculated according to the following formula:

$$MW\ (gm/mole) = (251 \times n_A) + (245 \times n_T) + (267 \times n_G) + (230 \times n_C) + (61 \times (N-1))$$

where N is the total number of bases, $n_A$ is the number of adenine bases, $n_T$ is the number of thymine bases, $n_G$ is the number of guanine bases, and $n_C$ is the number of cytosine bases. Using this formula, ODN #2059 (SEQ ID NO: 1) has a calculated MW of 7355 Da. While the phosphate groups (61 x (N-1)) were added to the bases, the hydration and the associated cations were not included in this calculation.

*Denaturing protein gels.* Crosslinked DNA-protein complexes were analyzed using conventional SDS-PAGE. The separation gel contained 7.5% - 10% acrylamide, 1 M Tris-HCl (pH 8.45), 0.1% SDS, and 0.5% glycerol. The stacking gel consisted of 4% acrylamide, 0.75 M Tris-HCl (pH 8.45) and 0.075% SDS. Protein samples were mixed with SDS sample buffer [50 mM Tris-Cl (pH 6.8), 1 % β-mercaptoethanol, 2% SDS, 0.1% bromphenol blue, 10% glycerol], boiled for 10 minutes, chilled on ice, and loaded on the gel. The electrophoresis buffer for the cathode was 0.1 M Tris-HCl, 0.1 M Tricin and 0.1 % SDS with a pH of 8.25. The anode buffer was 0.2 M Tris-HCl (pH 8.8). After electrophoresis the gel was dried on Whatman 3M paper and exposed to X-ray film.

*Silver stain assay*. After electrophoresis, the SDS gel was transferred into solution 1 [50% methanol, 12% TCA in $H_2O$] and incubated for 20 min. Then the gel was transferred in solution 2 [10% ethanol, 5% acetic acid] and incubated for another 10 min. Afterwards solution 2 was changed to solution 3 [0.06% ammonium persulfate in water] and incubated for another 10 min. Then solution 3 was replaced by solution 4 [0.1% $AgNO_3$ in water] and incubated for 15 min. Solution 4 was changed to developing solution [2% $K_2CO_3$, 0.04% $H_2CO$], and the reaction was stopped with 1% acetic acid when the protein bands were clearly visible.

*Results.* Crude cytoplasmic or nuclear extracts from Ramos B lymphocytes yielded several complexes as observed in EMSAs when the 24-mer immunostimulatory ODN #2059 was used as labeled probe (**FIG. 1**, lane 1). Complex C5 appeared to be composed of several bands running close to one another, rather than a single band. The slowly migrating complex C0 was best observed at high protein concentrations (>15 μg/lane) (**FIG. 1**, lane 1). DNA binding assays in the presence of various concentrations of unlabeled competitor ODN, either immunostimulatory ODN #2059 (**FIG. 1**, lanes 2-4) or non-immunostimulatory ODN #2060 (**FIG. 1**, lanes 5-7), revealed that complex C3 was specific for CpG motifs, whereas C4 and C5 were not. Complexes C0, C1, and C2 showed only weak specificity for immunostimulatory ODN (**FIG. 1**).

When different digoxigenin-labeled ODN were added to cytosolic extracts of Ramos (B lymphocyte) cells, YT-Indy (NK) cells and HeLa (carcinoma) cells and analyzed by crosslink assays, three sets of bands corresponding to ODN binding proteins could be distinguished (**FIG. 2**): a high MW set composed of about three bands in the range of 113 to 104 kDa (113, 108, and 104 kDa); a middle MW set composed of at least four bands in the range of 93 to 74 kDa (93, 88, 78, and 74 kDa); and a low MW set was composed of at least four or five bands in the range of 58 to 36 kDa (58, 55, 51, 43, and 36 kDa). Not evident from **FIG. 2** but also present within the middle MW set are at least two more ODN binding protein bands, which crosslinked only poorly in the present assays. Lanes in **FIG. 2** correspond to the following crosslink partners for cytosol extract from each indicated cell line: lane 1, phosphorothioate CpG ODN #5007; lane 2, phosphodiester CpG ODN #5004; lane 3, phosphodiester methylated CpG ODN #5012; lane 4, phosphodiester non-CpG ODN #5016; lane 5, poly T ODN #5017; and lane 6, poly C ODN #5009.

The proteins in the high MW set crosslinked with relatively equal efficiency to phosphorothioate and phosphodiester ODN. However, binding of high MW proteins to poly T ODN or poly C ODN was weaker.

Unlike proteins in the high MW and the middle MW sets, proteins in the low MW set crosslinked only very weakly, if at all, to phosphorothioate ODN. When comparing the crosslinking efficiency of different ODN, these low MW proteins crosslinked best to poly T ODN and only poorly, if at all, to poly C ODN.

A striking difference was seen by comparing crosslinks in Ramos B-cell cytosol to NK-cell cytosol. Two of the high MW bands (106 and 108 kDa) appeared much more prominent in B-cell and HeLa-cell cytosol than in NK-cell cytosol. Nuclear extracts of these cell lines displayed less prominent differences in ODN binding protein distribution among the cell lines.

## EXAMPLE 2

## Chromatographic Isolation of Immunostimulatory DNA Binding Proteins

Chromatographic isolation of immunostimulatory DNA binding proteins from cell lysates proceeded along successive steps of heparin affinity chromatography, anion or cation exchange chromatography, and immunostimulatory ODN-coupled sepharose column affinity chromatography.

*A. Affinity chromatography with heparin column.* The first purification step of immunostimulatory DNA binding proteins from cell extracts was performed on a heparin affinity column. All purification steps were carried out at 4°C. Generally only cytoplasmic fractions were used, since it is believed that immunostimulatory DNA binding proteins can be isolated from the cytosol with fewer other contaminating DNA binding proteins than can immunostimulatory DNA binding proteins isolated from the nuclei. The crude cytosolic or nuclear extracts were adjusted to buffer HA [50 mM Tris-HCl (pH 8.4), 50 mM NaCl, 1 mM EDTA] on a PD10 gel filtration column (Pharmacia Biotech) according to the manufacturer. The samples were then filtered sequentially through 1.2 μm, 0.8 μm, 0.45 μm, and 0.2 μm syringe filters (Gelman, PVDF membrane). The processed protein extracts, containing up to 240 mg of protein, were loaded on a 7.5 ml Heparin Hyper D column (Biosepra). With the flow rate set at I ml/min, proteins were eluted with increasing salt concentration, from 0 to 1000 mM NaCl. Fractions were collected and stored at -80°C. Four identical heparin runs were performed, each starting from approximately 18 ml of cell pellet. The resulting fractions were analyzed by EMSA and crosslink assays, as described above.

*Results.* As a first separation step, different immunostimulatory DNA binding proteins present in the crude cytosol were bound to a heparin affinity column and eluted with an increasing salt gradient. The resulting fractions from the heparin affinity chromatography were divided into four pooled fractions, termed H1, H2, H3, and H4, which were subsequently analyzed in EMSA and crosslink assays with CpG ODN #2059. For some assays fractions H1 and H2 were combined and used as a single pooled fraction, H1/2. Results from this analysis are summarized in Table 2 below and shown in **FIG. 3** (panel A, EMSA; panel B, crosslink). Lane L in both panels of **FIG. 3** is the load fraction, and lane U is the unbound fraction (4 times more loaded than in any other lane). Fraction numbers within pooled fractions H1/2, H3, and H4 are indicated. MW standard markers are shown on the right in panel B. Binding proteins BP1 - BP5 are also indicated.

Table 2.

| Analysis of Heparin Affinity Chromatography Fractions with CpG ODN #2059 | | | |
|---|---|---|---|
| Fraction | mM NaCl | Binding Proteins | Crosslink Bands MW (kDa) |
| Unbound | N.A. | BP5 | -- |
| H1/2 | 100 - 300 | BP5, BP6 | 80, 120 |
| H3 | 300 - 450 | BP2, BP3, BP4 | 45, 50, 58, 108 |
| H4 | 450 - 720 | BP1, BP2, BP3 | 50, 58, 108 |

EMSA revealed that pooled fractions H1/2, composed of the fractions eluted from the heparin affinity column over the salt range from 100 - 300 mM NaCl, contained at least three complexes, none of which showed up very well in the crosslink assay.

Pooled fractions H3, composed of the fractions eluted from the heparin affinity column over the salt range from 300 - 450 mM NaCl. On crosslink assay H3 contained two predominant proteins with MW of ca. 50 kDa and 58 kDa and lesser amounts of a 45 kDa protein and a 108 kDa protein.

Pooled fractions H4, composed of the fractions eluted from the heparin affinity column over the salt range from 450 - 720 mM NaCl. On crosslink analysis H4 contained three proteins with MW of ca. 50 kDa, 58 kDa, and 108 kDa.

*B. Ion exchange chromatography with UNO-Q and UNO-S columns.* After the heparin chromatography, pooled fraction H4 was desalted in buffer QA [50 mM NaCl (pH 7.5), 1 mM EDTA] on a PD10 column and then loaded on a UNO-Q anion exchange column (Bio-Rad). With the flow rate set at 1 ml/min, bound proteins were eluted with an increasing salt gradient from 0 to 1000 mM NaCl. Three anion exchange fractions (A1, A2, A3) corresponded to fractions collected from 130 - 260 mM, 260 - 400 mM, and 400 - 530 mM NaCl. Similarly, pooled fraction H1/2 was desalted, loaded onto a UNO-Q anion exchange column, and eluted.

Heparin fraction H3 was desalted in buffer QA, loaded on a UNO-S cation exchange column (Bio-Rad), and, with the flow rate set at 1 ml/min, eluted over an increasing salt gradient from 0 to 1000 mM NaCl. All bound proteins were eluted over the range 230 - 400 mM NaCl and were collected in a single cation exchange fraction (C 1). These anion and cation exchange fractions were stored at -80°C prior to analysis by EMSA and crosslink assays with ODN #5004.

*Results*. This second separation step resolved the 50 kDa, 58 kDa, and 108 kDa proteins present in H4 from one another. Results are shown in **FIG. 4** (EMSA in panel A; crosslink in panel B). The early fraction (A1) contained two proteins, BP2 and BP3, with MW of 50 kDa and 58 kDa. The middle fraction (A2) contained the 50 kDa protein together with the 108 kDa protein. The late fraction (A3) contained the 108 kDa protein, BP 1. The load fraction (lane L) showed the 50 kDa, 58 kDa, and 108 kDa proteins in the crosslink assay.

The 50 kDa and 45 kDa immunostimulatory DNA binding proteins BP3 and BP4 present in H3 were resolved using cation exchange rather than anion exchange. Results are shown in **FIG. 8** (EMSA in panel A and crosslink in panel B). CpG ODN #5004 was used with the following fractions: lane 1, H3 load fraction; lane 2, unbound fraction; lanes 4 - 10, fractions 11, 13, 15, 17, 19, 21, and 23. The C1 fraction contained BP3 and BP4.

*C. Affinity purification with immunostimulatory ODN-coated sepharose columns.* A version of the phosphodiester CpG ODN #2059 with an additional amino group at the 5' end (SEQ ID NO:10) was conjugated to 1 ml NHS-activated Sepharose HiTrap columns (Pharmacia) according to the manufacturer's instructions.

To purify immunostimulatory ODN-binding proteins, pooled fractions from the ion exchange column chromatography containing the same proteins were combined and their buffer changed to glycerol- and detergent-free DNA binding buffer on PD 10 columns (Pharmacia). This protein solution was then applied on the 1 ml ODN-sepharose column at a flow rate of 100 µl/min. After application of the sample, the flow rate was increased to 200 µl/min. Loaded columns were then washed with 1 ml of DNA binding buffer supplemented with an additional 100 mM NaCl and 20 µg/ml poly(dI-dC)·poly(dI-dC), followed by a second wash with 1 ml of DNA binding buffer supplemented with an additional 200 mM NaCl. Stepwise elutions were performed with 1 ml of 0.5 M KCl in DNA binding buffer, 1 ml of 1 M KCl in DNA binding buffer, and 1 ml of 1 M KSCN in DNA binding buffer. Eluted fractions were stored at -80°C until tested in EMSA, crosslink, or silver stain assays.

*Results.* Results of EMSA and crosslink assays of proteins isolated from fraction A 1 with labeled CpG ODN #5004 are shown in panels A and B of **FIG. 5.** In each panel the left lane is the load fraction, the middle lane is the 0.5 M KCl eluate, and the right lane is the 1 M K KCl eluate. A single protein, BP2, with an apparent MW of 58 kDa eluted from anion exchange fraction A1 at 0.5 M KCl, and a weaker band at 50 kDa corresponding to BP3 eluted at 1M KCl. These two bands are consistent with the crosslink results from fraction A 1 discussed above. As shown in **FIG. 7**, no other bands were evident on silver stain (laned 3 and 6 (0.5 M KCl) and lane 4 (1M KCl)).

Results of EMSA and crosslink assays of proteins isolated from fraction A3 with labeled CpG ODN #5004 are shown in panels A and B of **FIG. 6.** In each panel the left lane is the load fraction and the right lane is the 0.5 M K KCl eluate. As shown in **FIG. 6**, a single protein, BP1, with a MW of 108 kDa eluted from fraction A3 at 0.5 M KCl. This very dominant band is consistent with the crosslink results from fraction A3 discussed above. Although the EMSA showed only one ODN binding band in the A3 fraction, several weak protein bands at lower MW were visible in the silver stain (lane 5, **FIG. 7**). The lower MW bands present with the very large amount of the 108 kDa protein were found by MS analysis to represent degradation products of the main protein.

Results of EMSA and crosslink assays of proteins isolated from fraction C 1 with labeled CpG ODN #5004 are shown in panels A and B of **FIG. 9.** In each panel lane 1 is the load fraction; lanes 3-5, the 0.5 M KCl eluate fractions 31-33; lanes 6-8, the 1 M KCl eluate fractions 36-38; and lanes 9 and 10, the 1 M KSCN eluate fractions 42 and 43. As shown in **FIG. 9,** a single protein, BP4, with a MW of 45 kDa eluted from fraction C1 at 0.5 M KCl. A second, more prominent protein, BP3, with a MW of 50 kDa eluted at 1 M KCl. Silver stain analysis is shown in **FIG. 10.** Loadings in **FIG. 10** are as follows: lane 1, C1 load; lane 2, A1 elution with 500 mM KCl, containing BP2; lane 3, A1 elution with 1 M KCl, containing BP3; lane 4, C1 elution with 500 mM KCl, containing BP4; lane 5, C1 elution with 1 M KCl, containing BP3; and lane 6, C1 elution with 1 M KSCN, containing a minor amount of BP3.

## EXAMPLE 3

### Sequence Specificity of Isolated Immunostimulatory DNA Binding Proteins

Individual immunostimulatory DNA binding proteins BP1 - BP5 were isolated according to the methods described in Example 2. Cold competition EMSA using isolated BP1, BP2, BP3, and BP4 were performed in a first experiment with 10 ng of digoxigenin-labeled phosphodiester CpG ODN #5004 and varying amounts of unlabeled phosphodiester ODN selected from: CpG ODN #1619, methylated CpG ODN #1765, and non-CpG ODN #2049. In a similar experiment cold competition EMSA using isolated BP5 were performed with 10 ng of digoxigenin-labeled phosphodiester CpG ODN #5004 and varying amounts of unlabeled phosphodiester ODN selected from: CpG ODN #1619, methylated CpG ODN #1765, and non-CpG ODN #2049. Similarly, cold competition EMSA using isolated BP1, BP2, and BP3 were performed in a third experiment with 10 ng of digoxigenin-labeled CpG ODN #5004 and varying amounts of unlabeled ODN selected from: CpG ODN #2059, poly T ODN #5021, and poly C ODN #5031.

*Results*. Individual isolated immunostimulatory DNA binding proteins exhibited individual patterns of sequence specificity as measured by the competition EMSA method. The results of the first experiment are shown in **FIG. 11.** Lanes are as follows in all panels: lane 1 is the indicated binding protein with 10 ng of labeled CpG ODN #5004 alone. In lanes 2-4 CpG ODN #1619 was added as cold competitor in amounts of 300 ng, 30 ng, and 3 ng, respectively. In lanes 5-7 methylated CpG ODN #1765 was added as cold competitor in amounts of 300 ng, 30 ng, and 3 ng, respectively. In lanes 8-10 non-CpG ODN #2049 was added as cold competitor in amounts of 300 ng, 30 ng, and 3 ng, respectively. CpG ODN #1619 and non-CpG ODN #2049 differ only by the substitution of a GpC dinucleotide for a CpG dinucleotide.

Binding of CpG ODN to BP1 was highly competitive with other CpG ODN, moderately competitive with methylated CpG, and less competitive with non-CpG ODN. CpG and methylated CpG ODN were weak competitors for BP2, whereas non-CpG ODN appeared not to compete at all. Both CpG and methylated CpG ODN competed very effectively for BP3, while non-CpG ODN competed poorly. In contrast, binding of CpG ODN to BP4 was poorly and equally competitive with other CpG ODN, methylated CpG, and non-CpG ODN alike.

Results of the second experiment are shown in **FIG. 12.** Lanes in this figure correspond to lanes in **FIG. 11.** Competition was moderate with CpG ODN, weaker with methylated CpG, and weaker still with non-CpG ODN. This pattern was most similar to that observed for BP2 in the first experiment.

In the third experiment BP1 did not show specificity for poly T. BP2 and BP3 showed very strong competition by poly T, strong competition by unlabeled CpG ODN, and weak competition by poly C.

## EXAMPLE 4

### Backbone Specificity of Isolated Immunostimulatory DNA Binding Proteins

Crosslink assays shown in **FIG. 2** suggested that at least certain immunostimulatory DNA binding proteins might have a backbone preference. Crosslink assays were performed with BP6 isolated from fraction 17 of the H1 anion exchange of Example 2, and 5'-digoxigenin-labeled pairs of phosphodiester ODN (O-ODN) and phosphorothioate ODN (S-ODN). ODN pairs were CpG O-ODN #5004 and corresponding S-ODN #5007; poly T O-ODN #5017 and corresponding S-ODN #5018; poly C O-ODN #5009 and corresponding S-ODN #5030. Also included were 5'-digoxigenin-labeled non-CpG O-ODN #5016 and 3'-digoxigenin-labeled CpG O-ODN #5027.

*Results.* BP6 demonstrated a preference for S-ODN. Results are shown in **FIG. 13**. Lanes in **FIG. 13** are as follows: lane 1, S-ODN #5007; lane 2, O-ODN #5004; lane 3, O-ODN #5027; lane 4, O-ODN #5016; lane 5, O-ODN #5017; lane 6, S-ODN #5018; lane 7, O-ODN #5009; and lane 8, S-ODN #5030. Only lanes with S-ODN had visible crosslink products.

## EXAMPLE 5

### Identification of Isolated Immunostimulatory DNA Binding Proteins

*Identification of Binding Proteins 1 - 4.* Eluates A1, A3, and C 1 from ion exchange chromatography were run on similarly designated ODN-coated affinity columns (A1, A3, and Cl) as described in Section C of Example 2. Eluates from the A1, A3, and C1 ODN-coated affinity columns were desalted and concentrated with Centricon spin columns. Samples of the concentrated eluates were run on denaturing SDS-PAGE and protein bands were visualized by Coomassie staining. Individual bands were cut out of the gel, subjected to ingel sequence-specific cleavage with porcine trypsin, extracted and purified using a $C_{18}$ cartridge for analysis by mass spectroscopy. Proteins present in the gel at levels of 0.5 pmole or more can be analyzed by matrix-assisted laser desorption/ionization (MALDI)-MS analysis to yield a mass fingerprint of the protein fragment products that is compared to a database of predicted fragments derived from fully sequenced proteins. If the protein is not present in databases with its full amino acid sequence or full coding region, if the starting amount is less than 0.5 pmole, or if the protein is part of a mixture, then the identification method of choice is electrospray ionization tandem mass spectroscopy (ESI-MS). Analysis of the different eluates by MALDI-TOF-MS and ESI-MS was performed at M-Scan Ltd., (Ascot, GB).

Based on the MS findings, a Western blot for nucleolin was performed with various cytosolic extracts and fractions. SDS-PAGE was performed as previously described. No ODN was used. The gel was electro-blotted on a Biodyne Plus membrane (Pierce), UV-crosslinked, and blocked with blocking solution (Roche). For detection, a monoclonal mouse anti-human nucleolin antibody (C-23; sc-8031, Santa Cruz Biotech) was used 1:1000 as the first antibody and and a peroxidase-conjugate sheep anti-mouse IgG (#A7282, Sigma) 1:10,000 as the second antibody. Detection was performed with a chemiluminecence detection kit according to directions provided by the supplier (Roche). An x-ray film (Kodak) was exposed in the dark for 1-10 min.

*Results.* BP1 present in the 0.5 M KCl eluate of the A3 affinity column migrated at a MW of 110 kDa on SDS-PAGE. By delayed extraction (DE) MALDI-TOF-MS and ESI-MS it was identified as nucleolin, SwissProt accession no. P19338. Nucleolin contains 4 RBDs and one RGG domain.

BP2 present in the 0.5 M KCl eluate derived from the A1 affinity column migrated at a MW of 58 kDa on SDS-PAGE. By DE-MALDI-TOF-MS and ESI-MS it was identified as lupus La protein, SwissProt accession number P05455, equivalent to Sjögren syndrome Type B antigen La/SS-B protein. This protein is thought to be an RNA transcription termination factor, and it binds to the 3' termini of virtually all nascent RNA polymerase III transcripts. Lupus La protein contains a single RBD.

BP3 present in the 1 M KCl eluate derived from the C1 affinity column migrated at a MW of 50 kDa on SDS-PAGE. By MALDI-MS and ESI-MS it was identified as an isoform of hnRNP D, consistent with both SwissProt accession number Q14100 and Q12771. Isoforms of hnRNP D have a calculated pI near 8.8 and migrate on SDS-polyacrylamide gel electrophoresis (SDS-PAGE) at higher apparent molecular mass than predicted. Kajita Y et al. (1995) *J Biol Chem* 270:22167-22175. AUF 1 and hnRNP D each contain two RBDs and three RGG motifs.

BP4 present in the 0.5 M KCl eluate derived from the C1 affinity column migrated at a MW of 45 kDa on SDS-PAGE. By MALDI-MS and ESI-MS it was identified as hnRNP A1, SwissProt accession number P09651. HnRNP A1 also contains two RBDs and one RGG motif.

Results of the Western blotting for nucleolin are shown in **FIG. 14.** In the left panel the lanes represent cytosols from the following human cell types: N, NK cells, J, Jurkat T cells; H, HeLa cells; and R, Ramos B cells. Nucleolin staining was present in all cell types but in cell-type specific patterns. In the right panel the lanes correspond to Ramos cytosol, heparin affinity fractions H1 - H4, and isolated BP1. BP1 clearly stained very heavily, and displayed a predominant band at about 112 kDa. This band and another at 108 kDa stained heavly in the H4 fraction, while a minor amount of the 112 kDa band and none of the 108 kDa band were observed in H3. Discrete bands at lower MW, probably representing degradation products of intact nucleolin, were also present in fractions H3 and H4. No staining whatsoever was observed in the H1 or H2 fractions.

Taking into account that ssDNA binding protein nucleolin, together with hnRNP D, forms the B-cell proliferation-inducing transcription factor LR1, and that hnRNPs generally form heterocomplexes in the nucleus, it is expected that all four identified proteins may participate, possibly in various stoichiometries, in forming one or more sequence-specific ssDNA (ODN) binding complexes. Binding specificity is expected to be dependent on and maintained by individual components in a given complex. Furthermore, since nucleolin is described to be expressed at the cell surface and to shuttle between the outside of the cell and the nucleolus, nucleolin, complexed together with particular RNA binding proteins (e.g., hnRNP A1, hnRNP D, AUF1, or lupus La protein), is expected not only to mediate CpG-specific binding to ODN but also to trigger specific transcriptional events within the nucleus of the cell.

The proposed three sets of different MW immunostimulatory DNA binding proteins apparently exhibit different affinities for phosphorothioate or phosphodiester ODN when analyzed independently in crosslink assays. These differences observed *in vitro* are expected to be representative of the *in vivo* affinity for these ODN and suggest that binding proteins acting together as a complex might effect such binding selectivity, through the combined contributions from individual binding proteins' distinct binding affinities.

The 108 kDa protein we identified as nucleolin shows strong affinity for phosphorothioate and phosphodiester ODN. This might be crucial for nuceolin acting as surface receptor for phosphorothioate ODN. In contrast, the proteins in the lower MW range do not show strong affinity to phosphorothioate ODN. Instead, the 58 kDa and 50 kDa proteins exhibit a clear sequence specificity for immunostimulatory ODN and a particular high affinity for poly T ODN. Complexes containing more than one of these proteins may therefore exhibit a diverse pattern of sequence specificity in ssDNA/ODN binding. Nucleolin may function as a master protein in these complexes, recruiting one or all of the other proteins like hnRNP A1, hnRNP D, AUF1, or lupus La protein (and other, non-ODN-binding proteins too) to fulfill specific functions in ODN recognition.

It is expected that *the in vivo* function of these complexes begins with the high affinity binding of a broad panel of ODN by nucleolin, at the cell surface and/or in the cytoplasm, and, together with other cytosolic RNA binding proteins, e.g., various hnRNPs and lupus La protein, form sequence-specific complexes. Eventually ODN with certain sequences are passed on to high affinity proteins. Relocation of at least one component of the complex to the nucleus leads to triggering of transcriptional events. Alternatively immunostimulatory DNA binding proteins could be retained in the cytosol, blocking pre-mRNA processing, transport of mRNA out of the nucleus, and/or translation. immunostimulatory DNA binding proteins retained in the cytosol could also be prevented from or induced to bind to unstable mRNA.

## Claims

1. A screening method for identifying candidate ligands which bind to an immunostimulatory DNA binding protein and act as an immunostimulatory DNA functional modifier, comprising:

   contacting an immunostimulatory DNA binding protein with a sample containing at least one candidate ligand of said immunostimulatory DNA binding protein, wherein the immunostimulatory DNA binding protein is an RNA binding protein,
   determining if the at least one candidate ligand of immunostimulatory DNA binding protein binds to the immunostimulatory DNA binding protein, and
   determining if the at least one candidate ligand of immunostimulatory DNA binding protein that binds the immunostimulatory DNA binding protein belongs to a group of immunostimulatory DNA functional modifiers consisting of:

      immunostimulatory DNA binding inhibitors, immunostimulatory DNA mimics, immunostimulatory DNA agonists, and immunostimulatory DNA antagonists.

2. A method as claimed in claim 1, wherein binding of candidate ligand to the immunostimulatory DNA binding protein is detected using a method selected from the group consisting of: electrophoretic mobility shift assay (EMSA), enzyme-linked immunosorbent assay (ELISA), surface plasmon resonance (BIA), fluorimetry, bioluminescence, refractometry, autoradiography, autoradiometry, polymerase chain reaction (PCR), and scintillation detection.

3. A method as claimed in claim 1, wherein the step of determining if the at least one candidate ligand of immunostimulatory DNA binding protein that binds the immunostimulatory DNA binding protein belongs to a group of immunostimulatory DNA functional modifiers comprises contacting the candidate ligand that binds to the immunostimulatory DNA binding protein with an immune cell, and measuring a response of the immune cell.

4. A method as claimed in claim 3, further comprising contacting a reference immunostimulatory ODN with the immune cell.

5. A screening method for identifying an immunostimulatory DNA binding competitor, comprising:

   contacting an immunostimulatory DNA binding protein with a sample containing at least one candidate immunostimulatory DNA competitor compound and a reference immunostimulatory ODN, wherein the immunostimulatory DNA binding protein is an RNA binding protein, and
   determining a first amount of reference immunostimulatory ODN bound to the immunostimulatory DNA binding protein in the presence of the sample relative to a second amount of reference immunostimulatory ODN bound to the immunostimulatory binding protein in the absence of an immunostimulatory DNA competitor compound, wherein the sample includes at least one immunostimulatory DNA competitor compound when the first amount is less than the second amount.

6. A method as claimed in claim 5, wherein the sample and the reference immunostimulatory ODN are contacted with the immunostimulatory DNA binding protein simultaneously or sequentially.

7. A method as claimed in claim 5, wherein the amount of bound reference immunostimulatory ODN is detected using a method selected from the group consisting of: EMSA, ELISA, BIA, fluorimetry, bioluminescence, refractometry, autoradiography, autoradiometry, PCR, and scintillation detection.

8. The method as claimed in claim 1 or 5, wherein the at least one candidate ligand of claim 1, or the at least one candidate immunostimulatory DNA competitor compound of claim 5, is an isolated molecule selected from the group consisting of:

   polynucleotides, peptide nucleic acids, polypeptides, carbohydrates, lipids, hormones, small organic molecules, small inorganic molecules, variants of a reference immunostimulatory ODN molecule incorporating at least one substitution of a phosphorothioate-type bond for a phosphodiester bond, variants of a reference immunostimulatory ODN molecule incorporating at least one substitution of a phosphodiester bond for a phosphorothioate-type bond, variants of a reference immunostimulatory ODN molecule incorporating at least one substitution of one phosphorothioate-type bond for a different phosphorothioate-type bond, and

variants of a reference immunostimulatory ODN molecule incorporating at least one substitution of one nucleotide with a different nucleotide.

**9.** A method as claimed in claim 5, further comprising the steps of contacting the immunostimulatory DNA competitor compound with an immune cell, and measuring a response of the immune cell to determine whether the immunostimulatory DNA competitor compound is an immunostimulatory DNA functional mimic.

**10.** A method as claimed in claim 5, further comprising the steps of contacting the immunostimulatory DNA competitor compound and a reference immunostimulatory ODN with an immune cell, and measuring a response of the immune cell to determine whether the immunostimulatory DNA competitor compound is an immunostimulatory DNA inhibitor.

**11.** A method as claimed in claim 4 or 10, wherein the candidate ligand and the reference immunostimulatory ODN of claim 4, or the immunostimulatory DNA binding competitor compound and the reference immunostimulatory ODN of claim 10, are contacted with the immune cell simultaneously or sequentially.

**12.** A method as claimed in any one of claims 3, 9 or 10, wherein the response of the immune cell is measured by:-

(a) determining an amount of at least one cytokine secreted by the immune cell;
(b) determining an amount of antibody secreted by the immune cell;
(c) determining an isotype of antibody secreted by the immune cell;
(d) determining expression of at least one cell surface molecule on the immune cell;
(e) determining activation of at least one cellular kinase of the immune cell; or
(f) determining a change in redox potential of the immune cell.

**13.** A method for optimizing an immunostimulatory ODN for immune stimulation, comprising:

providing an immunostimulatory DNA binding protein, a reference immunostimulatory ODN that binds to the immunostimulatory DNA binding protein, and at least one candidate optimized immunostimulatory ODN that differs from the reference immunostimulatory ODN by at least one nucleotide or at least one intemucleotide linkage, wherein the immunostimulatory DNA binding protein is an RNA binding protein,
contacting the immunostimulatory DNA binding protein with a sample containing at least one candidate optimized immunostimulatory ODN and the reference immunostimulatory ODN,
determining which, if any, candidate optimized immunostimulatory ODN binds the immunostimulatory DNA binding protein to a greater extent than the reference immunostimulatory ODN,
redesignating a candidate optimized immunostimulatory ODN which binds the immunostimulatory DNA binding protein to a greater extent than the reference immunostimulatory ODN as the reference immunostimulatory DNA, and
repeating the steps of providing, contacting, determining, and redesignating until no candidate optimized immunostimulatory ODN binds the immunostimulatory DNA binding protein better than the reference immunostimulatory ODN against which it is compared.

**14.** A method for identifying cellular targets of an immunostimulatory ODN, comprising:

screening a panel of candidate target molecules with a complex formed between an immunostimulatory ODN and an immunostitnulatory DNA binding protein, wherein the immunostimulatory DNA binding protein is an RNA binding protein,
selecting the candidate target molecules that bind to the complex, and
determining the sequence of the bound candidate target molecules to identify the cellular targets of the immunostimulatory ODN.

**15.** A method as claimed in claim 14, wherein the panel of candidate target molecules is:-

(a) cDNA library;
(b) a nucleic acid microchip; or
(c) a polypeptide library.

**16.** A method as claimed in claim 14, wherein the complex is composed of an immunostimulatory ODN tethered to an immunostimulatory DNA binding protein.

**17.** An isolated immunostimulatory ODN-immunostimulatory DNA binding protein complex, wherein the immunostimulatory DNA binding protein is an RNA binding protein.

**18.** A complex as claimed in claim 17, wherein the immunostimulatory ODN is:-

(a) tethered to the immunostimulatory DNA binding protein; or
(b) associated with the immunostimulatory DNA binding protein.

**19.** A method as claimed in anyone of claims 4, 5, 10 or a complex as claimed in claim 17, wherein the reference immunostimulatory ODN of claim 4 or 10, the at least one candidate immunostimulatory DNA competitor compound of claim 5, or the immunostimulatory ODN of claim 18 is:

(a) a phosphorothioate ODN;
(b) a CpG ODN;
(c) a T-rich ODN, preferably a poly T ODN; or
(d) a poly G ODN.

**20.** A method as claimed in any one of claims 1, 5 or 14, or a complex as claimed in claim 17, wherein the candidate ligand of claim 1, reference immunostitnulatory ODN of claim 5, immunostimulatory ODN of claim 14 or 17, immunostimulatory DNA binding protein of claim 14 or 17, or the candidate target molecule of claim 14 is labelled with a marker selected from the group consisting of: digoxigenin, biotin, an isotope, a fluorophore, and an enzyme.

**21.** A method as claimed in claim 5 or 14, or a complex as claimed in claim 17, wherein the reference immunostimulatory ODN of claim 5, or the immunostimulatory ODN of claim 14 or 17 is selected from the group consisting of: SEQ ID NO:1 (#2059), SEQ ID NO:2 (#2080), SEQ ID NO:3 (#1619), SEQ ID NO:11 (#5007), and SEQ ID NO:12 (#5004).

**22.** A kit for identifying cellular targets of an immunostimulatory ODN, comprising:

an immunostimulatory ODN in a container,
an immunostimulatory DNA binding protein in a container, wherein the immunostimulatory DNA binding protein is an RNA binding protein, and
instructions for preparing an immunostimulatory ODN-iromunostimulatory DNA binding protein complex and for using the complex to screen a panel of candidate target molecules to identify cellular targets of an immunostimulatory ODN.

**23.** A method as claimed in any of claims 1, 5, 13 or 14, a complex as claimed in claim 17 or a kit as claimed in claim 22, wherein the immunostimulatory DNA binding protein is:-

(a) a single-stranded DNA binding protein;
(b) a RNA binding protein having at least one motif selected from the group consisting of RBD, RGG domain, KH motif and ARM;
(c) a RNA binding protein selected from the group consisting of hnRNPs, nucleolin, and lupus La protein;
(d) a RNA binding protein selected from the group consisting of nucleolin, hnRNP D, AUF1, hnRNP A1, and lupus La protein; or
(e) is part of a complex comprising a protein selected from the group consisting of nucleolin, lupus La protein, hn RNP D, AUF1, and hnRNP A1.

**24.** A method as claimed in claim 1 or 5, wherein the candidate ligand of claim 1, or the candidate immunostimulatory DNA competitor compound of claim 5, is part of a combinatorial library of compounds.

**25.** A method as claimed in claim 1 or 5, wherein the immunostimulatory DNA binding protein is attached to a substrate selected from the group consisting of; a plastic multiwell plate, a bead, a resin, a filter, a glass slide, a BIAcore chip and a silicon chip.

## Patentansprüche

1. Screening-Verfahren zur Identifizierung von in Frage kommenden Liganden, die an ein immunstimulatorische DNA bindendes Protein binden und als Funktions-Modifikator immunstimulatorischer DNA agieren, umfassend das:

   In-Kontakt-bringen eines immunstimulatorische DNA bindenden Proteins mit einer Probe, die mindestens einen in Frage kommenden Liganden für ein immunstimulatorische DNA bindendes Protein enthält, wobei das immunstimulatorische DNA bindende Protein ein RNA-bindendes Protein ist,

   Feststellen, ob der mindestens eine in Frage kommende Ligand des immunstimulatorische DNA bindenden Proteins das immunstimulatorische DNA bindende Protein bindet, und

   Feststellen, ob der mindestens eine in Frage kommende Ligand des immunstimulatorische DNA bindenden Proteins, der an das immunstimulatorische DNA bindende Protein bindet, zur Gruppe der Funktions-Modifikatoren immunstimulatorischer DNA gehört, bestehend aus: Inhibitoren der Bindung immunstimulatorischer DNA, Imitatoren immunstimulatorischer DNA, Agonisten immunstimulatorischer DNA und Antagonisten immunstimulatorischer DNA.

2. Verfahren nach Anspruch 1, wobei das Binden des in Frage kommenden Liganden an das immunstimulatorische DNA bindende Protein unter Verwendung einer Methode festgestellt wird, die ausgewählt ist aus der Gruppe bestehend aus:

   Elektromobilitätsshift-Assay (EMSA), enzymgekoppelter Immunassay (ELISA), Oberflächen-Plasmon-Resonanz (BIA), Fluorimetrie, Biolumineszenz, Refraktometrie, Autoradiographie, Autoradiometrie, Polymerasekettenreaktion (PCR) und Szintillationsdetektion.

3. Verfahren nach Anspruch 1, wobei der Schritt des Feststellens, ob der wenigstens eine in Frage kommende Ligand des immunstimulatorische DNA bindenden Proteins, der an das immunstimulatorische DNA bindende Protein bindet, zur Gruppe der Funktions-Modifikatoren immunstimulatorischer DNA gehört, das In-Kontakt-bringen des in Frage kommenden Liganden, der an das immunstimulatorische DNA bindende Protein bindet, mit einer Immunzelle und Messen der Antwort der Immunzelle umfaßt.

4. Verfahren nach Anspruch 3, weiter umfassend das In-Kontakt-bringen eines immunstimulatorischen Referenz-ODNs mit einer Immunzelle.

5. Screening-Verfahren zur Identifizierung eines immunstimulatorische DNA bindenden Kompetitoren, umfassend:

   In-Kontakt-bringen eines immunstimulatorische DNA bindenden Proteins mit einer Probe, die mindestens. eine in Frage kommende Kompetitor-Verbindung und ein immunstimulatorisches Referenz-ODN enthält, wobei das immunstimulatorische DNA bindende Protein ein RNA-bindendes Protein ist, und

   Feststellen einer ersten Menge von in Gegenwart der Probe an das immunstimulatorische DNA bindende Protein gebundenem immunstimulatorischen Referenz-ODN im Vergleich zu einer zweiten Menge von in Abwesenheit einer Kompetitor-Verbindung zu der immunstimulatorischen DNA an das immunstimulatorische DNA bindende Protein gebundenem immunstimulatorischen Referenz-ODN, wobei die Probe mindestens eine in Frage kommende Kompetitor-Verbindung beinhaltet, wenn die ersten Menge geringer ist als die zweite Menge.

6. Verfahren nach Anspruch 5, wobei die Probe und das immunstimulatorische Referenz-ODN mit dem immunstimulatorische DNA bindenden Protein gleichzeitig oder nacheinander in Kontakt gebracht werden.

7. Verfahren nach Anspruch 5, wobei die Menge von gebundenem immunstimulatorischen Referenz-ODN unter Verwendung einer Methode bestimmt wird, die ausgewählt ist aus der Gruppe bestehend aus: EMSA, ELISA, BIA, Fluorimetrie, Biolumineszenz, Refraktometrie, Autoradiographie, Autoradiometrie, PCR und Szintillationsdetektion.

8. Verfahren nach Anspruch 1 oder 5, wobei der mindestens eine in Frage kommende Ligand gemäß Anspruch 1 oder die mindestens eine in Frage kommende Kompetitor-Verbindung zur immunstimulatorischen DNA gemäß Anspruch 5 ein isoliertes Molekül ist, das ausgewählt ist aus der Gruppe bestehend aus: Polynukleotiden, Peptidnukleinsäuren, Polypeptiden, Kohlenhydraten, Lipiden, Hormonen, kleinen organischen Molekülen, kleinen anorganischen Molekülen, Varianten eines mindestens eine Substitution einer Bindung vom Phosphorthioat-Typ durch eine Phosphodiesterbindung enthaltenden immunstimulatorischen Referenz-ODN-Moleküls, Varianten eines mindestens eine Substitution einer Phosphodiesterbindung durch eine Bindung vom Phosphorthioat-Typ enthaltenden immunstimulatorischen Referenz-ODN-Moleküls, Varianten eines mindestens eine Substitution einer Bindung vom Phosphorthioat-Typ durch eine andere Bindung vom Phosphorthioat-Typ enthaltenden immunstimulatorischen Referenz-ODN-Moleküls, und Varianten eines mindestens eine Substitution eines Nukleotids durch ein anderes Nukleotid enthaltenden immunstimulatorischen Referenz-ODN-Moleküls.

**9.** Verfahren nach Anspruch 5, weiter umfassend die Schritte des In-Kontakt-bringens der Kompetitor-Verbindung zur immunstimulatorischen DNA mit einer Immunzelle und Messens der Antwort der Immunzelle zur Feststellung, ob die Kompetitor-Verbindung zur. immunstimulatorischen DNA ein Funktions-Imitator einer immunstimulatorischen DNA ist.

**10.** Verfahren nach Anspruch 5, weiter umfassend die Schritte des In-Kontakt-bringens der Kompetitor-Verbindung zur immunstimulatorischen DNA und eines immunstimulatorischen Referenz-ODNs mit einer Immunzelle und Messens der Antwort der Immunzelle zur Feststellung, ob die Kompetitor-Verbindung zur immunstimulatorischen DNA ein Inhibitor immunstimulatorischer DNA ist.

**11.** Verfahren nach Anspruch 4 oder 10, wobei der in Frage kommende Ligand und das immunstimulatorische Referenz-ODN gemäß Anspruch 4 oder die Kompetitor-Verbindung zur immunstimulatorischen DNA und das immunstimulatorische Referenz-ODN gemäß Anspruch 10 gleichzeitig oder nacheinander mit der Immunzelle in Kontakt gebracht werden.

**12.** Verfahren nach einem der Ansprüche 3, 9 oder 10, wobei die Antwort der Immunzelle gemessen wird durch:

(a) Feststellung einer Menge von mindestens einem durch die Immunzelle ausgeschiedenen Zytokin;
(b) Feststellung einer Menge eines durch die Immunzelle ausgeschiedenen Antikörpers;
(c) Feststellung eines Isotyp eines durch die Immunzelle ausgeschiedenen Antikörpers;
(d) Feststellung der Expression von mindestens einem Zelloberflächenmolekül auf der Immunzelle;
(e) Feststellung der Aktivierung von mindestens einer zellulären Kinase der Immunzelle; oder
(f) Feststellung einer Änderung des Redoxpotentials der Immunzelle.

**13.** Verfahren zur Optimierung eines immunstimulatorischen ODNs zur Immunstimulation, umfassend das:

Bereitstellen eines immunstimulatorische DNA bindenden Proteins, eines an das immunstimulatorische DNA bindende Protein bindenden immunstimulatorischen Referenz-ODNs und mindestens eines in Frage kommenden optimierten immunstimulatorischen ODNs, das sich von dem immunstimulatorischen Referenz-ODN durch mindestens ein Nukleotid oder mindestens eine Internukleotidverbindung unterscheidet, wobei das immunstimulatorische DNA bindende Protein ein RNA-bindendes Protein ist,

In-Kontakt-bringen des immunstimulatorische DNA bindenden Proteins mit einer Probe, die mindestens ein in Frage kommendes optimiertes immunstimulatorisches ODN und das immunstimulatorische Referenz-ODN enthält,

Feststellen, welches in Frage kommende optimierte immunstimulatorische ODN, sofern vorhanden, an das immunstimulatorische DNA bindende Protein in größerem Umfang als das immunstimulatorische Referenz-ODN bindet,

erneute Bestimmen eines in Frage kommenden optimierten immunstimulatorischen ODNs, das an das immunstimulatorische DNA bindende Protein in größerem Umfang als das immunstimulatorische Referenz-ODN als die immunstimulatorische Referenz-DNA bindet, und

Wiederholen der Schritte des Bereitstellens, In-Kontakt-bringens, Feststellens und erneuten Bestimmens, bis kein in Frage kommendes optimiertes immunstimulatorisches ODN das immunstimulatorische DNA bindende Protein besser als das immunstimulatorische Referenz-ODN, mit dem es verglichen wird, bindet.

**14.** Verfahren zur Identifizierung zellulärer Ziele eines immunstimulatorischen ODNs, umfassend das:

Screening einer Reihe von in Frage kommenden Zielmolekülen mit einem zwischen einem immunstimulatorischen ODN und einem immunstimulatorische DNA bindenden Protein gebildeten Komplex, wobei das immunstimulatorische DNA bindende Protein ein RNA-bindendes Protein ist,

Auswählen der in Frage kommenden, an den Komplex bindenden, Zielmoleküle, und

Feststellen der Sequenz der gebundenen in Frage kommenden Zielmoleküle zur Identifizierung der zellulären Ziele des immunstimulatorischen ODNs.

**15.** Verfahren nach Anspruch 14, wobei die Reihe von in Frage kommenden Zielmolekülen

(a) eine cDNA-Bibliothek;
(b) ein Nukleinsäure-Mikrochip; oder
(c) eine Polypeptid-Bibliothek

ist.

**16.** Verfahren nach Anspruch 14, wobei der Komplex zusammengesetzt ist aus einem immunstimulatorischen ODN, angebunden an ein immunstimulatorische DNA bindendes Protein.

**17.** Isolierter Immunstimulatorisches-ODN-immunstimulatorische-DNA-bindendes-Protein-Komplex, wobei das immunstimulatorische DNA bindende Protein ein RNA-bindendes Protein ist.

**18.** Komplex nach Anspruch 17, wobei das immunstimulatorische ODN

(a) an das immunstimulatorische DNA bindende Protein angebunden ist; oder
(b) mit dem immunstimulatorische DNA bindenden Protein assoziiert ist.

**19.** Verfahren nach einem der Ansprüche 4, 5, 10, oder ein Komplex nach Anspruch 17, wobei das immunstimulatorische Referenz-ODN gemäß Anspruch 4 oder 10, die mindestens eine in Frage kommende Kompetitor-Verbindung zur immunstimulatorischen DNA gemäß Anspruch 5 oder das immunstimulatorische ODN gemäß Anspruch 18

(a) ein Phosphorthioat-ODN;
(b) ein CpG-ODN;
(c) ein T-reiches ODN, vorzugsweise ein Poly-T-ODN; oder
(d) ein Poly-G-ODN

ist.

**20.** Verfahren nach einem der Ansprüche 1, 5 oder 14, oder ein Komplex nach Anspruch 17, wobei der in Frage kommende Ligand gemäß Anspruch 1, das immunstimulatorische Referenz-ODN gemäß Anspruch 5, das immunstimulatorische ODN gemäß Anspruch 14 oder 17, das immunstimulatorische DNA bindende Protein gemäß Anspruch 14 oder 17 oder das in Frage kommende Zielmolekül gemäß Anspruch 14 mit einem Marker markiert ist, der ausgewählt ist aus der Gruppe bestehend aus: die Digoxigenin, Biotin, einem Isotop, Fluorophor und einem Enzym.

**21.** Verfahren nach Anspruch 5 oder 14, oder Komplex nach Anspruch 17, wobei das immunstimulatorische Referenz-ODN gemäß Anspruch 5 oder das immunstimulatorische ODN gemäß Anspruch 14 oder 17 ausgewählt ist aus der Gruppe bestehend aus: SEQ ID NO: 1 (#2059), SEQ ID NO: 2 (#2080), SEQ ID NO: 3 (#1619), SEQ ID NO: 11 (#5007) und SEQ ID NO: 12 (#5004).

**22.** Kit zur Identifizierung zellulärer Ziele eines immunstimulatorischen ODNs, umfassend:

ein immunstimulatorisches ODN in einem Behälter,

ein immunstimulatorischen DNA bindendes Protein in einem Behälter, wobei das immunstimulatorische DNA bindende Protein ein RNA-bindendes Protein ist, und

Anweisungen zur Herstellung eines immunstimulatorisches-ODN--immunstimulatorische-DNA-bindendes-Protein-Komplexes und zur Verwendung des Komplexes zum Screening einer Reihe von in Frage kommenden Zielmolekülen zur Identifizierung zellulärer Ziele eines immunstimulatorischen ODNs

**23.** Verfahren nach einem der Ansprüche 1, 5, 13 oder 14, ein Komplex nach Anspruch 17 oder ein Kit nach Anspruch 22, wobei das immunstimulatorische DNA bindende Protein

(a) ein Einzelstrang-DNA bindendes Protein ist;
(b) ein RNA-bindendes Protein ist, das mindestens ein Motiv aufweist, das ausgewählt ist aus der Gruppe bestehend aus RBD, RGG-Domäne, KH-Motiv und ARM;
(c) ein RNA-bindendes Protein ist, das ausgewählt ist aus der Gruppe bestehend aus hnRNPs, Nukleolin und Lupus-La-Protein;
(d) ein RNA-bindendes Protein ist, das ausgewählt ist aus der Gruppe bestehend aus Nukleolin, hnRNP D, AUF1, hnRNP A1 und Lupus-La-Protein; oder
(e) Teil eines Komplexes ist, umfassend ein Protein, das ausgewählt ist aus der Gruppe bestehend aus Nukleolin, Lupus-La-Protein, hnRNP D, AUF1 und hnRNP A1.

**24.** Verfahren nach Anspruch 1 oder 5, wobei der in Frage kommende Ligand gemäß Anspruch 1, oder die in Frage kommende Kompetitor-Verbindung zu immunstimulatorischer DNA gemäß Anspruch 5 Teil einer kombinatorischen Bibliothek von Verbindungen ist.

**25.** Verfahren nach Anspruch 1 oder 5, wobei das immunstimulatorische DNA bindende Protein an ein Substrat angeheftet ist, das ausgewählt ist aus der Gruppe bestehend aus einer Kunststoff-Multiwellplatte, einer Perle, einem Harz, einem Filter, einem Glas-Objektträger, einem BIAcore-Chip und einem Silizium-Chip.

## Revendications

**1.** Procédé de sélection en vue de l'identification de ligands candidats qui se lient à une protéine de' liaison d'ADN immunostimulatrice et qui agissent en tant que modificateur fonctionnel d'ADN immunostimulateur, comprenant :

la mise en contact d'une protéine de liaison d'ADN immunostimulatrice avec un échantillon contenant au moins un ligand candidat de ladite protéine de liaison d'ADN immunostimulatrice, où la protéine de liaison d'ADN immunostimulatrice est une protéine de liaison d'ARN,
la détermination du fait de savoir si le au moins un ligand candidat de la protéine de liaison d'ADN immunostimulatrice se lie à la protéine de liaison d'ADN immunostimulatrice, et
la détermination du fait de savoir si le au moins un ligand candidat de la protéine de liaison d'ADN immunostimulatrice, qui lie la protéine de liaison d'ADN immunostimulatrice, appartient à un groupe de modificateurs fonctionnels d'ADN immunostimulateurs se composant : d'inhibiteurs de liaison d'ADN immunostimulateurs, d'agents mimétiques d'ADN immunostimulateurs, d'agonistes d'ADN immunostimulateurs et d'antagonistes d'ADN immunostimulateurs.

**2.** Procédé selon la revendication 1, dans lequel la liaison du ligand candidat à la protéine de liaison d'ADN immunostimulatrice est détectée par utilisation d'un procédé sélectionné parmi le groupe constitué : du test EMSA (essai de décalage de mobilité électrophorétique), du test ELISA, du test BIA (résonance plasmon de surface),de la fluorimétrie, de la bioluminescence, de la réfractométrie de l'autoradiographie, de l'autoradiométrie, de la réaction en chaîne de polymérase (PCR), et de la détection par scintillation.

**3.** Procédé selon la revendication 1, dans lequel l'étape de détermination du fait de savoir si le au moins un ligand candidat de la protéine de liaison d'ADN immunostimulatrice, qui lie la protéine de liaison d'ADN immunostimulatrice, appartient à un groupe de modificateurs fonctionnels d'ADN immunostimulateurs, comprend la mise en contact du ligand candidat, qui se lie à la protéine de liaison d'ADN immunostimulatrice, avec un cellule immunitaire et la mesure d'une réaction de la cellule immunitaire.

**4.** Procédé selon la revendication 3, comprenant en outre la mise en contact avec un ODN immunostimulateur de référence avec la cellule immunitaire.

5. Procédé de sélection en vue de l'identification d'un compétiteur de liaison d'ADN immunostimulateur, comprenant :

   la mise en contact d'une protéine de liaison d'ADN immunostimulatrice avec un échantillon contenant au moins un composé compétiteur d'ADN immunostimulateur candidat et un ODN immunostimulateur de référence, où la protéine de liaison d'ADN immunostimulatrice est une protéine de liaison d'ARN, et
   la détermination d'une première quantité d'ODN immunostimulateur de référence liée à la protéine de liaison d'ADN immunostimulatrice en présence de l'échantillon, par rapport à une deuxième quantité d'ODN immunostimulateur de référence liée à la protéine de liaison d'ADN immunostimulatrice en l'absence d'un composé compétiteur d'ADN immunostimulateur, dans lequel l'échantillon comprend au moins un composé compétiteur d'ADN immunostimulateur, lorsque la première quantité est inférieure à la deuxième quantité.

6. Procédé selon la revendication 5, dans lequel l'échantillon et l'ODN immunostimulateur de référence sont mis en contact avec la protéine de liaison d'ADN immunostimulatrice, simultanément ou séquentiellement.

7. Procédé selon la revendication 5, dans lequel la quantité d'ODN immunostimulateur de référence liée est détectée par utilisation d'un procédé sélectionné parmi le groupe constitué : des procédés EMSA, ELISA, BIA, fluorimétrie, bioluminescence, réfractométrie, autoradiographie, autoradiométrie, PCR, et détection par scintillation.

8. Procédé selon la revendication 1 ou la revendication 5, dans lequel le au moins un ligand candidat selon la revendication 1, ou le au moins un composé compétiteur d'ADN immunostimulateur candidat selon la revendication 5, est une molécule isolée, sélectionnée parmi le groupe constitué : de polynucléotides, d'acides nucléiques peptidiques, de polypeptides, de glucides, de lipides, d'hormones, de petites molécules organiques, de petites molécules inorganiques, de variantes d'une molécule d'ODN immunostimulateur de référence, incorporant au moins une substitution d'une liaison du type phosphorothioate pour une liaison de phosphodiester, des variantes d'une molécule d'ODN immunostimulateur de référence, incorporant au moins une substitution d'une liaison du type phosphodiester pour une liaison de type phosphorothioate, des variantes d'une molécule d'ODN immunostimulateur de référence, incorporant au moins une substitution d'une liaison du type phosphorothioate pour une liaison différente du type phosphorothioate, et des variantes d'une molécule d'ODN immunostimulateur de référence, incorporant au moins une substitution d'un nucléotide avec un nucléotide différent.

9. Procédé selon la revendication 5, comprenant en outre les étapes de mise en contact du composé compétiteur d'ADN immunostimulateur avec une cellule immunitaire, et de mesure d'une réaction de la cellule immunitaire pour déterminer si le composé compétiteur d'ADN immunostimulateur est un agent mimétique fonctionnel d'ADN immunostimulateur.

10. Procédé selon la revendication 5, comprenant en outre les étapes de mise en contact du composé compétiteur d'ADN immunostimulateur et d'un ODN immunostimulateur de référence avec une cellule immunitaire, et de mesure d'une réaction de la cellule immunitaire pour déterminer si le composé compétiteur d'ADN immunostimulateur est un agent inhibiteur d'ADN immunostimulateur.

11. Procédé selon la revendication 4 ou la revendication 10, dans lequel le ligand candidat et l'ODN immunostimulateur de référence selon la revendication 4, ou le composé compétiteur d'ADN immunostimulateur et l'ODN immunostimulateur de référence selon la revendication 10, sont mis en contact avec la cellule immunitaire, simultanément ou séquentiellement.

12. Procédé selon l'une quelconque des revendications 3, 9 ou 10, dans lequel la réaction de la cellule immunitaire est mesurée par :

   (a) la détermination d'une quantité d'au moins une cytokine sécrétée par la cellule immunitaire ;
   (b) la détermination d'une quantité d'anticorps secrétée par la cellule immunitaire ;
   (c) la détermination d'un isotype d'anticorps secrété par la cellule immunitaire ;
   (d) la détermination de l'expression d'au moins une molécule de surface de la cellule sur la cellule immunitaire ;
   (e) la détermination de l'activation d'au moins une kinase cellulaire de la cellule immunitaire ; ou
   (f) la détermination d'un changement en potentiel rédox de la cellule immunitaire.

13. Procédé d'optimisation d'un ODN immunostimulateur pour une stimulation immunitaire, comprenant :

la mise à disposition d'une protéine de liaison d'ADN immunostimulatrice, d'un ODN immunostimulateur de référence, qui se lie à la protéine de liaison d'ADN immunostimulatrice, and au moins un ODN immunostimulateur candidat optimisé qui diffère de l'ODN immunostimulateur de référence par au moins un nucléotide ou au moins une liaison internucléotide, où la protéine de liaison d'ADN immunostimulatrice est une protéine de liaison d'ARN,
la mise en contact de la protéine de liaison d'ADN immunostimulatrice avec un échantillon contenant au moins un ODN immunostimulateur candidat optimisé et l'ODN immunostimulateur de référence,
la détermination du fait de savoir, s'il y a lieu, si l'ODN immunostimulateur candidat optimisé lie la protéine de liaison d'ADN immunostimulatrice dans une plus large mesure que l'ODN immunostimulateur de référence,
la nouvelle conception d'un ODN immunostimulateur candidat optimisé qui lie la protéine de liaison d'ADN immunostimulatrice dans une plus large mesure que l'ODN immunostimulateur de référence, en tant qu'ADN immunostimulateur de référence, et
la répétition des étapes de mise à disposition, de mise en contact, de détermination et de nouvelle conception jusqu'à ce qu'aucun ODN immunostimulateur candidat optimisé ne lie la protéine de liaison d'ADN immunostimulatrice mieux que l'ODN immunostimulateur de référence auquel elle est comparée.

**14.** Procédé en vue de l'identification de cibles cellulaires d'un ODN immunostimulateur, comprenant :

la sélection d'une gamme de molécules-cible candidates avec un complexe formé entre un ODN immunostimulateur et une protéine de liaison d'ADN immunostimulatrice, où la protéine de liaison d'ADN immunostimulatrice est une protéine de liaison d'ARN,
la sélection des molécules-cible candidates qui se lient au complexe, et
la détermination de la séquence des molécules-cible candidates liées pour identifier les cibles cellulaires de l'ODN immunostimulateur.

**15.** Procédé selon la revendication 14, dans lequel la gamme de molécules-cible candidates est :

(a) une bibliothèque cADN ;
(b) une micro puce d'acides nucléiques ; ou
(c) une bibliothèque de polypeptides.

**16.** Procédé selon la revendication 14, dans lequel le complexe est composé d'un ODN immunostimulateur ancré sur une protéine de liaison d'ADN immunostimulatrice.

**17.** Complexe d'ODN immunostimulateur isolé - de protéine de liaison d'ADN immunostimulatrice, dans lequel la protéine de liaison d'ADN immunostimulatrice est une protéine de liaison d'ARN.

**18.** Complexe selon la revendication 17, dans lequel l'ODN immunostimulateur est :

(a) ancré sur la protéine de liaison d'ADN immunostimulatrice ; ou
(b) associé à la protéine de liaison d'ADN immunostimulatrice.

**19.** Procédé selon l'une quelconque des revendications 4, 5, 10 ou complexe selon la revendication 17, dans lequel l'ODN immunostimulateur de référence selon la revendication 4 ou 10, le au moins un composé compétiteur d'ADN immunostimulateur candidat selon la revendication 5 ou l'ODN immunostimulateur selon la revendication 18 est :

(a) un ODN de phosphorothioate ;
(b) un ODN CpG ;
(c) un ODN riche en T, de préférence un ODN poly T ; ou
(d) un ODN poly G.

**20.** Procédé selon l'une quelconque des revendications 1, 5 ou 14, ou complexe selon la revendication 17, dans lequel le ligand candidat selon la revendication 1, l'ODN immunostimulateur de référence selon la revendication 5, l'ODN immunostimulateur selon la revendication 14 ou la revendication 17, la protéine de liaison d'ADN immunostimulatrice selon la revendication 14 ou la revendication 17, ou la molécule-cible candidate selon la revendication 14 est étiqueté à l'aide d'un marqueur sélectionné parmi le groupe constitué : de la digoxigénine, de la biotine, d'un isotope, d'un fluorophore, et d'une enzyme.

**21.** Procédé selon la revendication 5 ou la revendication 14, ou complexe selon la revendication 17, dans lequel l'ODN immunostimulateur de référence selon la revendication 5 ou l'ODN immunostimulateur selon la revendication 14 ou 17, est sélectionné parmi le groupe constitué : des éléments SEQ ID NO:1 (#2059), SEQ ID NO:2 (#2080), SEQ ID NO:3 (#1619), SEQ ID NO:11 (#5007), et SEQ ID NO:12 (#5004).

**22.** Kit en vue de l'identification de cibles cellulaires d'un ODN immunostimulateur , comprenant :

un ODN immunostimulateur dans un récipient,
une protéine de liaison d'ADN immunostimulatrice dans un récipient, dans lequel la protéine de liaison d'ADN immunostimulatrice est une protéine de liaison d'ARN, et
d'instructions en vue de la préparation d'un complexe d'ODN immunostimulateur - d'une protéine de liaison d'ADN immunostimulatrice et en vue de l'utilisation du complexe pour sélectionner une gamme de molécules-cible candidates, afin d'identifier les cibles cellulaires d'un ODN immunostimulateur.

**23.** Procédé selon l'une quelconque des revendications 1, 5, 13 ou 14, ou complexe selon la revendication 17 ou kit selon la revendication 22, dans lequel la protéine de liaison d'ADN immunostimulatrice est :

(a) une protéine de liaison d'ADN monocaténaire ;
(b) une protéine de liaison d'ARN, ayant au moins un motif sélectionné parmi le groupe constitué de RBD, de domaine RGG, de motif KH et d'ARM ;
(c) une protéine de liaison d'ARN sélectionnée parmi le groupe constitué de hnRNPs, de nucléoline, et de protéine lupus La ;
(d) une protéine de liaisons d'ARN sélectionnée parmi le groupe constitué de nucléoline, de hnRNP D, de AUF1, de hnRNP A1, et de protéine lupus La ; ou
(e) ou fait partie d'un complexe comprenant une protéine sélectionnée parmi le groupe constitué de la nucléoline, de la protéine lupus La, de hnRNP D, de AUF1, et de hnRNP A1.

**24.** Procédé selon la revendication 1 ou la revendication 5, dans lequel le ligand candidat selon la revendication 1, ou le composé compétiteur d'ADN immunostimulateur candidat selon la revendication 5 fait partie d'une bibliothèque combinatoire de composés.

**25.** Procédé selon la revendication 1 ou 5, dans lequel la protéine de liaison d'ADN immunostimulatrice est attachée à un substrat sélectionné parmi le groupe constitué :

d'une plaque à puits multiples en plastique, d'une perle, d'une résine, d'un filtre, d'une lame de verre, d'une puce dite BIAcore et d'une puce en silicium.

Figure 1

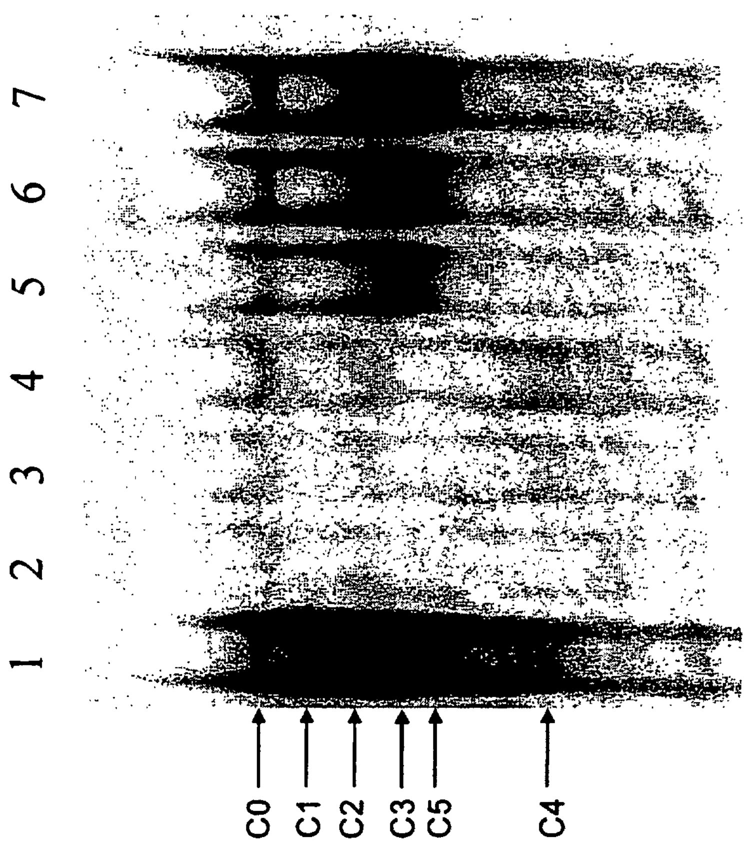

Figure 2

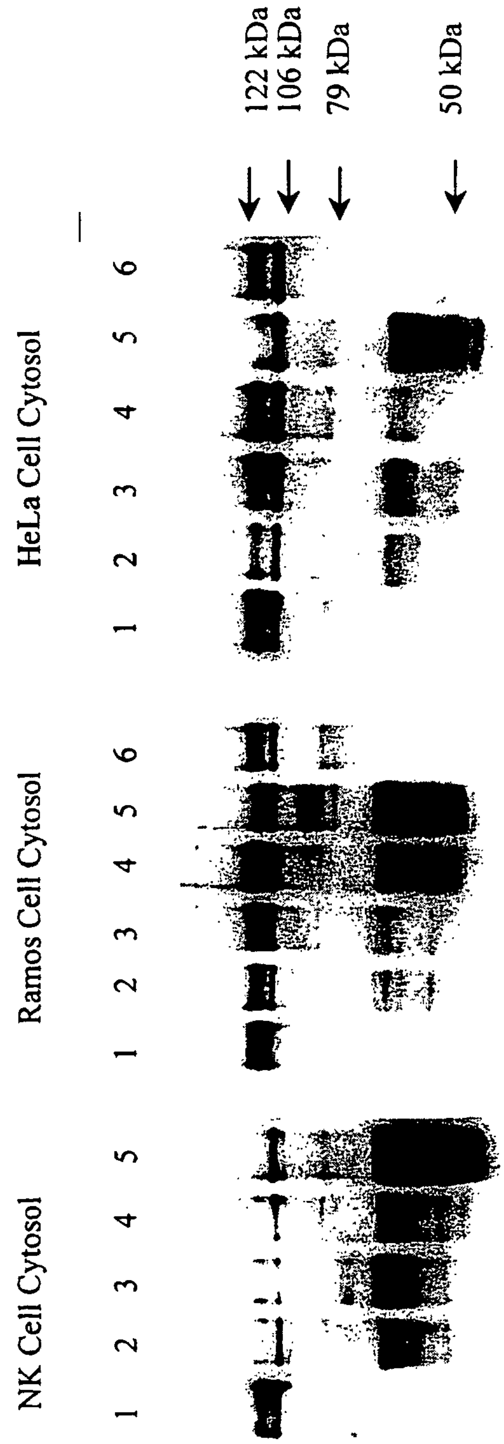
NK Cell Cytosol
1 2 3 4 5
Ramos Cell Cytosol
1 2 3 4 5 6
HeLa Cell Cytosol
1 2 3 4 5 6
122 kDa
106 kDa
79 kDa
50 kDa

Figure 3

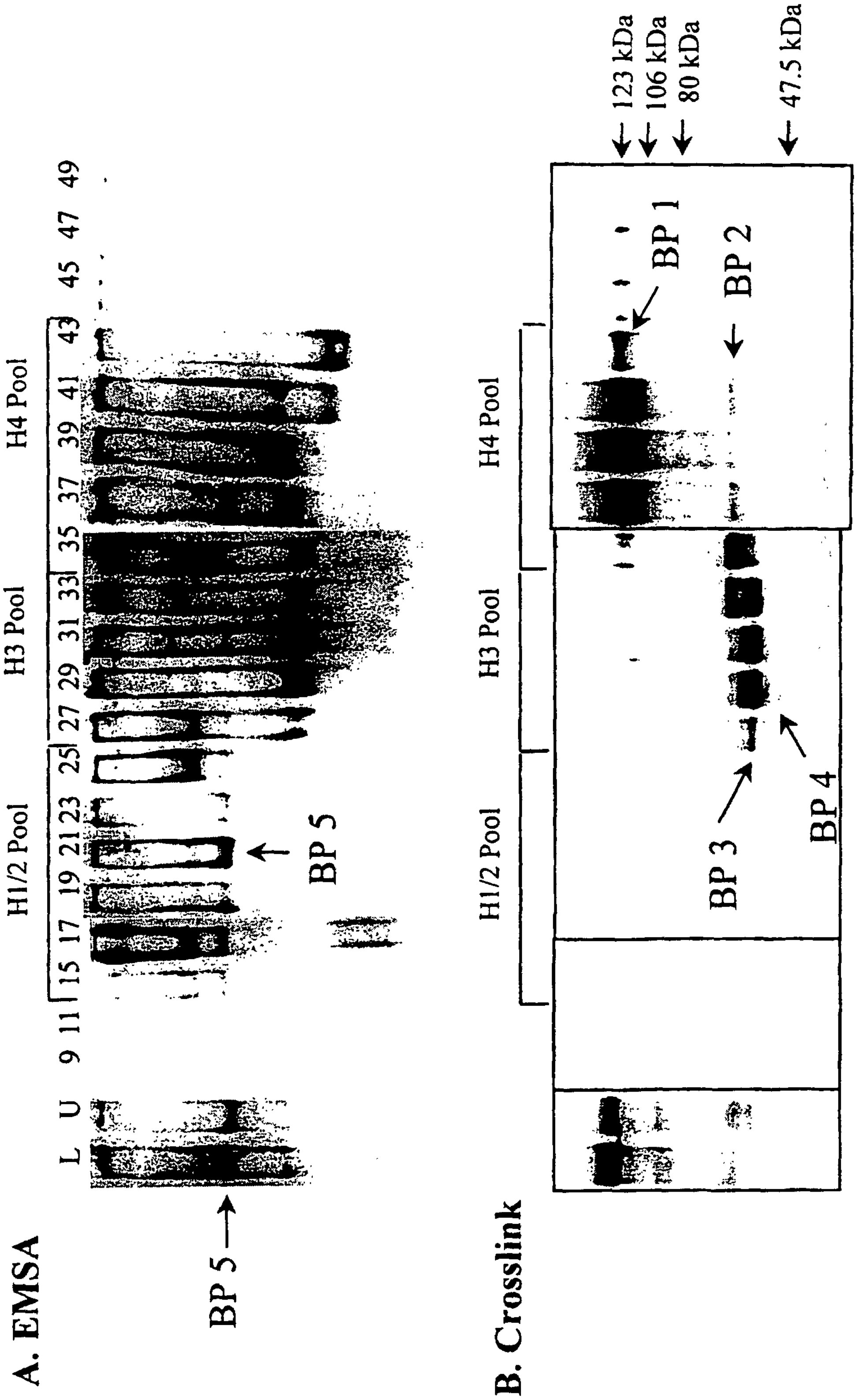
A. EMSA
BP 5
L U 9 11 15 17 19 21 23 25 27 29 31 33 35 37 39 41 43 45 47 49
H1/2 Pool
H3 Pool
H4 Pool
BP 5
B. Crosslink
H1/2 Pool
H3 Pool
H4 Pool
BP 1
BP 2
BP 3
BP 4
123 kDa
106 kDa
80 kDa
47.5 kDa

Figure 4

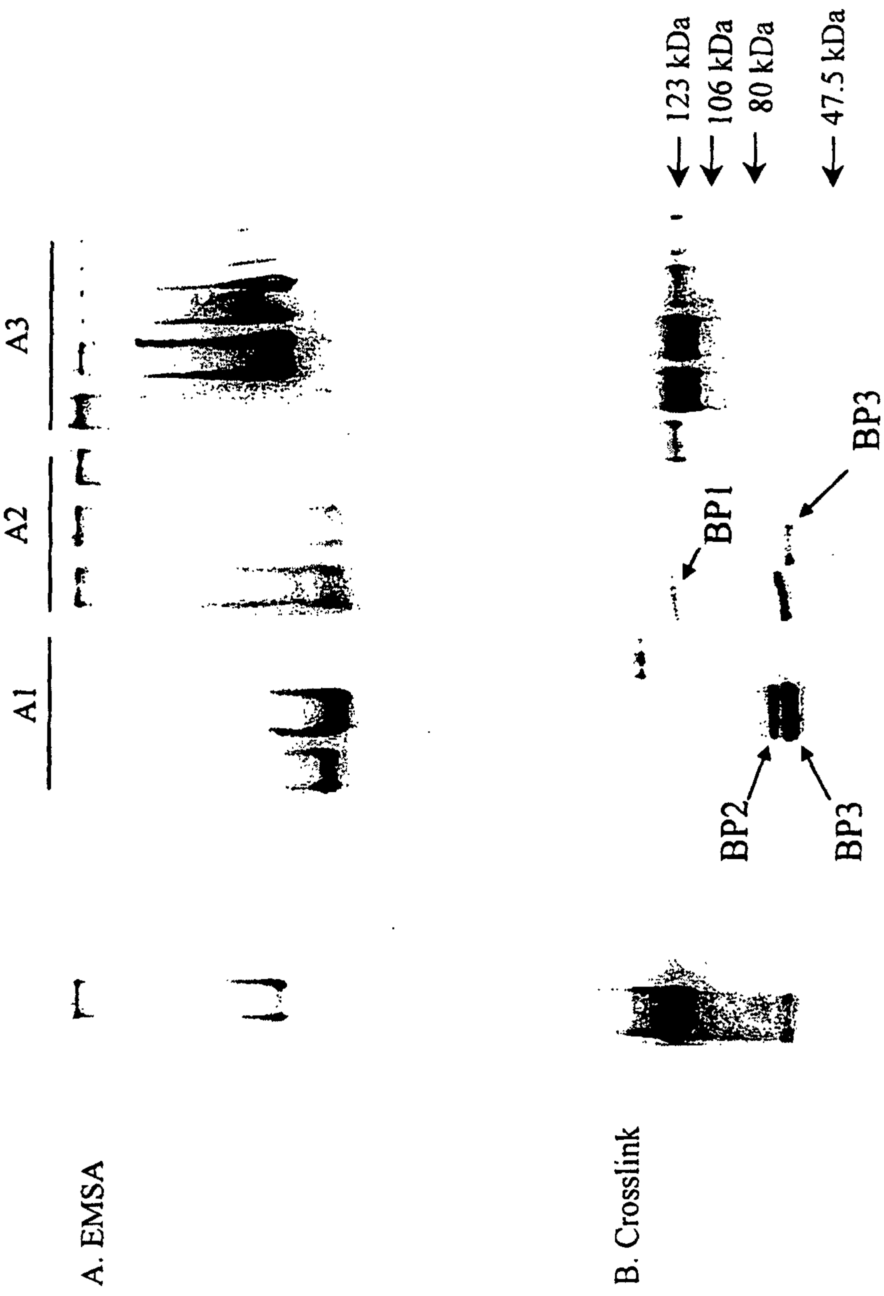
A. EMSA
A1
A2
A3
B. Crosslink
BP1
BP2
BP3
BP3
123 kDa
106 kDa
80 kDa
47.5 kDa

Figure 5

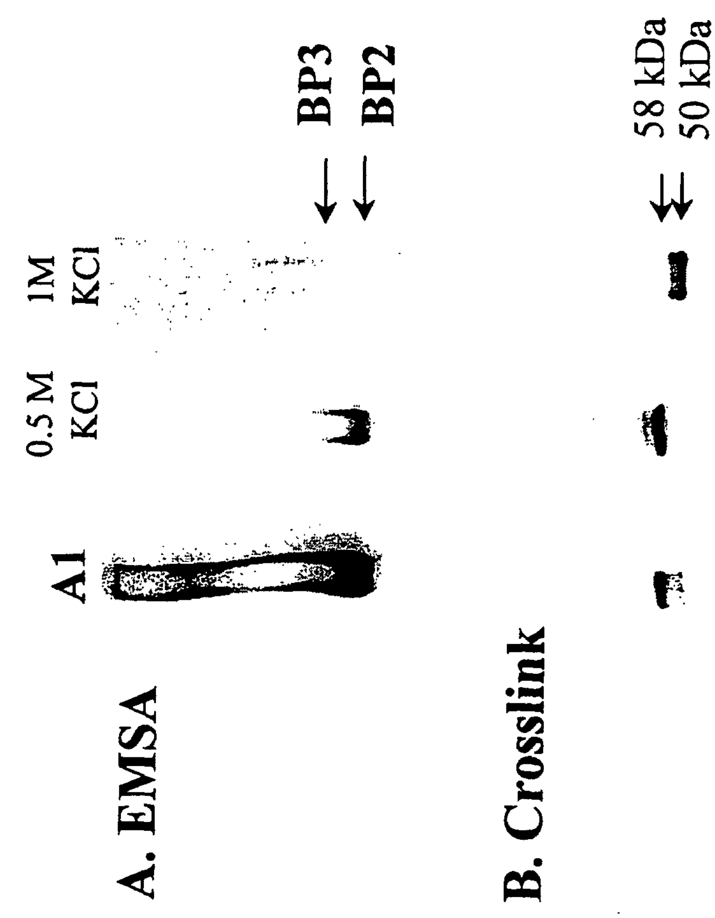

Figure 6

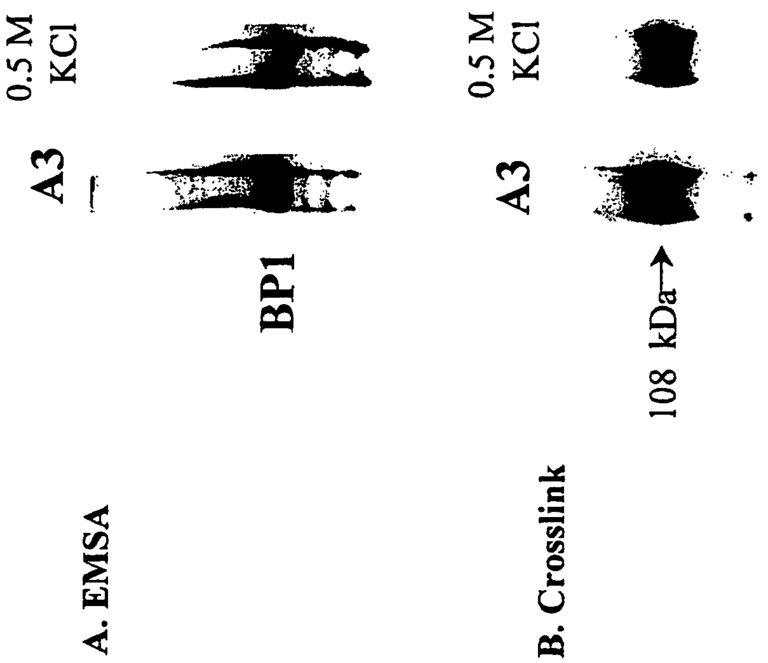

Figure 7

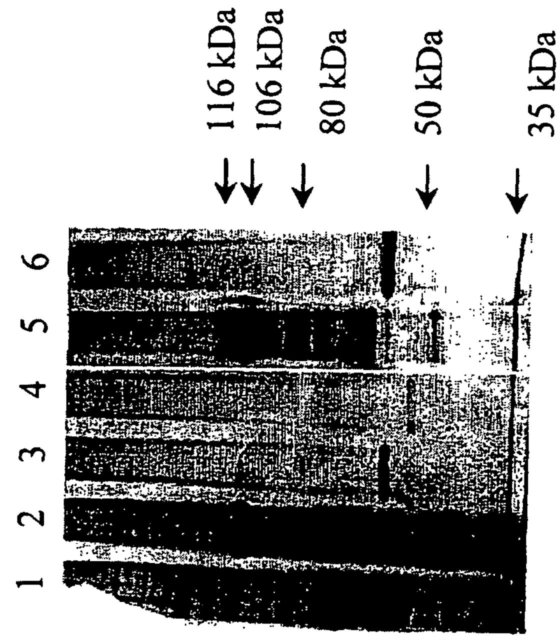

# Figure 8

A. EMSA

B. Crosslink

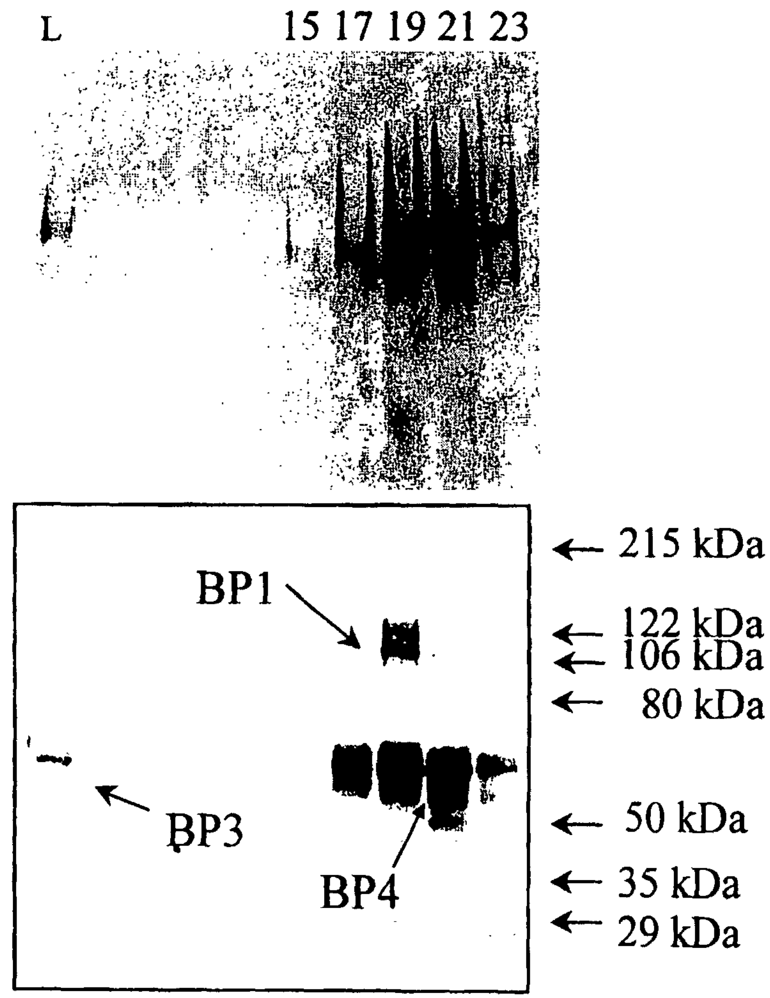

Figure 9

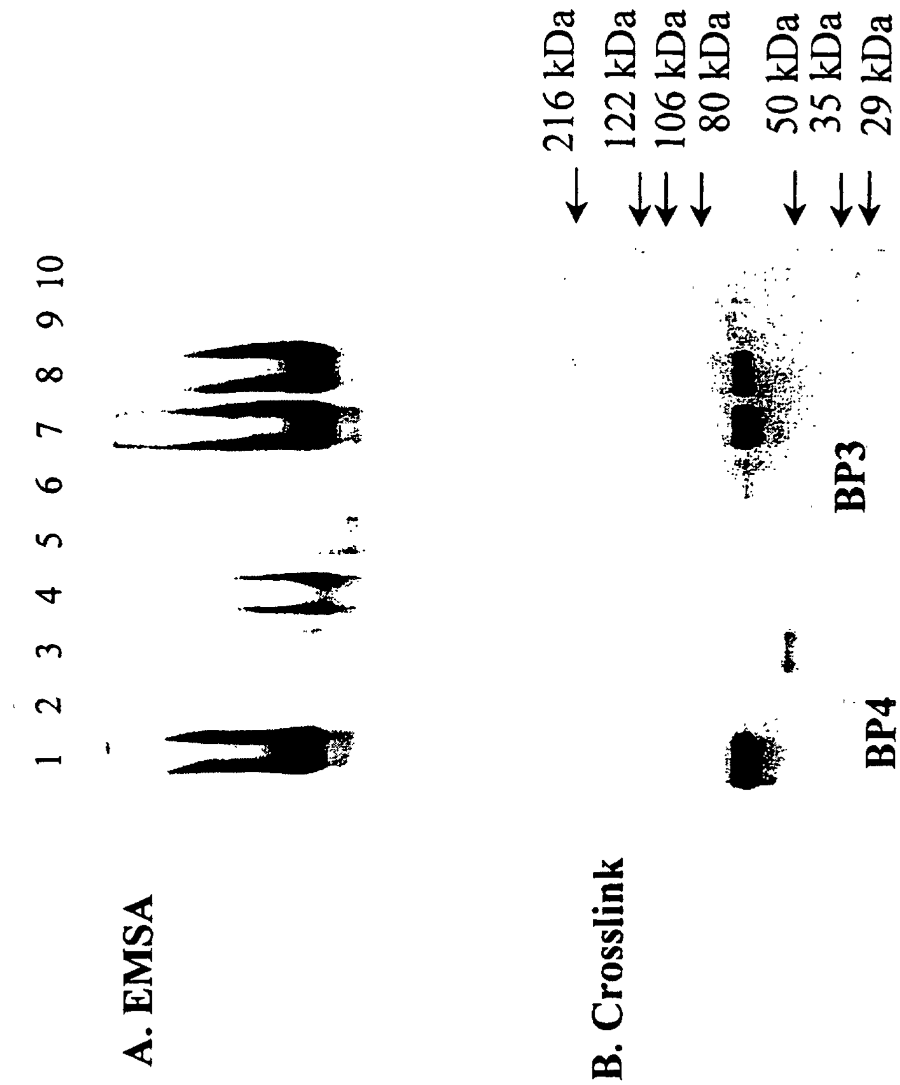
A. EMSA
1 2 3 4 5 6 7 8 9 10
B. Crosslink
BP4
BP3
216 kDa
122 kDa
106 kDa
80 kDa
50 kDa
35 kDa
29 kDa

Figure 10

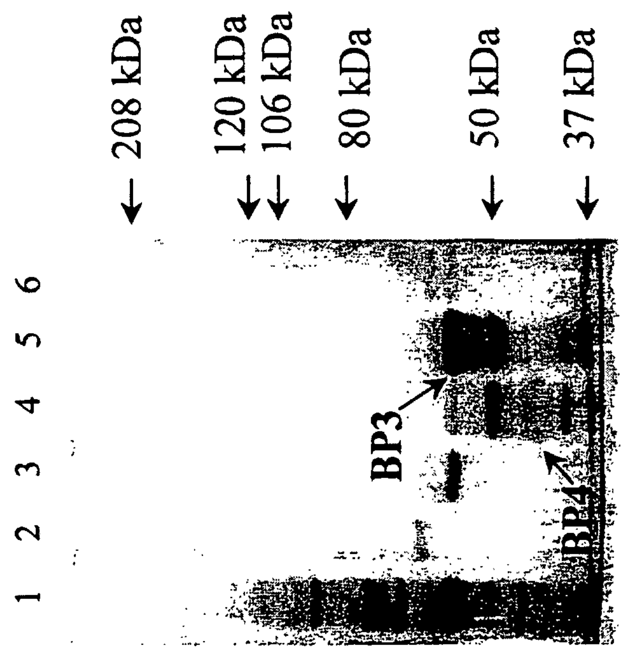

Figure 11

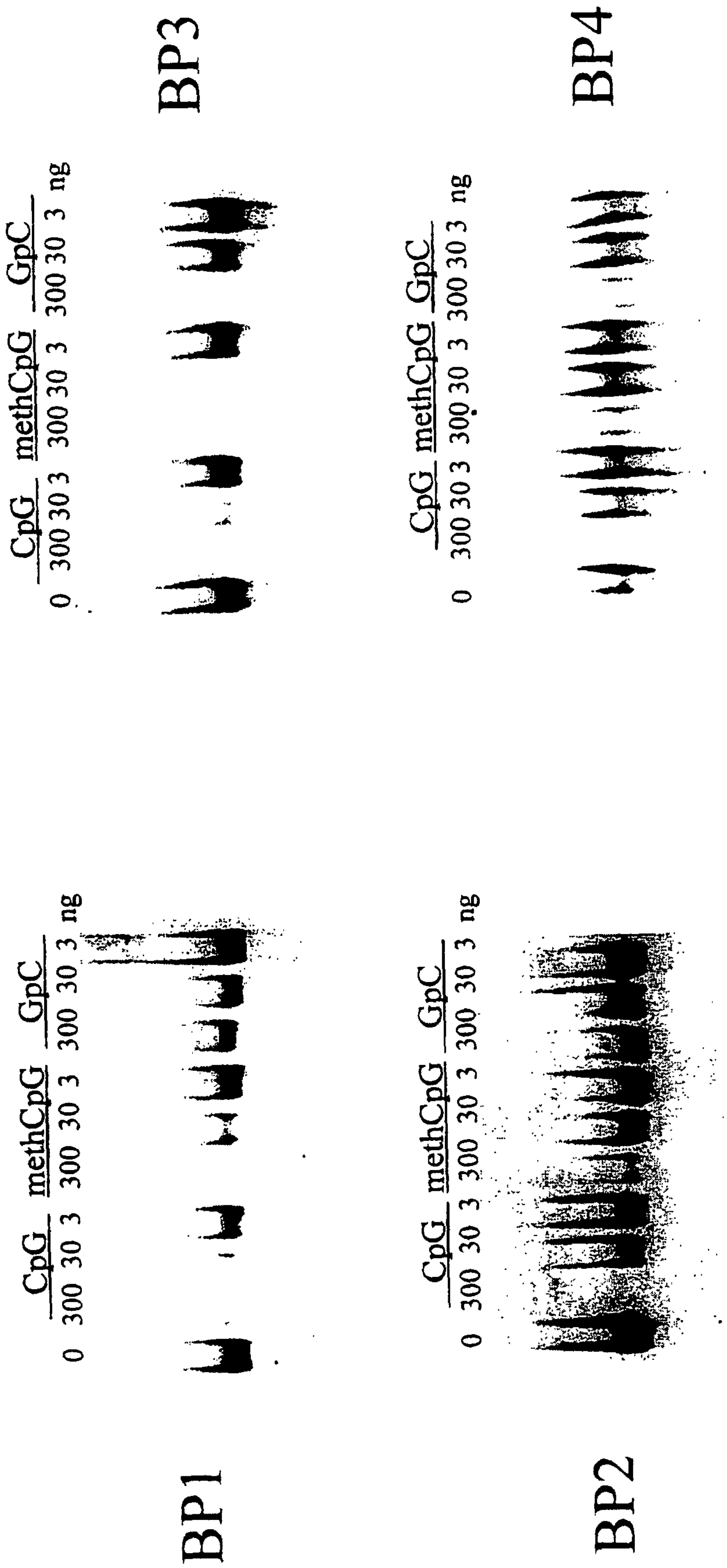
BP1
CpG methCpG GpC
0 300 30 3 300 30 3 300 30 3 ng
BP2
CpG methCpG GpC
0 300 30 3 300 30 3 300 30 3 ng
BP3
CpG methCpG GpC
0 300 30 3 300 30 3 300 30 3 ng
BP4
CpG methCpG GpC
0 300 30 3 300 30 3 300 30 3 ng

Figure 12

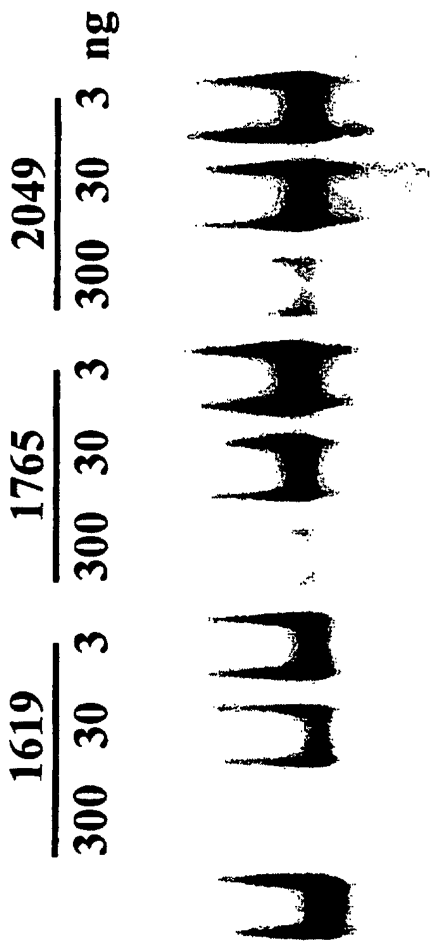

Figure 13

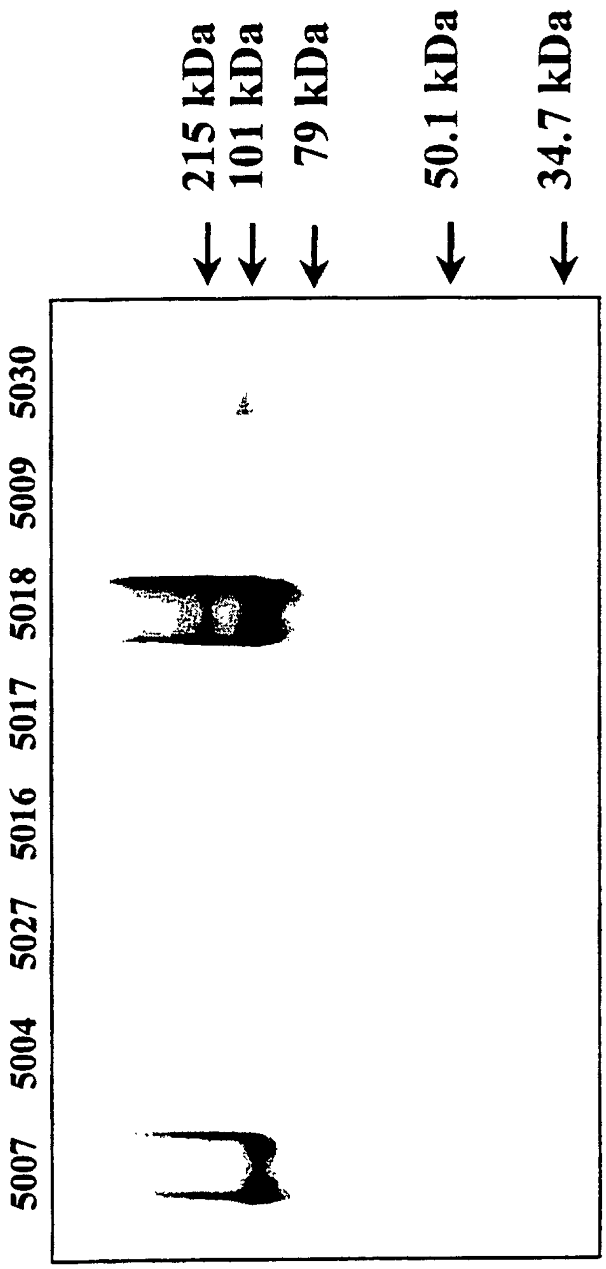
5007
5004
5027
5016
5017
5018
5009
5030
215 kDa
101 kDa
79 kDa
50.1 kDa
34.7 kDa

Figure 14

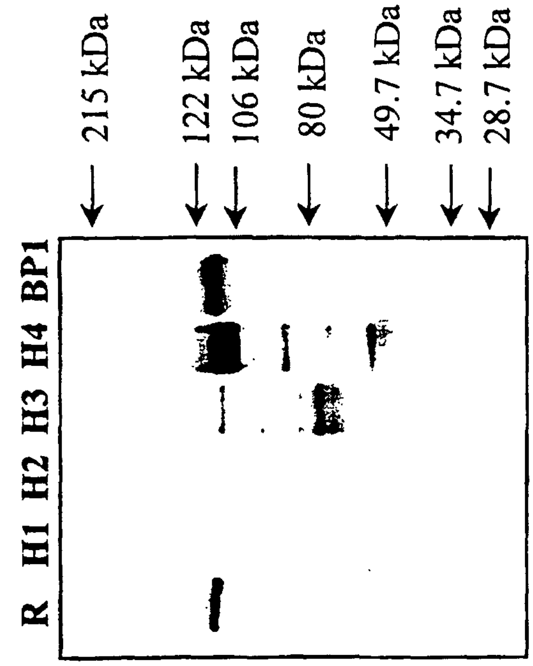
R H1 H2 H3 H4 BP1
215 kDa
122 kDa
106 kDa
80 kDa
49.7 kDa
34.7 kDa
28.7 kDa

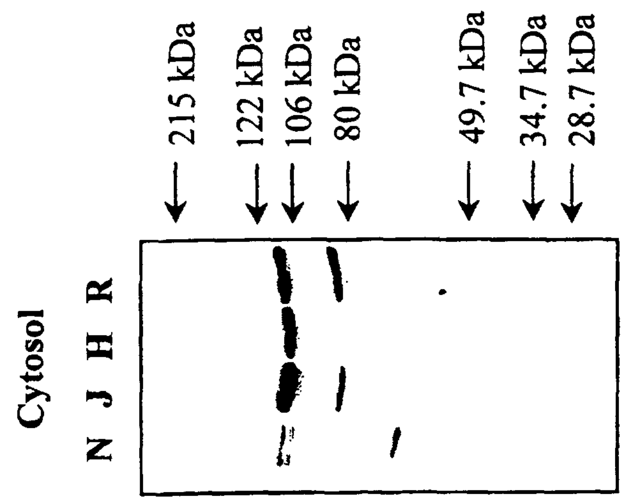
Cytosol
N J H R
215 kDa
122 kDa
106 kDa
80 kDa
49.7 kDa
34.7 kDa
28.7 kDa

Figure 15

SEQUENCE LISTING

<110> CpG ImmunoPharmaceuticals GmbH
University of Iowa Research Foundation

<120> Screening Assays for Identifying
Inhibitors, Mimics, Agonists, Antagonists, and Binding Compounds

<130> C1039/7036WO

<150> US 60/131,830

<151> 1999-04-29

<150> US 60/186,845

<151> 2000-03-03

<160> 22

<170> FastSEQ for Windows Version 3.0

<210> 1

<211> 24

<212> DNA

<213> Artificial Sequence

<220>

<223> synthetic oligonucleotide

<400> 1

tcgtcgtttt gtcgttttgt cgtt 24

<210> 2

<211> 20

<212> DNA

<213> Artificial Sequence

<220>

<223> synthetic oligonucleotide

<400> 2

tcgtcgttcc cccccccccc 20

<210> 3

<211> 20

<212> DNA

<213> Artificial Sequence

<220>

<223> synthetic oligonucleotide

<400> 3

tccatgtcgt tcctgatgct 20

<210> 4

<211> 20

<212> DNA

<213> Artificial Sequence

<220>

<223> synthetic oligonucleotide

<221> modified_base

<222> (8)...(8)

<223> m5c

<400> 4

tccatgtcgt tcctgatgct 20

<210> 5

<211> 20

<212> DNA

<213> Artificial Sequence

<220>

<223> synthetic oligonucleotide

<400> 5

tccatgtgct tcctgatgct 20

<210> 6

<211> 24

<212> DNA

<213> Artificial Sequence

<220>

<223> synthetic oligonucleotide

<221> modified_base

<222> (2) ... (2)

<223> m5c

<221> modified_base

<222> (5) ... (5)

<223> m5c

<221> modified_base

<222> (13)...(13)

<223> m5c

<221> modified base

<222> (21)...(21)

<223> m5c

<400> 6

tcgtcgtttt gtcgttttgt cgtt 24

<210> 7

<211> 24

<212> DNA

<213> Artificial Sequence

<220>

<223> synthetic oligonucleotide

<400> 7

tgctgctttt gtgcttttgt gctt 24

<210> 8

<211> 20

<212> DNA

<213> Artificial Sequence

<220>

<223> synthetic oligonucleotide

<221> misc_feature

<222> (1)...(1)

<223> modified with digoxigenin

<400> 8

cccccccccc cccccccccc 20

<210> 9

<211> 20

<212> DNA

<213> Artificial Sequence

<220>

<223> synthetic oligonucleotide

<221> misc_feature

<222> (1)...(1)

<223> modified with digoxigenin

<400> 9

tttttttttt tttttttttt 20

<210> 10

<211> 24

<212> DNA

<213> Artificial Sequence

<220>

<223> synthetic oligonucleotide

<221> misc_feature

<222> (1)...(1)

<223> modified with amino group

<400> 10

tcgtcgtttt gtcgttttgt cgtt 24

<210> 11

<211> 24

<212> DNA

<213> Artificial Sequence

<220>

<223> synthetic oligonucleotide

<221> misc_feature

<222> (1)...(24)

<223> phosphorothioate backbone

<221> misc_feature

<222> (1)...(1)

<223> modified with digoxigenin

<400> 11

tcgtcgtttt gtcgttttgt cgtt 24

<210> 12

<211> 24

<212> DNA

<213> Artificial Sequence

<220>

<223> synthetic oligonucleotide

<221> misc_feature

<222> (1)...(1)

<223> modified with digoxigenin

<400> 12

tcgtcgtttt gtcgttttgt cgtt 24

<210> 13

<211> 24

<212> DNA

<213> Artificial Sequence

<220>

<223> synthetic oligonucleotide

<221> misc_feature

<222> (1)...(1)

<223> modified with digoxigenin

<221> modified_base

<222> (2)...(2)

<223> m5c

<221> modified_base

<222> (5)...(5)

<223> m5c

<221> modified_base

<222> (13)...(13)

<223> m5c

<221> modified_base

<222> (21)...(21)

<223> m5c

<400> 13

tcgtcgtttt gtcgttttgt cgtt 24

<210> 14

<211> 24

<212> DNA

<213> Artificial Sequence

<220>

<223> synthetic oligonucleotide

<221> misc_feature

<222> (1) ... (1)

<223> modified with digoxigenin

<400> 14

tgctgctttt gtgcttttgt gctt 24

<210> 15

<211> 20

<212> DNA

<213> Artificial Sequence

<220>

<223> synthetic oligonucleotide

<221> misc_feature

<222> (1)...(20)

<223> phosphorothioate backbone

<221> misc_feature

<222> (1)...(1)

<223> modified with digoxigenin

<400> 15

tcgtcgttcc cccccccccc 20

<210> 16

<211> 24

<212> DNA

<213> Artificial Sequence

<220>

<223> synthetic oligonucleotide

<221> misc_feature

<222> (1)...(24)

<223> phosphorothioate backbone

<221> misc_feature

<222> (1)...(1)

<223> modified with amino group

<400> 16

tcgtcgtttt gtcgttttgt cgtt 24

<210> 17

<211> 20

<212> DNA

<213> Artificial Sequence

<220>

<223> synthetic oligonucleotide

<221> misc_feature

<222> (1)...(20)

<223> phosphorothioate backbone

<221> misc_feature

<222> (1)...(1)

<223> modified with digoxigenin

<400> 17

tttttttttt tttttttttt 20

<210> 18

<211> 20

<212> DNA

<213> Artificial Sequence

<220>

<223> synthetic oligonucleotide

<221> misc_feature

<222> (1)...(20)

<223> phosphorothioate backbone

<221> misc_feature

<222> (1)...(1)

<223> modified with digoxigenin

<400> 18

cccccccccc cccccccccc 20

<210> 19

<211> 24

<212> DNA

<213> Artificial Sequence

<220>

<223> synthetic oligonucleotide

<221> misc_feature

<222> (24)...(24)

<223> modified with digoxigenin

<400> 19

tcgtcgtttt gtcgttttgt cgtt 24

<210> 20

<211> 20

<212> DNA

<213> Artificial Sequence

<220>

<223> synthetic oligonucleotide

<400> 20

tttttttttt tttttttttt 20

<210> 21

<211> 20

<212> DNA

<213> Artificial Sequence

<220>

<223> synthetic oligonucleotide

<400> 21

cccccccccc cccccccccc 20

<210> 22

<211> 24

<212> DNA

<213> Artificial Sequence

<220>

<223> synthetic oligonucleotide

<400> 22

ctggtctttc tggttttttt ctgg 24